# EUROPÄISCHE PATENTSCHRIFT

(11) **EP 1 112 256 B1**
(45) Veröffentlichungstag und Bekanntmachung des Hinweises auf die Patenterteilung: **29.10.2003**
(21) Anmeldenummer: 99944541.4
(22) Anmeldetag: 27.08.1999
(51) Int. Cl.: C07D 215/18, A01N 43/42, C07D 215/14, C07D 215/36, C07D 405/12, C07D 401/08, C07D 401/06, C07F 9/60

(54) **CYCLOHEXENONCHINOLINOYL-DERIVATE ALS HERBIZIDE MITTEL**
CYCLOHEXENONQUINOLINOYL-DERIVATIVES AS HERBICIDAL AGENTS
UTILISATION DE DERIVES DE CYCLOHEXENONCHINOLINOYL COMME HERBICIDES

(30) Priorität: 08.09.1998 DE 19840799
(43) Veröffentlichungstag der Anmeldung: 04.07.2001
(73) Patentinhaber: BASF AKTIENGESELLSCHAFT, 67056 Ludwigshafen (DE)
(72) Erfinder: WITSCHEL, Matthias, D-67061 Ludwigshafen (DE); MISSLITZ, Ulf, D-67433 Neustadt (DE); BAUMANN, Ernst, D-67373 Dudenhofen (DE); VON DEYN, Wolfgang, D-67435 Neustadt (DE); LANGEMANN, Klaus, D-67551 Worms (DE); MAYER, Guido, D-67433 Neustadt (DE); NEIDLEIN, Ulf, D-68165 Mannheim (DE); GÖTZ, Roland, D-68809 Neulu heim (DE); GÖTZ, Norbert, D-67547 Worms (DE); RACK, Michael, D-69123 Heidelberg (DE); ENGEL, Stefan, D-55268 Nieder-Olm (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: EP9906322
(87) Internationale Veröffentlichungsnummer: WO00014069

(56) Entgegenhaltungen:
- EP-A- 0 283 261
- WO-A-98/12180

## Beschreibung

Die vorliegende Erfindung betrifft neue Cyclohexanonchinolinoyl-Derivate der Formel I, 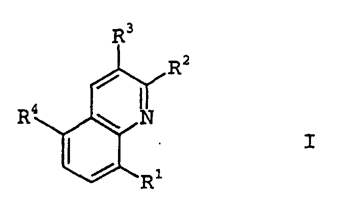 in der die Variablen folgende Bedeutung haben:
- R¹: Wasserstoff, Nitro, Halogen, Cyano, C₁-C₆-Alkyl, C₁-C₆-Halogenalkyl, C₁-C₆-Alkoxyiminomethyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino, N-(C₁-C₆-Alkyl)-N-(C₁-C₆-alkylsulfonyl)-amino, N-(C₁-C₆-Alky)-N-(C₁-C₆-halogenalkylsulfonyl)-amino, Phenoxy, Heterocyclyloxy, Phenylthio oder Heterocyclylthio, wobei die vier letztgenannten Reste partiell oder vollständig halogeniert sein können und/oder einen bis drei der nachfolgend genannten Substituenten tragen können: Nitro, Cyano, C₁-C₄-Alkyl, C₁-C₄-Halogenalkyl, C₁-C₄-Alkoxy oder C₁-C₄-Halogenalkyoxy;
- R², R³: Wasserstoff, C₁-C₆-Alkyl, C₁-C₆-Halogenalkyl oder Halogen;
- R⁴: eine Verbindung IIa oder IIb 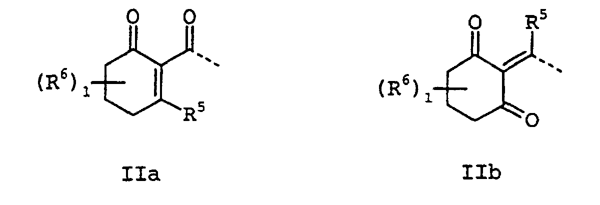
wobei
- R⁵: Halogen, OR⁷, SR⁷, SOR^{8,} SO₂R^{8,} OSO₂R⁸, POR⁸R⁹, OPR⁸R⁹, OPOR⁸R⁹, OPSR⁸R⁹, NR¹⁰R¹¹, ONR¹¹R¹², N-gebundenes Heterocyclyl oder O-(N-gebundenes Heterocyclyl), wobei der Heterocyclyl-Rest der beiden letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R⁶: Nitro, Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylcarbonyl, C₁-C₆-Halogenalkylcarbonyl, C₁-C₆-Alkoxycarbonyl oder C₁-C₆-Halogenalkoxycarbonyl;
oder
- zwei Reste R⁶,: die am gleichen Kohlenstoff gebunden sind, bilden gemeinsam eine -O-(CH₂)ₘ-O-, -O-(CH₂)ₘ-S-, -S-(CH₂)ₘ-S-, -O-(CH₂)ₙ- oder -S-(CH₂)ₙ-Kette, die durch einen bis drei Reste aus folgender Gruppe substituiert sein kann: Halogen, Cyano, C₁-C₄-Alkyl, C₁-C₄-Halogenalkyl oder C₁-C₄-Alkoxycarbonyl;
oder
- zwei Reste R⁶,: die am gleichen Kohlenstoff gebunden sind, bilden gemeinsam eine -(CH₂)ₚ-Kette, die durch Sauerstoff oder Schwefel unterbrochen sein kann und/oder durch einen bis vier Reste aus folgender Gruppe substituiert sein kann:
Halogen, Cyano, C₁-C₄-Alkyl, C₁-C₄-Halogenalkyl oder C₁-C₄-Alkoxycarbonyl;
oder
- zwei Reste R⁶,: die am gleichen Kohlenstoff gebunden sind, bilden gemeinsam eine Methylidengruppe, die durch einen bis zwei Reste aus folgender Gruppe substituiert sein kann: Halogen, Hydroxy, Formyl, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
oder
- zwei Reste R⁶,: die am gleichen Kohlenstoff gebunden sind, bilden gemeinsam mit diesem Kohlenstoff eine Carbonylgruppe aus;
oder
- zwei Reste R⁶,: die an verschiedenen Kohlenstoffen gebunden sind, bilden gemeinsam eine -(CH₂)ₙ-Kette, die durch einen bis drei Reste aus folgender Gruppe substituiert sein kann:
Halogen, C₁-C₆-Alkyl, C₁-C₆-Alkoxy, Hydroxy oder C₁-C₆-Alkoxycarbonyl;
- R⁷: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₂₀-Alkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, (C₁-C₂₀-Alkylthio)carbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, C₁-C₄-Alkoxy, C₁-C₄-Alkylthio, Di-(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Heterocyclyloxycarbonyl, Phenoxythiocarbonyl, Heterocyclyloxythiocarbonyl, Phenoxy-C₁-C₆-alkylcarbonyl, Heterocyclyloxy-C₁-C₆-alkylcarbonyl, Phenylaminocarbonyl, N-(C₁-C₆-Alkyl)-N-(phenyl)-aminocarbonyl, Heterocyclylaminocarbonyl, N-(C₁-C₆-Alkyl)-N-(heterocyclyl)-aminocarbonyl, Phenyl-C₂-C₆-alkenylcarbonyl oder Heterocyclyl-C₂-C₆-alkenylcarbonyl, wobei der Phenyl- und der Heterocyclyl-Rest der 20 letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R⁸, R⁹: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, Amino, C₁-C₆-Alkylamino, C₁-C₆-Halogenalkylamino, Di-(C₁-C₆-alkyl)amino, Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁰: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-Alkyl)-amino oder C₁-C₆-Alkylcarbonylamino, wobei die genannten Alkyl-, Cycloalkyl- und Alkoxyreste partiell oder vollständig halogeniert sein können und/oder einen bis drei Reste der folgenden Gruppe tragen können: Cyano, C₁-C₄-Alkoxy, C₁-C₄-Alkylthio, Di-(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹¹, R¹²: C₁-C₆-Alkyl, C₃-C₆-Alkenyl, C₃-C₆-Alkinyl oder C₁-C₆-Alkylcarbonyl;
- 1: 0 bis 6;
- m: 2 bis 4;
- n: 1 bis 5;
- p: 2 bis 5;
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus WO 98/12 180 und EP-A 283 261 sind Chinolinoyl- bzw. anellierte Phenyl-Derivate, die mit einem gegebenenfalls substituierten (1-Hydroxy-3-oxo-cyclohex-1-en-2-yl)carbonyl-Rest verknüpft sind, bekannt. Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, weitere, biologisch, insbesondere herbizid wirksame, Verbindungen zu finden.

Demgemäß wurden die Cyclohexenonchinolinoyl-Derivate der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹² oder als Reste an Phenyl- und Heterocyclyl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl-, N,N-Dialkylaminosulfonyl-, N-Alkylamino-, N,N-Dialkylamino-, N-Halogenalkylamino-, N-Alkoxyamino-, N-Alkoxy-N-alkylamino-, N-Alkylcarbonylamino-, N-Alkylsulfonylamino-, N-Halogenalkylsulfonylamino-, N-Alkyl-N-alkylsulfonylamino-, N-Alkyl-N-halogenalkylsulfonylamino-, Alkylcarbonyl-, Halogenalkylcarbonyl-, Alkoxycarbonyl-, Halogenalkoxycarbonyl-, Alkylthiocarbonyl-, Alkylcarbonyloxy-, Alkylaminocarbonyl-, Dialkylaminocarbonyl-, Dialkylaminothiocarbonyl-, Alkoxyalkyl-, Dialkoxymethyl-, Dialkylthiomethyl-, (Alkoxy)(alkylthio)methyl-, Alkylcarbonylalkyl-, Alkoxyiminomethyl, Alkoxyiminoalkyl-, N-(Alkylamino)-iminoalkyl-, N-(Dialkylamino)-iminoalkyl-, Phenylalkenylcarbonyl-, Heterocyclylalkenylcarbonyl-, Phenoxyalkylcarbonyl, Heterocyclyloxyalkylcarbonyl, N-Alkoxy-N-alkylaminocarbonyl-, N-Alkyl-N-phenylaminocarbonyl-, N-Alkyl-N-heterocyclylaminocarbonyl-, Alkoxycarbonyloxy, Phenylalkyl-, Heterocyclylalkyl-, Phenylcarbonylalkyl-, Heterocyclylcarbonylalkyl-, Dialkylaminoalkoxycarbonyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl-, Alkenyloxy, Alkinyloxy und Alkoxyalkoxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: z.B. Methyl, Ethyl, Propyl, 1-Methylethyl, Butyl, 1-Methylpropyl, 2-Methylpropyl oder 1,1-Dimethylethyl;
- C₁-C₆-Alkyl, sowie die Alkylteile von C₁-C₆-Alkoxyimino-C₁-C₆alkyl, N-(C₁-C₆-Alkylamino)-imino-C₁-C₆-alkyl, N-(Di-C₁-C₆alkylamino)-imino-C₁-C₆-alkyl, N-(C₁-C₆-Alkoxy)-N-(C₁-C₆alkyl)-aminocarbonyl, N-(C₃-C₆-Alkenyl)-N-(C₁-C₆-alkyl)-aminocarbonyl, (C₃-C₆-Alkinyl)-N-(C₁-C₆-alkyl)-aminocarbonyl, N-(C₁-C₆-Alkyl)-N-phenylaminocarbonyl, N-(C₁-C₆-Alkyl)-N-heterocyclylaminocarbonyl, Phenyl-C₁-C₆-alkyl, N-(C₁-C₆-Alkyl)-N-(C₁-C₆-alkylsulfonyl)-amino, N-(C₁-C₆-Alkyl)-N-(C₁-C₆-halogenalkylsulfonyl)-amino, Heterocyclyl-C₁-C₆-alkyl, Phenylcarbonyl-C₁-C₆-alkyl, Heterocyclylcarbonyl-C₁-C₆-alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl, sowie die Halogenalkylteile von N-C₁-C₆-Halogenalkylamino: C₁-C₄-Halogenalkyl, wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy: z.B. Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von N-C₁-C₆-Alkoxyamino, Di-(C₁-C₆-alkoxy)methyl, (C₁-C₆-Alkoxy)(C₁-C₆-alkylthio)methyl, C₁-C₆-Alkoxyiminomethyl, C₁-C₆-Alkoxyimino-C₁-C₆-alkyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl und N-(C₃-C₆-Alkinyl)-N-(C₁-C₆-alkoxy)-aminocarbonyl: C₁-C₄-Alkoxy, wie voranstehend genannt, sowie z.B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio: z.B. Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio, sowie die Alkylthioteile von (C₁-C₆-Alkylthio) carbonyl, Di-(C₁-C₆-alkylthio)methyl und (C₁-C₆-Alkoxy)-(C₁-C₆-alkylthio)methyl: C₁-C₄-Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₂₀-Alkylthio als Alkylthiorest von (C₁-C₂₀-Alkylthio)carbonyl: C₁-C₆-Alkylthio wie voranstehend genannt, sowie z.B. Heptylthio, Octylthio, Hexadecylthio oder Octadecylthio;
- C₁-C₄-Halogenalkylthio: einen C₁-C₄-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₆-Halogenalkylthio: C₁-C₄-Halogenalkylthio, wie voranstehend genannt, sowie z.B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₆-Alkylsulfonyl (C₁-C₆-Alkyl-S(=O)₂-), sowie die Alkylsulfonylreste von N-(C₁-C₆-Alkylsulfonyl)-amino und N-(C₁-C₆-Alkyl)-N-(C₁-C₆-alkylsulfonyl)-amino: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
- C₁-C₆-Halogenalkylsulfonyl, sowie die Halogenalkylsulfonylreste von N-(C₁-C₆-Halogenalkylsulfonyl)-amino und N-(C₁-C₆-Alkyl)-N-(C₁-C₆-halogenalkylsulfonyl)-amino: einen C₁-C₆-Alkylsulfonylrest, wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₁-C₆-Alkylamino, sowie die Alkylaminoreste von N-(C₁-C₆-Alkylamino)-imino-C₁-C₆-alkyl, also z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- (C₁-C₄-Alkylamino)sulfonyl: z.B. Methylaminosulfonyl, Ethylaminosulfonyl, Propylaminosulfonyl, 1-Methylethylaminosulfonyl, Butylaminosulfonyl, 1-Methylpropylaminosulfonyl, 2-Methylpropylaminosulfonyl oder 1,1-Dimethylethylaminosulfonyl;
- (C₁-C₆-Alkylamino)sulfonyl: (C₁-C₄-Alkylamino)sulfonyl, wie vorstehend genannt, sowie z.B. Pentylaminosulfonyl, 1-Methylbutylaminosulfonyl, 2-Methylbutylaminosulfonyl, 3-Methylbutylaminosulfonyl, 2,2-Dimethylpropylaminosulfonyl, 1-Ethylpropylaminosulfonyl, Hexylaminosulfonyl, 1,1-Dimethylpropylaminosulfonyl, 1,2-Dimethylpropylaminosulfonyl, 1-Methylpentylaminosulfonyl, 2-Methylpentylaminosulfonyl, 3-Methylpentylaminosulfonyl, 4-Methylpentylaminosulfonyl, 1,1-Dimethylbutylaminosulfonyl, 1,2-Dimethylbutylaminosulfonyl, 1,3-Dimethylbutylaminosulfonyl, 2,2-Dimethylbutylaminosulfonyl, 2,3-Dimethylbutylaminosulfonyl, 3,3-Dimethylbutylaminosulfonyl, 1-Ethylbutylaminosulfonyl, 2-Ethylbutylaminosulfonyl, 1,1,2-Trimethylpropylaminosulfonyl, 1,2,2-Trimethylpropylaminosulfonyl, 1-Ethyl-1-methylpropylaminosulfonyl oder 1-Ethyl-2-methylpropylaminosulfonyl;
- Di-(C₁-C₄-alkyl)-aminosulfonyl: z.B. N,N-Dimethylaminosulfonyl, N,N-Diethylaminosulfonyl, N,N-Di-(1-methylethyl)aminosulfonyl, N,N-Dipropylaminosulfonyl, N,N-Dibutylaminosulfonyl, N,N-Di-(1-methylpropyl)-aminosulfonyl, N,N-Di-(2-methylpropyl)-aminosulfonyl, N,N-Di-(1,1-dimethylethyl)-aminosulfonyl, N-Ethyl-N-methylaminosulfonyl, N-Methyl-N-propylaminosulfonyl, N-Methyl-N-(1-methylethyl)-aminosulfonyl, N-Butyl-N-methylaminosulfonyl, N-Methyl-N-(1-methylpropyl)-aminosulfonyl, N-Methyl-N-(2-methylpropyl)-aminosulfonyl, N-(1,1-Dimethylethyl)-N-methylaminosulfonyl, N-Ethyl-N-propylaminosulfonyl, N-Ethyl-N-(1-methylethyl)-aminosulfonyl, N-Butyl-N-ethylaminosulfonyl, N-Ethyl-N-(1-methylpropyl)-aminosulfonyl, N-Ethyl-N-(2-methylpropyl)-aminosulfonyl, N-Ethyl-N-(1,1-dimethylethyl)-aminosulfonyl, N-(1-Methylethyl)-N-propylaminosulfonyl, N-Butyl-N-propylaminosulfonyl, N-(1-Methylpropyl)-N-propylaminosulfonyl, N-(2-Methylpropyl)-N-propylaminosulfonyl, N-(1,1-Dimethylethyl)-N-propylaminosulfonyl, N-Butyl-N-(1-methylethyl)-aminosulfonyl, N-(1-Methylethyl)-N-(1-methylpropyl)-aminosulfonyl, N-(1-Methylethyl)-N-(2-methylpropyl)-aminosulfonyl, N-(1,1-Dimethylethyl)-N-(1-methylethyl)-aminosulfonyl, N-Butyl-N-(1-methylpropyl)-aminosulfonyl, N-Butyl-N-(2-methylpropyl)-aminosulfonyl, N-Butyl-N-(1,1-dimethylethyl)-aminosulfonyl, N-(1-Methylpropyl)-N-(2-methylpropyl)-aminosulfonyl, N-(1,1-Dimethylethyl)-N-(1-methylpropyl)-aminosulfonyl oder N-(1,1-Dimethylethyl)-N-(2-methylpropyl)-aminosulfonyl;
- Di-(C₁-C₆-alkyl)-aminosulfonyl: Di-(C₁-C₄-alkyl)-aminosulfonyl, wie voranstehend genannt, sowie z.B. N-Methyl-N-pentylaminosulfonyl, N-Methyl-N-(1-methylbutyl)-aminosulfonyl, N-Methyl-N-(2-methylbutyl)-aminosulfonyl, N-Methyl-N-(3-methylbutyl)-aminosulfonyl, N-Methyl-N-(2,2-dimethylpropyl)-aminosulfonyl, N-Methyl-N-(1-ethylpropyl)-aminosulfonyl, N-Methyl-N-hexylaminosulfonyl, N-Methyl-N-(1,1-dimethylpropyl)-aminosulfonyl, N-Methyl-N-(1,2-dimethylpropyl)-aminosulfonyl, N-Methyl-N-(1-methylpentyl)-aminosulfonyl, N-Methyl-N-(2-methylpentyl)-aminosulfonyl, N-Methyl-N-(3-methylpentyl)-aminosulfonyl, N-Methyl-N-(4-methylpentyl)-aminosulfonyl, N-Methyl-N-(1,1-dimethylbutyl)-aminosulfonyl, N-Methyl-N-(1,2-dimethylbutyl)-aminosulfonyl, N-Methyl-N-(1,3-dimethylbutyl)-aminosulfonyl, N-Methyl-N-(2,2-dimethylbutyl)-aminosulfonyl, N-Methyl-N-(2,3-dimethylbutyl)-aminosulfonyl, N-Methyl-N-(3,3-dimethylbutyl)-aminosulfonyl, N-Methyl-N-(1-ethylbutyl)-aminosulfonyl, N-Methyl-N-(2-ethylbutyl)-aminosulfonyl, N-Methyl-N-(1,1,2-trimethylpropyl)-aminosulfonyl, N-Methyl-N-(1,2,2-trimethylpropyl)-aminosulfonyl, N-Methyl-N-(1-ethyl-1-methylpropyl)-aminosulfonyl, N-Methyl-N-(l-ethyl-2-methylpropyl)-aminosulfonyl, N-Ethyl-N-pentylaminosulfonyl, N-Ethyl-N-(1-methylbutyl)-aminosulfonyl, N-Ethyl-N-(2-methylbutyl)-aminosulfonyl, N-Ethyl-N-(3-methylbutyl)-aminosulfonyl, N-Ethyl-N-(2,2-dimethylpropyl)-aminosulfonyl, N-Ethyl-N-(1-ethylpropyl)-aminosulfonyl, N-Ethyl-N-hexylaminosulfonyl, N-Ethyl-N-(1,1-dimethylpropyl)-aminosulfonyl, N-Ethyl-N-(1,2-dimethylpropyl)-aminosulfonyl, N-Ethyl-N-(1-methylpentyl)-aminosulfonyl, N-Ethyl-N-(2-methylpentyl)-aminosulfonyl, N-Ethyl-N-(3-methylpentyl)-aminosulfonyl, N-Ethyl-N-(4-methylpentyl)-aminosulfonyl, N-Ethyl-N-(1,1-dimethylbutyl)-aminosulfonyl, N-Ethyl-N-(1,2-dimethylbutyl)-aminosulfonyl, N-Ethyl-N-(1,3-dimethylbutyl)-aminosulfonyl, N-Ethyl-N-(2,2-dimethylbutyl)-aminosulfonyl, N-Ethyl-N-(2,3-dimethylbutyl)-aminosulfonyl, N-Ethyl-N-(3,3-dimethylbutyl)-aminosulfonyl, N-Ethyl-N-(1-ethylbutyl)-aminosulfonyl, N-Ethyl-N-(2-ethylbutyl)-aminosulfonyl, N-Ethyl-N-(1,1,2-trimethylpropyl)-aminosulfonyl, N-Ethyl-N-(1,2,2-trimethylpropyl)-aminosulfonyl, N-Ethyl-N-(1-ethyl-1-methylpropyl)-aminosulfonyl, N-Ethyl-N-(1-ethyl-2-methylpropyl)-aminosulfonyl, N-Propyl-N-pentylaminosulfonyl, N-Butyl-N-pentylaminosulfonyl, N,N-Dipentylaminosulfonyl, N-Propyl-N-hexylaminosulfonyl, N-Butyl-N-hexylaminosulfonyl, N-Pentyl-N-hexylaminosulfonyl oder N,N-Dihexylaminosulfonyl;
- Di-(C₁-C₄-alkyl)amino, sowie die Dialkylaminoreste von Di-(C₁-C₄-alkyl)amino-C₁-C₄-alkoxycarbonyl und N-(Di-C₁-C₄alkylamino)-imino-C₁-C₆-alkyl, also z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
- Di-(C₁-C₆-alkyl)amino, sowie die Dialkylaminoreste von Di-(C₁-C₆-alkyl)amino-imino-C₁-C₆-alkyl: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino.
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von Phenoxy-C₁-C₆-alkylcarbonyl, Heterocyclyloxy-C₁-C₆-alkylcarbonyl, C₁-C₆-Alkylcarbonylamino, C₁-C₆-Alkylcarbonyl-C₁-C₆-alkyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₁-C₂₀-Alkylcarbonyl: C₁-C₆-Alkylcarbonyl, wie voranstehend genannt, sowie Heptylcarbonyl, Octylcarbonyl, Pentadecylcarbonyl oder Heptadecylcarbonyl;
- C₁-C₆-Halogenalkylcarbonyl: einen C₁-C₆-Alkylcarbonylrest, wie vorstehend genannt, der partiell oder vollständig durch Fluor, Chlor, Brom und/oder Iod substituiert ist, also z.B. Chloracetyl, Dichloracetyl, Trichloracetyl, Fluoracetyl, Difluoracetyl, Trifluoracetyl, Chlorfluoracetyl, Dichlorfluoracetyl, Chlordifluoracetyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, 2,2,3,3,3-Pentafluorpropylcarbonyl, Heptafluorpropylcarbonyl, 1-(Fluormethyl)-2-fluorethylcarbonyl, 1-(Chlormethyl)-2-chlorethylcarbonyl, 1-(Brommethyl)-2-bromethylcarbonyl, 4-Fluorbutylcarbonyl, 4-Chlorbutylcarbonyl, 4-Brombutylcarbonyl, Nonafluorbutylcarbonyl, 5-Fluorpentylcarbonyl, 5-Chlorpentylcarbonyl, 5-Brompentylcarbonyl, Perfluorpentylcarbonyl, 6-Fluorhexylcarbonyl, 6-Chlorhexylcarbonyl, 6-Bromhexylcarbonyl oder Perfluorhexylcarbonyl;
- C₁-C₄-Alkoxycarbonyl, sowie die Alkoxycarbonylteile von Di-(C₁-C₄-alkyl)amino-C₁-C₄-alkoxycarbonyl, also z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- (C₁-C₆-Alkoxy)carbonyl; sowie die Alkoxycarbonylteile von C₁-C₆-Alkoxycarbonyloxy: (C₁-C₄-Alkoxy)carbonyl, wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₆-Halogenalkoxycarbonyl: einen C₁-C₆-Alkoxycarbonylrest, wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl, 4-Iodbutoxycarbonyl, 5-Fluorpentoxycarbonyl, 5-Chlorpentoxycarbonyl, 5-Brompentoxycarbonyl, 6-Fluorhexoxycarbonyl, 6-Chlorhexoxycarbonyl oder 6-Bromhexoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆-Alkylamino)carbonyl: (C₁-C₄-Alkylamino)carbonyl, wie vorstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di-(C₁-C₄-alkyl)-aminocarbonyl: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N,N-Dimethylaminothiocarbonyl, N,N-Diethylaminothiocarbonyl, N,N-Di-(1-methylethyl) aminothiocarbonyl, N,N-Dipropylaminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl, N-Pentyl-N-hexylaminothiocarbonyl oder N,N-Dihexylaminothiocarbonyl;
- C₁-C₄-Alkoxy-C₁-C₄-alkyl: durch C₁-C₄-Alkoxy, wie vorstehend genannt, substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)-methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₄-Alkoxy-C₁-C₄-alkoxy, sowie die Alkoxyalkoxyteile von C₁-C₄-Alkoxy-C₁-C₄-alkoxycarbonyl: durch C₁-C₄-Alkoxy, wie vorstehend genannt, substituiertes C₁-C₄-Alkoxy, also z.B. für Methoxymethoxy, Ethoxymethoxy, Propoxymethoxy, (1-Methylethoxy)methoxy, Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)-propoxy, 2-(1,1-Dimethylethoxy)propoxy, 3-(Methoxy)-propoxy, 3-(Ethoxy)propoxy, 3-(Propoxy)propoxy, 3-(1-Methylethoxy)-propoxy, 3-(Butoxy)propoxy, 3-(1-Methylpropoxy)-propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)-butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)-butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₃-C₆-Alkenyl, sowie die Alkenylteile von C₃-C₆-Alkenylcarbonyl, C₃-C₆-Alkenyloxy, C₃-C₆-Alkenyloxycarbonyl, C₃-C₆-Alkenylaminocarbonyl, N-(C₃-C₆-Alkenyl)-N-(C₁-C₆)alkylaminocarbonyl, N-(C₃-C₆-Alkenyl)-N-(C₁-C₆-alkoxy)aminocarbonyl: z.B. Prop-2-en-1-yl, But-1-en-4-yl, 1-Methylprop-2-en-1-yl, 2-Methyl-prop-2-en-1-yl, 2-Buten-1-yl, 1-Penten-3-yl, 1-Penten-4-yl, 2-Penten-4-yl, 1-Methylbut-2-en-1-yl, 2-Methyl-but-2-en-1-yl, 3-Methylbut-2-en-1-yl, 1-Methyl-but-3-en-1-yl, 2-Methylbut-3-en-1-yl, 3-Methyl-but-3-en-1-yl, 1,1-Dimethylprop-2-en-1-yl, 1,2-Dimethyl-prop-2-en-1-yl, 1-Ethylprop-2-en-1-yl, Hex-3-en-1-yl, Hex-4-en-1-yl, Hex-5-en-1-yl, 1-Methyl-pent-3-en-1-yl, 2-Methyl-pent-3-en-1-yl, 3-Methylpent-3-en-1-yl, 4-Methyl-pent-3-en-1-yl, 1-Methylpent-4-en-1-yl, 2-Methyl-pent-4-en-1-yl, 3-Methylpent-4-en-1-yl, 4-Methyl-pent-4-en-1-yl, 1,1-Dimethylbut-2-en-1-yl, 1,1-Dimethyl-but-3-en-1-yl, 1,2-Dimethylbut-2-en-1-yl, 1,2-Dimethyl-but-3-en-1-yl, 1,3-Dimethylbut-2-en-1-yl, 1,3-Dimethyl-but-3-en-1-yl, 2,2-Dimethylbut-3-en-1-yl, 2,3-Dimethyl-but-2-en-1-yl, 2,3-Dimethylbut-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-l-yl, 1,1,2-Trimethyl-prop-2-en-1-yl, 1-Ethyl-1-methyl-prop-2-en-1-yl oder 1-Ethyl-2-methylprop-2-en-1-yl;
- C₂-C₆-Alkenyl, sowie die Alkenylteile von C₂-C₆-Alkenylcarbonyl, Phenyl-C₂-C₆-alkenylcarbonyl und Heterocyclyl-C₂-C₆-alkenylcarbonyl: C₃-C₆-Alkenyl, wie voranstehend genannt, sowie Ethenyl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinyloxy, C₃-C₆-Alkinyloxycarbony, C₃-C₆-Alkinylaminocarbonyl, N-(C₃-C₆-Alkinyl)-N-(C₁-C₆alkyl)-aminocarbonyl, N-(C₃-C₆-Alkinyl)-N-(C₁-C₆-alkoxyaminocarbonyl: z.B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl;
- C₂-C₆-Alkinyl, sowie die Alkinylteile von C₂-C₆-Alkinylcarbonyl: C₃-C₆-Alkinyl, wie voranstehend genannt, sowie Ethinyl;
- C₃-C₆-Halogenalkinyl: einen C₃-C₆-Alkinylrest, wie vorstehend genannt, der partiell oder vollständig durch Fluor, Chlor, Brom und/oder Iod substituiert ist, also z.B. 1,1-Difluorprop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iodbut-3-in-1-yl, 5-Fluorpent-3-in-1-yl, 5-Iod-pent-4-in-1-yl, 6-Fluor-hex-4-in-1-yl oder 6-Iod-hex-5-in-1-yl;
- C₃-C₆-Cycloalkyl, sowie die Cycloalkylteile von C₃-C₆-Cycloalkylcarbonyl: z.B. Cyclopropyl, Cyclobutyl, Cyclopentyl oder Cyclohexyl;
- Heterocyclyl, sowie Heterocyclylteile von Heterocyclylcarbonyl, Heterocyclyl-C₁-C₆-alkyl, Heterocyclyloxy, Heterocyclylthio, Heterocyclyloxyalkylcarbonyl, Heterocyclyloxycarbonyl, Heterocyclyloxythiocarbonyl, Heterocyclylcarbonyl-C₁-C₆-alkyl, N-(C₁-C₆-Alkyl)-N-(heterocyclyl)-aminocarbonyl, Heterocyclylaminocarbonyl: ein gesättigter, partiell gesättigter oder ungesättiger 5- oder 6-gliedriger, C-gebundener, heterocyclischer Ring, der ein bis vier gleiche oder verschiedene Heteroatome, ausgewählt aus folgender Gruppe: Sauerstoff, Schwefel oder Stickstoff, enthält, also z.B. 5-gliedrige Ringe, mit einem Heteroatom wie z.B.
   Tetrahydrofuran-2-yl, Tetrahydrofuran-3-yl, Tetrahydrothien-2-yl, Tetrahydrothien-3-yl,Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 2-Furyl, 3-Furyl, 2-Thienyl, 3-Thienyl, Pyrrol-2-yl oder Pyrrol-3-yl;
   5-gliedrige Ringe mit zwei Heteroatomen wie z.B. Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl oder Thiazol-5-yl;
   5-gliedrige Ringe mit 3 Heteroatomen wie z.B. 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadiazolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,3,2-Dioxathiolan-4-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl oder 1,2,4-Triazol-3-yl; 5-gliedrige Ringe mit 4 Heteroatomen wie z.B.
   Tetrazol-5-yl,
   6-gliedrige Ringe mit 1 Heteroatome wie z.B.
   Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, Pyridin-2-yl, Pyridin-3-yl oder Pyridin-4-yl;
   6-gliedrige Ringe mit 2 Heteroatomen wie z.B.
   1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl, Pyridazin-3-yl, Pyridazin-4-yl, Pyrimidin-2-yl, Pyrimidin-4-yl, Pyrimidin-5-yl oder Pyrazin-2-yl;
   6-gliedrige Ringe mit 3 Heteroatomen wie z.B.
   1,3,5-Triazin-2-yl, 1,2,4-Triazin-3-yl, 1,2,4-Triazin-5-yl oder 1,2,4-Triazin-6-yl;
   6-gliedrige Ringe mit 4 Heteroatomen wie z.B.
   1,2,4,5-Tetrazin-3-yl;
   wobei ggf. der Schwefel der genannten Heterocyclen zu S=O oder S(=O)₂ oxidiert sein kann;
   und wobei mit einem ankondensierten Phenylring oder mit einem C₃-C₆-Carbocyclus oder mit einem weiteren 5- bis 6-gliedrigen Heterocyclus ein bicyclisches Ringsystem ausgebildet werden kann.
- N-gebundenes Heterocyclyl: ein gesättigter, partiell gesättigter oder ungesättigter 5- oder 6-gliedriger N-gebundener heterocyclischer Ring, der mindestens einen Stickstoff und gegebenenfalls ein bis drei gleiche oder verschiedene Heteroatome, ausgewählt aus folgender Gruppe: Sauerstoff, Schwefel oder Stickstoff enthält, also z.B.
   N-gebundene 5-gliedrige Ringe mit 1 Heteroatom wie z.B.
   Tetrahydropyrrol-1-yl, 2,3-Dihydro-1H-pyrrol-1-yl, 2,5-Dihydro-1H-pyrrol-1-yl oder Pyrrol-1-yl;
   N-gebundene 5-gliedrige Ringe mit 2 Heteroatomen wie z.B.
   Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, Pyrazol-1-yl oder imidazol-1-yl;
   N-gebundene 5-gliedrige Ringe mit 3 Heteroatomen wie z.B.
   1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl, 1,2,3-Triazol-1-yl oder 1,2,4-Triazol-1-yl;
   N-gebundene 5-gliedrige Ringe mit 4 Heteroatomen wie z.B.
   Tetrazol-1-yl;
   sowie N-gebundene 6-gliedrige Ringe mit 1 Heteroatome wie z.B.
   Piperidin-1-yl, 1,2,3,4-Tetrahydropyridin-1-yl, 1,2,5,6-Tetrahydropyridin-1-yl, 1,4-Dihydropyridin-1-yl oder 1,2-Dihydropyridin-1-yl;
   N-gebundene 6-gliedrige Ringe mit 2 Heteroatomen wie z.B.
   Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazinoxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihydro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl, sowie N-gebundene cyclische Imide wie:
   Phthalsäureimid, Tetrahydrophthalsäureimid, Succinimid, Maleinimid oder Glutarimid, sowie 4-Oxo-1,4-dihydropyridin-1-yl;

Alle Phenylringe bzw. Heterocyclylreste sowie alle Phenylkomponenten in Phenyl-C₁-C₆-alkyl, Phenylcarbonyl-C₁-C₆-alkyl, Phenoxy, Phenylthio, Phenylcarbonyl, Phenylalkenylcarbonyl, Phenoxycarbonyl, Phenoxyalkylcarbonyl, Phenylaminocarbonyl und N-(C₁-C₆-Alkyl)-N-phenylaminocarbonyl bzw. Heterocyclylkomponenten in Heterocyclyl-C₁-C₆-alkyl, Heterocyclylcarbonyl-C₁-C₆-alkyl, Heterocyclyoxy, Heterocyclylthio, Heterocyclylcarbonyl, Heterocyclylalkenylcarbonyl, Heterocycloxyalkylcarbonyl, Heterocyclyloxycarbonyl, Heterocyclylaminocarbonyl und N(C₁-C₆-Alkyl)-N-heterocyclylaminocarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

Die erfindungsgemäßen Verbindungen der Formel I mit R⁴ = IIa werden als Verbindungen der Formel Ia sowie Verbindungen der Formel I mit R⁴ = IIb als Ib bezeichnet.

Besonders zu betonen sind die Verbindungen der Formel I, wobei
- R⁷: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₂₀-Alkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, C₁-C₄-Alkoxy, C₁-C₄-Alkylthio, Di-(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Heterocyclyloxycarbonyl, Phenoxythiocarbonyl, Heterocyclyloxythiocarbonyl, Phenoxy-C₁-C₆-alkylcarbonyl, Heterocyclyloxy-C₁-C₆-alkylcarbonyl, Phenylaminocarbonyl, N-(C₁-C₆-Alkyl)-N-(phenyl)-aminocarbonyl, Heterocyclylaminocarbonyl, N-(C₁-C₆-Alkyl)-N-(heterocyclyl)-aminocarbonyl, Phenyl-C₂-C₆-alkenylcarbonyl oder Heterocyclyl-C₂-C₆-alkenylcarbonyl, wobei der Phenyl- und der Heterocyclyl-Rest der 20 letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl oder C₁-C₆-Halogenalkylsulfonyl;
- R², R³: Wasserstoff, C₁-C₆-Alkyl oder Halogen;
- R⁴: eine Verbindung IIa oder IIb 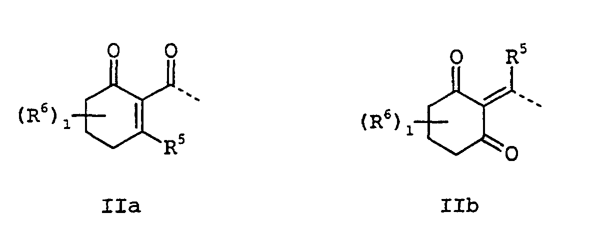
wobei
- R⁵: Halogen, OR⁷, SR⁷, SO₂R^{8,} OSO₂R⁸, OPOR⁸R⁹, OPR⁸R⁹, OPSR⁸R⁹, NR¹⁰R¹¹, ONR¹³R¹², N-gebundenes Heterocyclyl oder O-(N-gebundenes Heterocyclyl), wobei der Heterocyclyl-Rest der beiden letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R⁶: Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio, C₁-C₆-Halogenalkylthio, C₁-C₆-Alkylsulfonyl, C₁-C₆-Halogenalkylsulfonyl, C₁-C₆-Alkylcarbonyl, C₁-C₆-Halogenalkylcarbonyl, C₁-C₆-Alkoxycarbonyl oder C₁-C₆-Halogenalkoxycarbonyl;
oder
- zwei Reste R⁶,: die am gleichen Kohlenstoff gebunden sind, bilden gemeinsam eine -O-(CH₂)ₘ-O-, -O-(CH₂)ₘ-S-, -S-(CH₂)ₘ-S-, -O-(CH₂)ₙ- oder -S-(CH₂)ₙ-Kette, die durch einen bis drei Reste aus folgender Gruppe substituiert sein kann: Halogen, Cyano, C₁-C₄-Alkyl, C₁-C₄-Halogenalkyl oder C₁-C₄-Alkoxycarbonyl;
oder
- zwei Reste R⁶,: die am gleichen Kohlenstoff gebunden sind, bilden gemeinsam eine -(CH₂)ₚ-Kette, die durch Sauerstoff oder Schwefel unterbrochen sein kann und/oder durch einen bis vier Reste aus folgender Gruppe substituiert sein kann:
Halogen, Cyano, C₁-C₄-Alkyl, C₁-C₄-Halogenalkyl oder C₁-C₄-Alkoxycarbonyl;
oder
- zwei Reste R⁶,: die am gleichen Kohlenstoff gebunden sind, bilden gemeinsam mit diesem Kohlenstoff eine Carbonylgruppe aus;
oder
- zwei Reste R⁶,: die an verschiedenen Kohlenstoffen gebunden sind, bilden gemeinsam eine - (CH₂)ₙ-Kette, die durch einen bis drei Reste aus folgender Gruppe substituiert sein kann:
Halogen, C₁-C₆-Alkyl, C₁-C₆-Alkoxy, Hydroxy oder C₁-C₆-Alkoxycarbonyl;
- R⁷: C₁-C₆-Alkyl, C₃-C₆-Alkenyl, C₃-C₆-Halogenalkenyl, C₃-C₆-Alkinyl, C₁-C₂₀-Alkylcarbonyl, C₂-C₆-Alkenylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, (C₁-C₂₀-Alkylthio)carbonyl (besonders bevorzugt (C₁-C₆-Alkylthio)carbonyl), C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können: Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Heterocyclyloxycarbonyl, Phenoxythiocarbonyl, Heterocyclyloxythiocarbonyl, Phenoxy-C₁-C₆-alkylcarbonyl, Heterocyclyloxy-C₁-C₆-alkylcarbonyl, Phenyl-C₂-C₆-alkenylcarbonyl oder Heterocyclyl-C₂-C₆-alkenylcarbonyl, wobei der Phenyl- und der Heterocyclyl-Rest der 16 letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R⁸, R⁹: C₁-C₆-Alkyl, C₃-C₆-Alkenyl, C₃-C₆-Halogenalkenyl, C₃-C₆-Cycloalkyl, Hydroxy, C₁-C₆-Alkoxy, Di-C₁-C₆-alkylamino, oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁰: C₁-C₆-Alkyl, C₃-C₆-Alkenyl, C₃-C₆-Halogenalkenyl, C₃-C₆-Cycloalkyl, C₁-C₆-Alkoxy, C₃-C₆-Alkenyloxy oder Di-(C₁-C₆-Alkyl)-amino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹¹, R¹²: C₁-C₆-Alkyl oder C₃-C₆-Alkenyl;
- l: 0 bis 6;
- m: 2 bis 4;
- n: 1 bis 5;
- p: 2 bis 5;

Besonders bevorzugt sind Verbindungen der Formel I, wobei die Variablen folgende Bedeutungen haben, und zwar für sich allein oder in Kombination:
- R¹: Halogen, C₁-C₆-Alkyl, C₁-C₆-Halogenalkyl, C₁-C₆-Alkoxy, C₁-C₆-Alkylthio oder C₁-C₆-Alkylsulfonyl;
insbesondere Halogen wie Fluor oder Chlor, C₁-C₆-Alkyl wie Methyl oder Ethyl oder C₁-C₆-Halogenalkyl wie Difluormethyl oder Trifluormethyl;
besonders bevorzugt Fluor, Chlor, Methyl, Difluormethyl oder Trifluormethyl;
- R²: Wasserstoff oder C₁-C₆-Alkyl, wie Methyl oder Ethyl;
insbesondere Wasserstoff oder Methyl;
- R³: Wasserstoff oder C₁-C₆-Alkyl; insbesondere Wasserstoff;
- R⁴: eine Verbindung IIa oder IIb 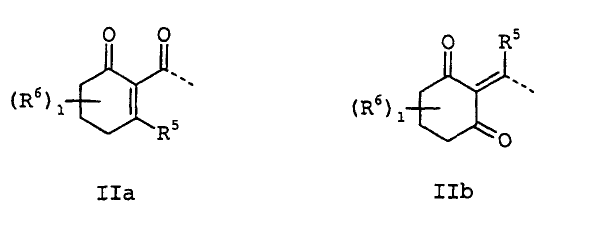
wobei
- R⁵: Halogen, OR⁷, SR⁷, SO₂R^{8,} OSO₂R⁸, NR¹⁰R¹¹, ONR¹¹R¹², N-gebundenes Heterocyclyl oder O-(N-gebundenes Heterocyclyl), wobei der Heterocyclyl-Rest der beiden letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R⁶: Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio oder C₁-C₆-Halogenalkylthio;
oder
- zwei Reste R⁶,: die am gleichen Kohlenstoff gebunden sind, bilden gemeinsam mit diesem Kohlenstoff eine Carbonylgruppe aus;
- R⁷: C₁-C₆-Alkyl, C₃-C₆-Alkenyl, C₃-C₆-Halogenalkenyl, C₃-C₆-Alkinyl, C₁-C₂₀-Alkylcarbonyl, C₃-C₆-Cycloalkylcarbonyl, C₁-C₆-Alkoxycarbonyl, C₃-C₆-Alkenyloxycarbonyl, C₁-C₆-Alkylaminocarbonyl, C₃-C₆-Alkenylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, Di-(C₁-C₆-alkyl)-aminothiocarbonyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Heterocyclyloxycarbonyl, Phenoxythiocarbonyl, Heterocyclyloxythiocarbonyl, Phenoxy-C₁-C₆-alkylcarbonyl oder Heterocyclyloxy-C₁-C₆alkylcarbonyl, wobei der Phenyl- und der Heterocyclyl-Rest der 14 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R⁸: C₁-C₆-Alkyl, C₃-C₆-Alkenyl, C₃-C₆-Halogenalkenyl, C₃-C₆-Cycloalkyl, Hydroxy, C₁-C₆-Alkoxy, Di-C₁-C₆-alkylamino oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁰: C₁-C₆-Alkyl, C₃-C₆-Alkenyl, C₃-C₆-Halogenalkenyl, C₃-C₆-Cycloalkyl, C₁-C₆-Alkoxy, C₃-C₆-Alkenyloxy oder Di-(C₁-C₆-Alkyl)-amino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹¹, R¹²: C₁-C₆-Alkyl oder C₃-C₆-Alkenyl;
- l: 0 bis 6;

Ebenso besonders bevorzugt sind die Verbindungen der Formel I, wobei die Variablen folgende Bedeutung haben, und zwar für sich allein oder in Kombination:
- R¹: Halogen, C₁-C₆-Alkyl, C₁-C₆-Halogenalkyl, C₁-C₆-Alkoxy, C₁-C₆-Alkylthio, Heterocyclyloxy oder Phenylthio, wobei die zwei letztgenannten Reste partiell oder vollständig halogeniert sein können und/oder einen bis drei der nachfolgend genannten Substituenten tragen können:
Nitro, Cyano, C₁-C₄-Alkyl, C₁-C₄-Halogenalkyl, C₁-C₄-Alkoxy oder C₁-C₄-Halogenalkoxy;
besonders bevorzugt Halogen, C₁-C₆-Alkyl oder C₁-C₆-Alkylthio;
- R²: Wasserstoff, C₁-C₆-Alkyl oder C₁-C₆-Halogenalkyl; besonders bevorzugt Wasserstoff;
- R³: Wasserstoff;
- R⁵: Halogen, OR⁷, SR⁷, SOR⁸, SO₂R⁸, OSO₂R⁸, OPR⁸R⁹, OPOR⁸R⁹, OPSR⁸R⁹, NR¹⁰R¹¹ oder N-gebundenes Heterocyclyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
besonders bevorzugt Halogen, OR⁷, NR¹⁰R¹¹ oder N-gebundenes Heterocyclyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
insbesondere bevorzugt Fluor, OR⁷, NR¹⁰R¹¹ oder N-gebundenes Heterocyclyl ausgewählt aus der Gruppe:
4-Morpholinyl oder 4-Oxo-1,4-dihydropyrid-1-yl;
- R⁶: C₁-C₆-Alkyl
oder zwei Reste R⁶, die am gleichen Kohlenstoff gebunden sind, bilden gemeinsam mit diesem Kohlenstoff eine Carbonylgruppe aus;
- R⁷: C₁-C₆-Alkyl, C₁-C₂₀-Alkylcarbonyl, C₁-C₆-Alkoxycarbonyl, (C₁-C₂₀-Alkylthio)carbonyl, N,N-Di(C₁-C₆-alkyl)aminocarbonyl, Phenyl, Phenylcarbonyl oder Phenoxy-C₁-C₆-alkylcarbonyl, wobei der Phenylrest der drei letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
besonders bevorzugt C₁-C₆-Alkyl, C₁-C₂₀-Alkylcarbonyl, C₁-C₆-Alkoxycarbonyl, (C₁-C₆-Alkylthio)carbonyl, N,N-Di(C₁-C₆-alkyl)aminocarbonyl, Phenyl, Phenylcarbonyl oder Phenoxy-C₁-C₆-alkylcarbonyl, wobei der Phenylrest der drei letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
insbesondere bevorzugt C₁-C₂₀-Alkylthiocarbonyl; außerordentlich bevorzugt C₁-C₆-Alkylthiocarbonyl;
- R⁸,R⁹: C₁-C₆-Alkyl, C₁-C₆-Alkoxy, Di(C₁-C₆-alkyl)amino oder Phenyl, wobei der letztgenannten Reste partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁰: C₁-C₆-Alkyl oder C₁-C₆-Alkoxy;
- R¹¹: C₁-C₆-Alkyl;
- l: 0 bis 6;
besonders bevorzugt 4 bis 6;
insbesondere 6;

Ebenso besonders bevorzugt sind Verbindungen der Formel I,
wobei
- R⁶: Nitro, Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio, C₁-C₆-Halogenalkylthio, C₁-C₆-Alkylsulfinyl, C₁-C₆-Halogenalkylsufinyl, C₁-C₆-Alkylsulfonyl, C₁-C₆-Halogenalkylsulfonyl, C₁-C₆-Alkylcarbonyl, C₁-C₆-Halogenalkylcarbonyl, C₁-C₆-Alkoxycarbonyl oder C₁-C₆-Halogenalkoxycarbonyl;
bedeutet
oder
- zwei Reste R⁶,: die am gleichen Kohlenstoff gebunden sind, bilden gemeinsam eine -O-(CH₂)ₘ-O-, -O-(CH₂)ₘ-O-, -O-(CH₂)ₘ-S-, -S-(CH₂)ₘ-S-, -O-(CH₂)ₙ- oder -S-(CH₂)ₙ-Kette, die durch einen bis drei Reste aus folgender Gruppe substituiert sein kann:
Halogen, Cyano, C₁-C₄-Alkyl, C₁-C₄-Halogenalkyl oder C₁-C₄-Alkoxycarbonyl;
oder
- zwei Reste R⁶,: die am gleichen Kohlenstoff gebunden sind, bilden eine -(CH₂)ₚ-Kette, die durch Sauerstoff oder Schwefel unterbrochen sein kann und/oder durch einen bis vier Reste aus folgender Gruppe substituiert sein kann: Halogen, Cyano, C₁-C₄-Alkyl, C₁-C₄-Halogenalkyl oder C₁-C₄-Alkoxycarbonyl;
oder
- zwei Reste R⁶,: die am gleichen Kohlenstoff gebunden sind, bilden gemeinsam mit diesem Kohlenstoff eine Carbonylgruppe aus.

Insbesondere bevorzugt sind Verbindungen der Formel I, wobei
- R⁶: Nitro, Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio, C₁-C₆-Halogenalkylthio, C₁-C₆-Alkylsulfinyl, C₁-C₆-Halogenalkylsufinyl, C₁-C₆-Alkylsulfonyl, C₁-C₆-Halogenalkylsulfonyl, C₁-C₆-Alkylcarbonyl, C₁-C₆-Halogenalkylcarbonyl, C₁-C₆-Alkoxycarbonyl oder C₁-C₆-Halogenalkoxycarbonyl;
bedeutet
oder
- zwei Reste R⁶,: die am gleichen Kohlenstoff gebunden sind, bilden gemeinsam mit diesem Kohlenstoff eine Carbonylgruppe aus.

Ebenso insbesondere bevorzugt sind die Verbindungen der Formel I, wobei
- R⁵: Halogen oder (C₁-C₂₀-Alkylthio)carbonyloxy; besonders bevorzugt Fluor oder (C₁-C₆-Alkylthio)carbonyloxy;
bedeutet.

Ebenso insbesondere bevorzugt sind die Verbindungen der Formel I, wobei
- R⁵: NR¹⁰R¹¹ oder N-gebundenes Heterocyclyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
bedeutet.

Ebenso insbesondere bevorzugt sind die Verbindungen der Formel I, wobei R⁴ folgende Bedeutung hat:

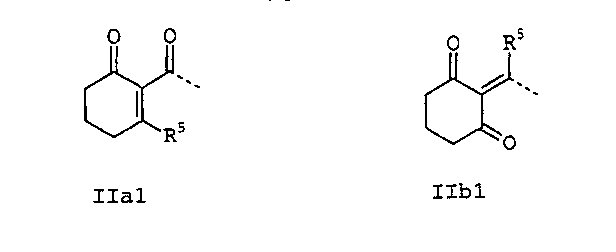

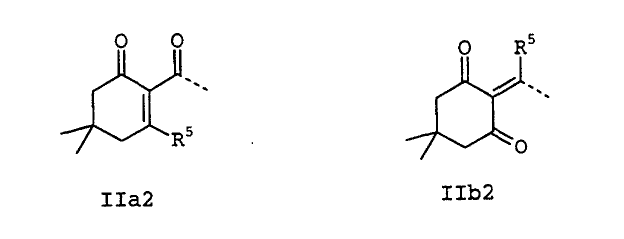

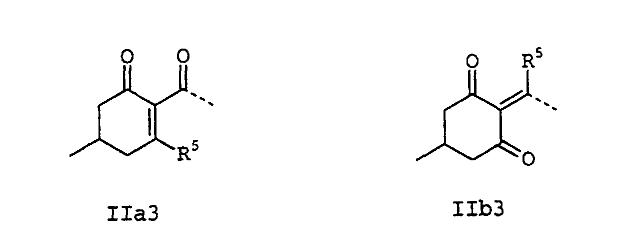

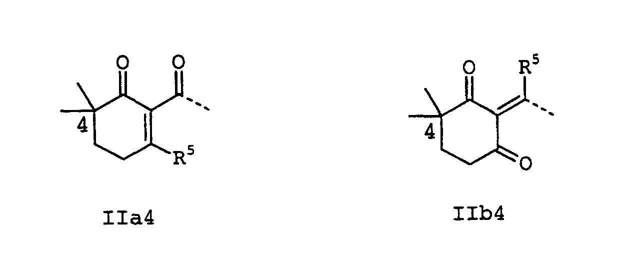

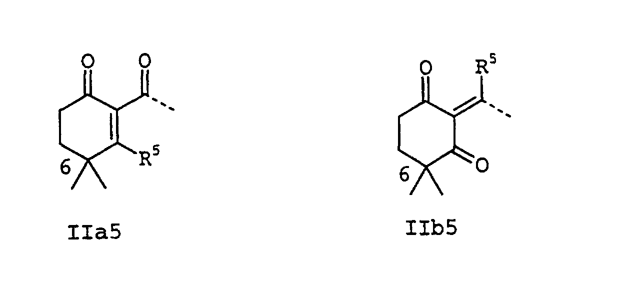

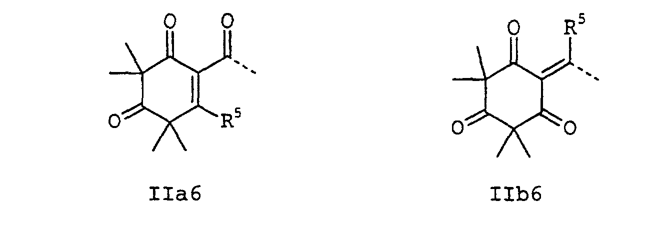

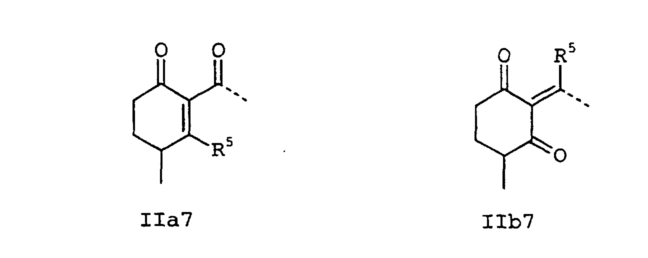

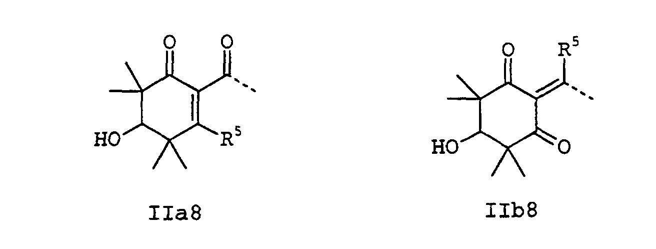

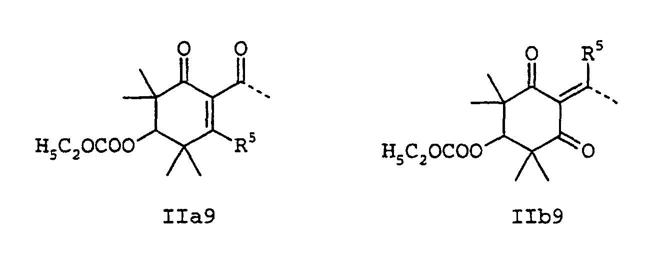

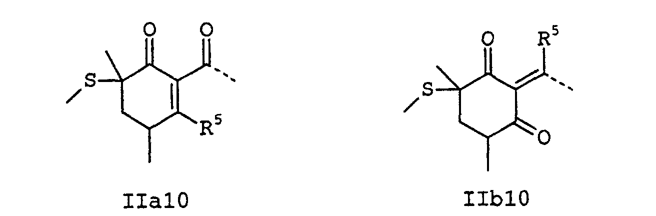

Insbesonderst bevorzugt sind die Verbindungen der Formel I, wobei
- R⁵: NR¹⁰R¹¹ oder Tetrahydropyrrol-1-yl, 2,3-Dihydro-1H-pyrrol-1-yl, 2,5-Dihydro-1H-pyrrol-1-yl, Pyrrol-1-yl, Tetrahydropyrazol-1-yl, Tetrahydroisoxazol-2-yl, Tetrahydrothiazol-2-yl, Tetrahydroimidazol-1-yl, Tetrahydrooxazol-3-yl, Tetrahydrothiazol-3-yl, Pyrazol-1-yl, Imidazol-1-yl, 1,2,4-Triazol-1-yl, Tetrazol-1-yl, Piperidin-1-yl, 4-Oxo-1,4-dihydro-1-pyridyl, Hexahydropyrimidin-1-yl, Hexahydropyrazin-1-yl, Tetrahydro-1,4-oxazin-4-yl, Tetrahydro-1,2-oxazin-2-yl, Succinimid, Maleinimid oder Glutarimid, wobei die genannten Heterocyclen partiell oder vollständig halogeniert sein können und/oder einen bis drei der folgenden Reste tragen können:
Nitro, Cyano, C₁-C₄-Alkyl, wie Methyl oder Ethyl, C₁-C₄-Halogenalkyl wie Chlormethyl, Difluormethyl oder Trifluormethyl, C₁-C₄-Alkoxy wie Methoxy oder Ethoxy oder C₁-C₄-Halogenalkoxy wie Difluormethoxy oder Trifluormethoxy;
- R¹⁰: C₁-C₆-Alkoxy;
bedeuten.

Außerordentlich bevorzugt sind die Verbindungen der Formel Ia1 und Ibl (≡ I mit l = 0), insbesondere die Verbindungen Ia1.1 bis Ia1.456 und die Verbindungen Ib1.1 bis Ib1.456, wobei die Restedefinitionen R¹ bis R⁵ und l nicht nur in Kombination miteinander, sondern auch jeweils für sich allein betrachtet für die erfindungsgemäßen Verbindungen eine bevorzugte Bedeutung haben.

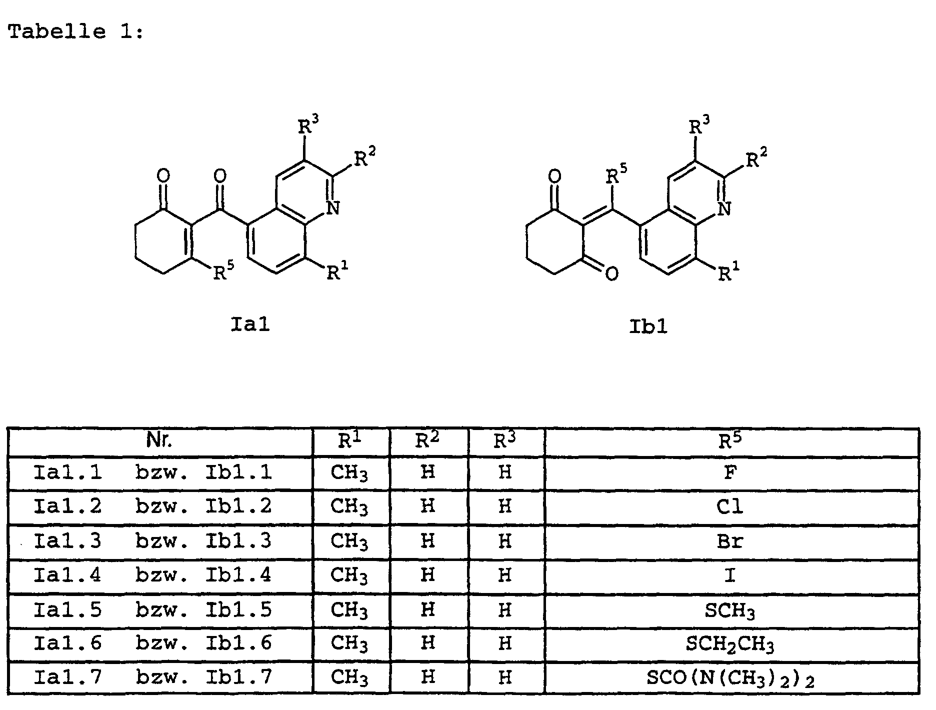

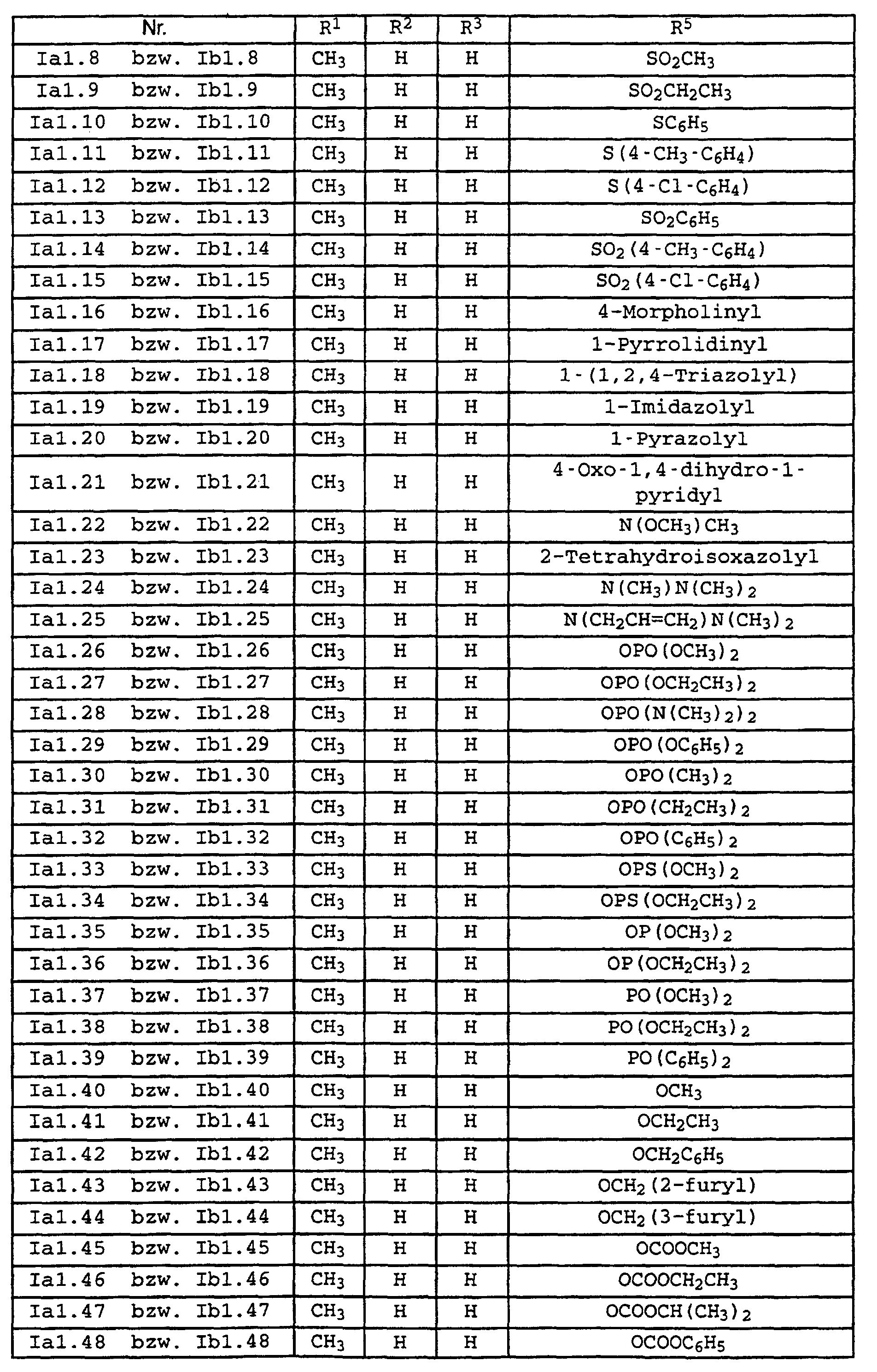

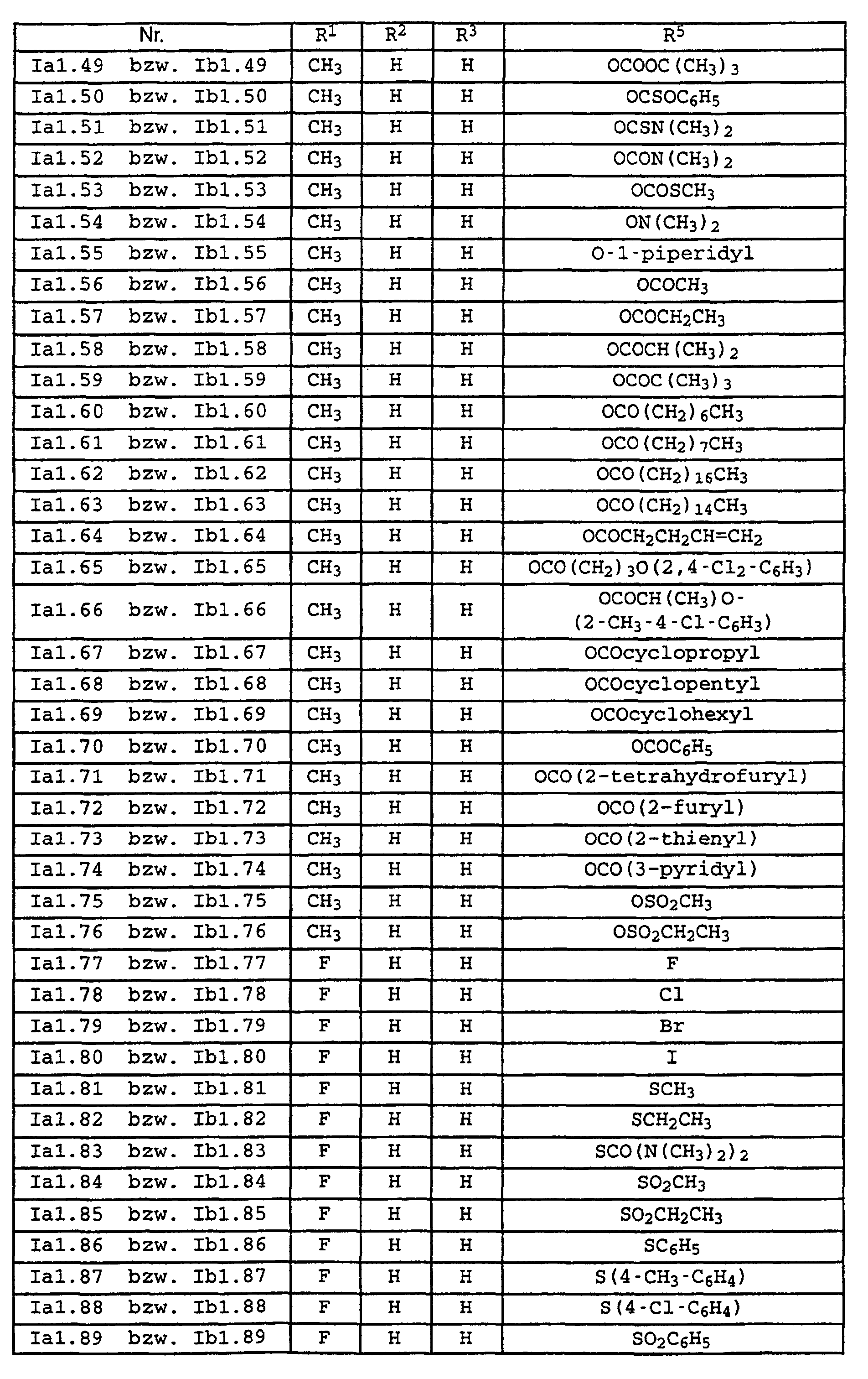

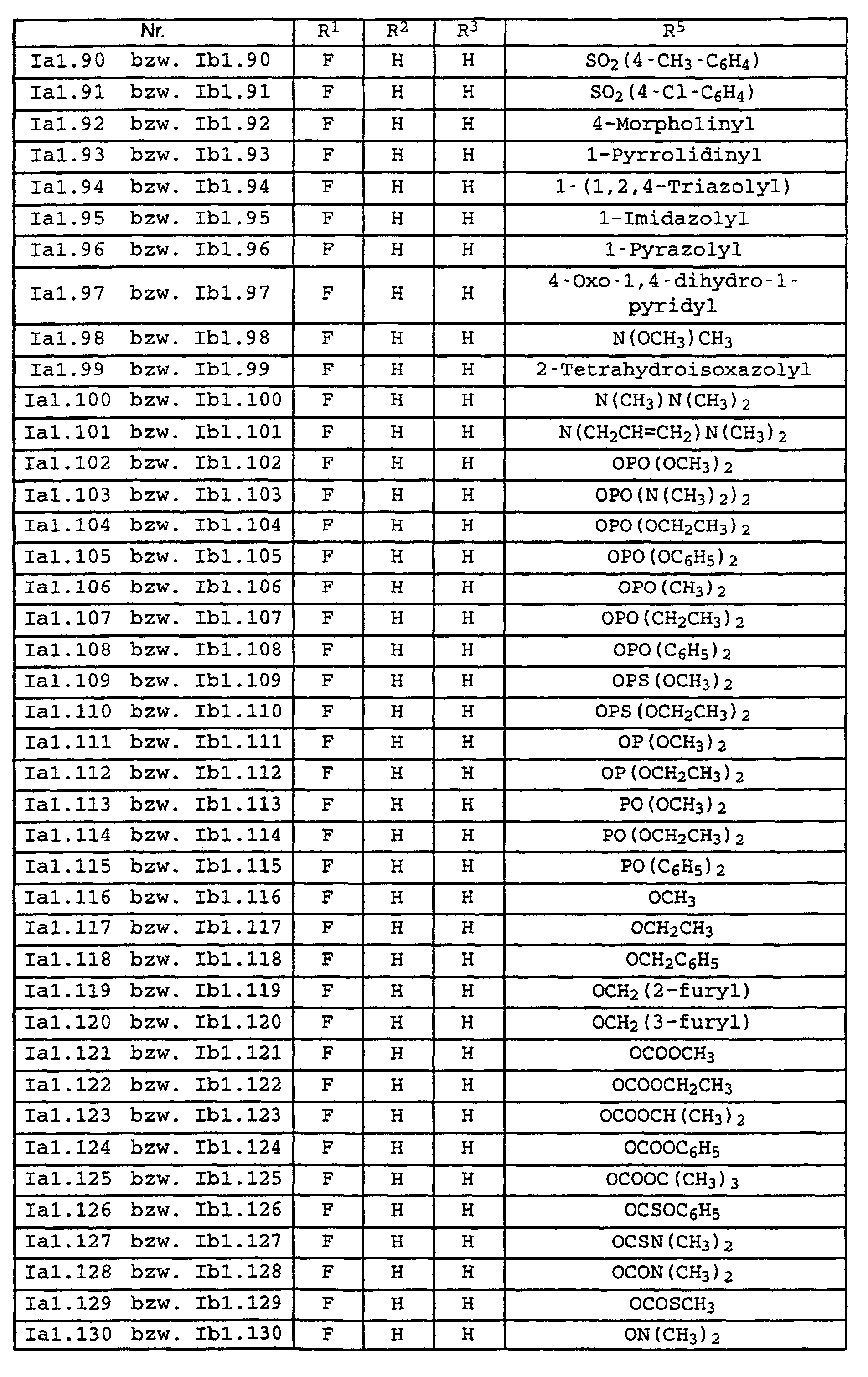

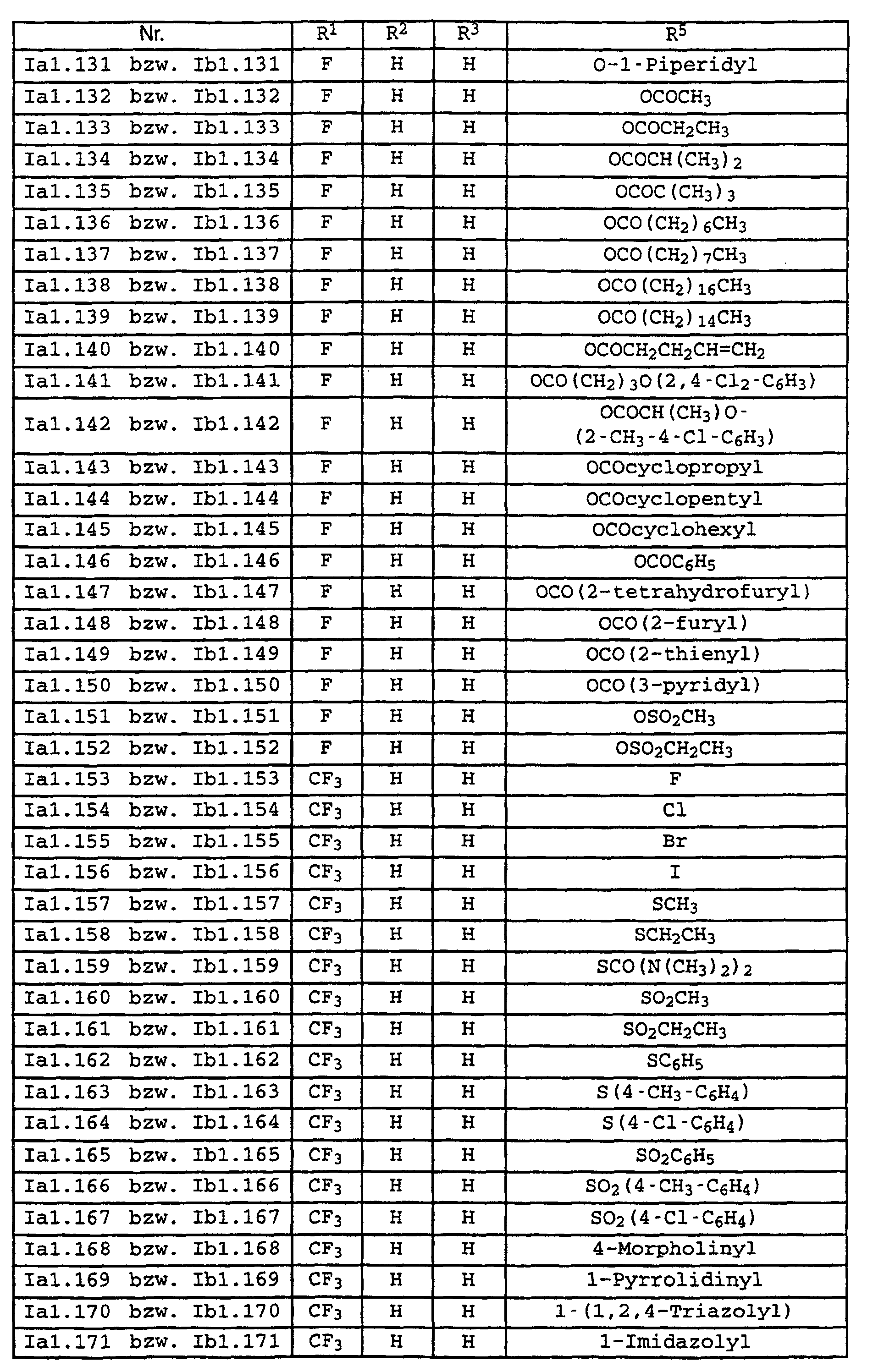

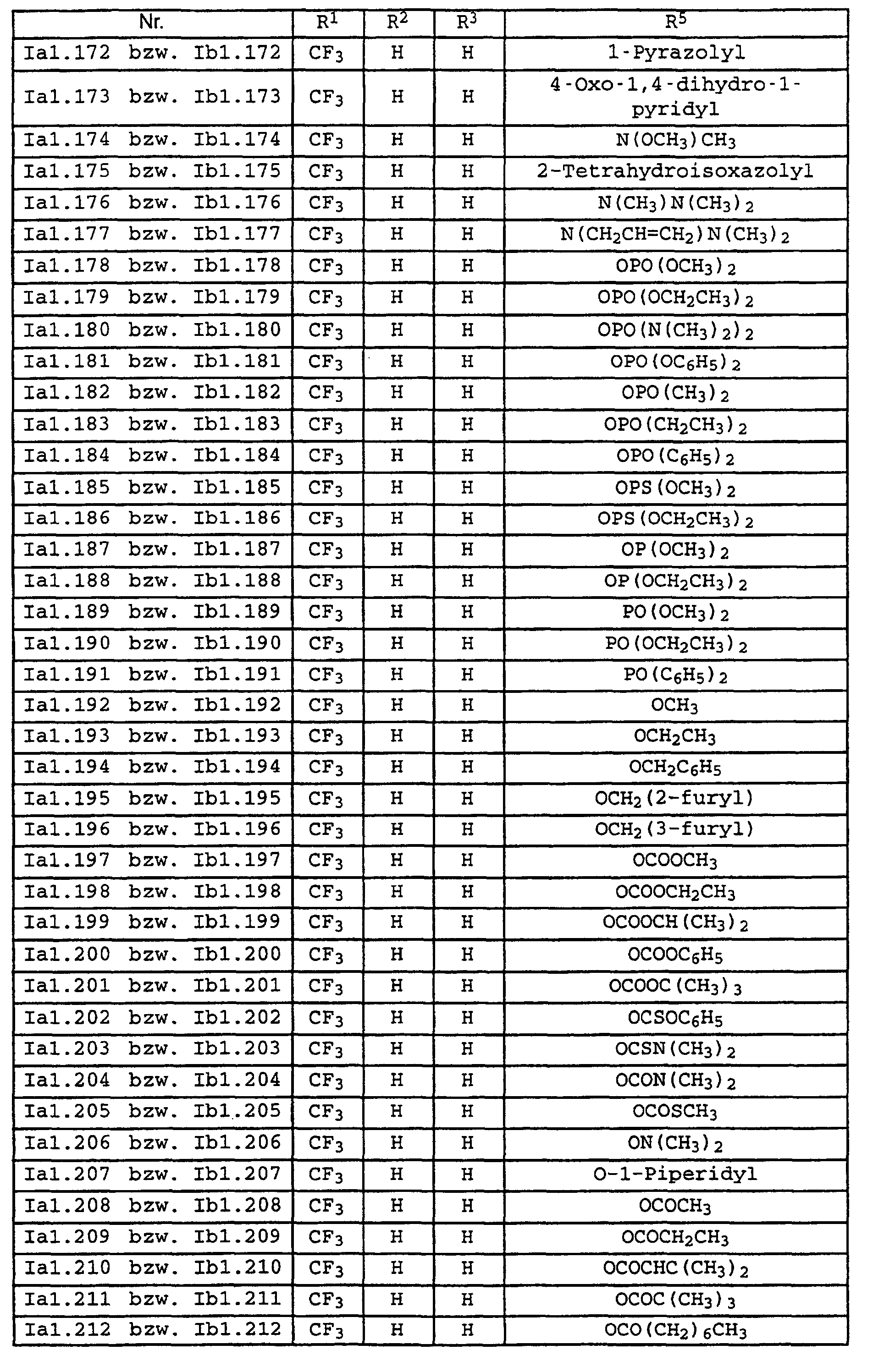

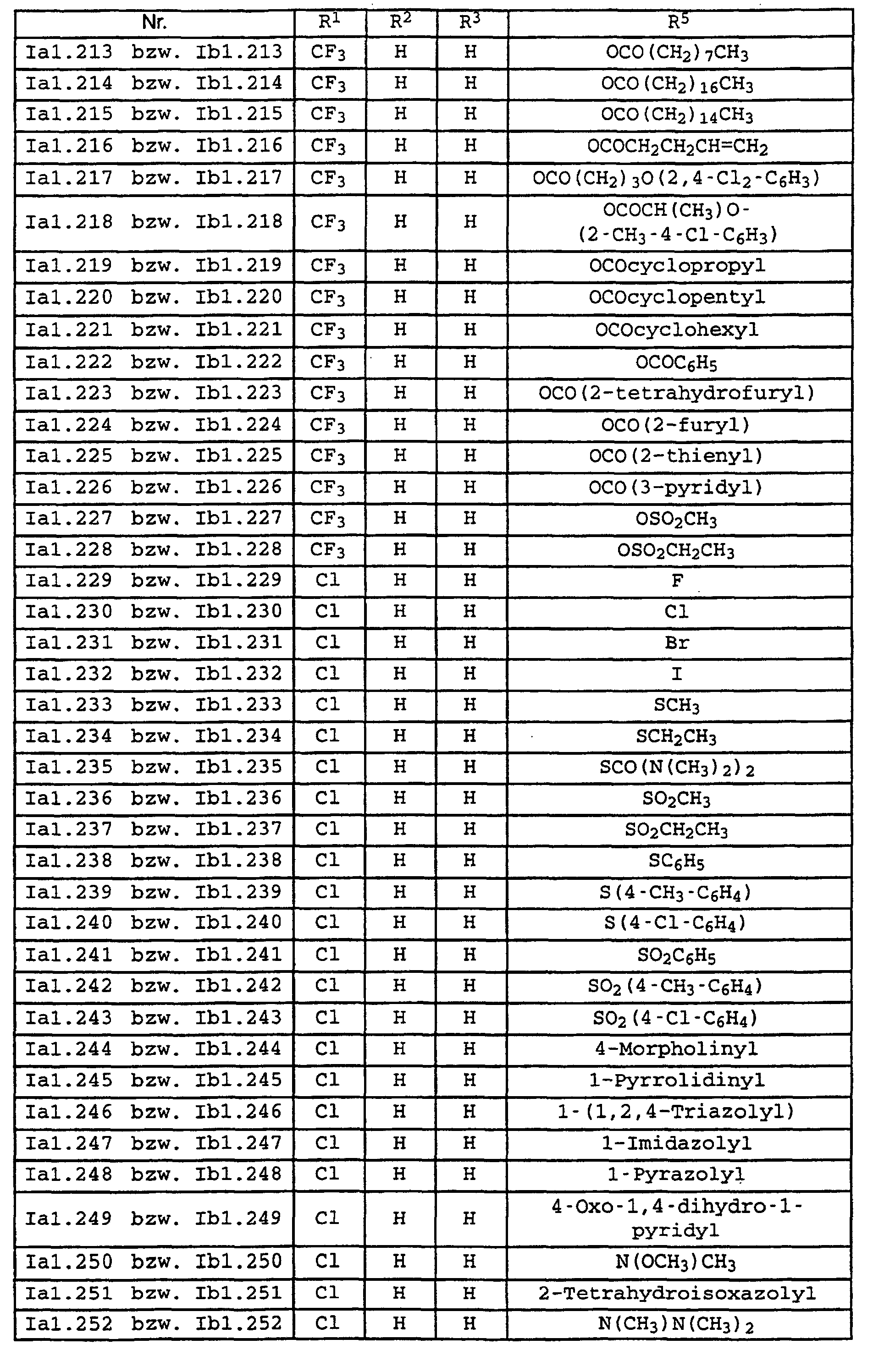

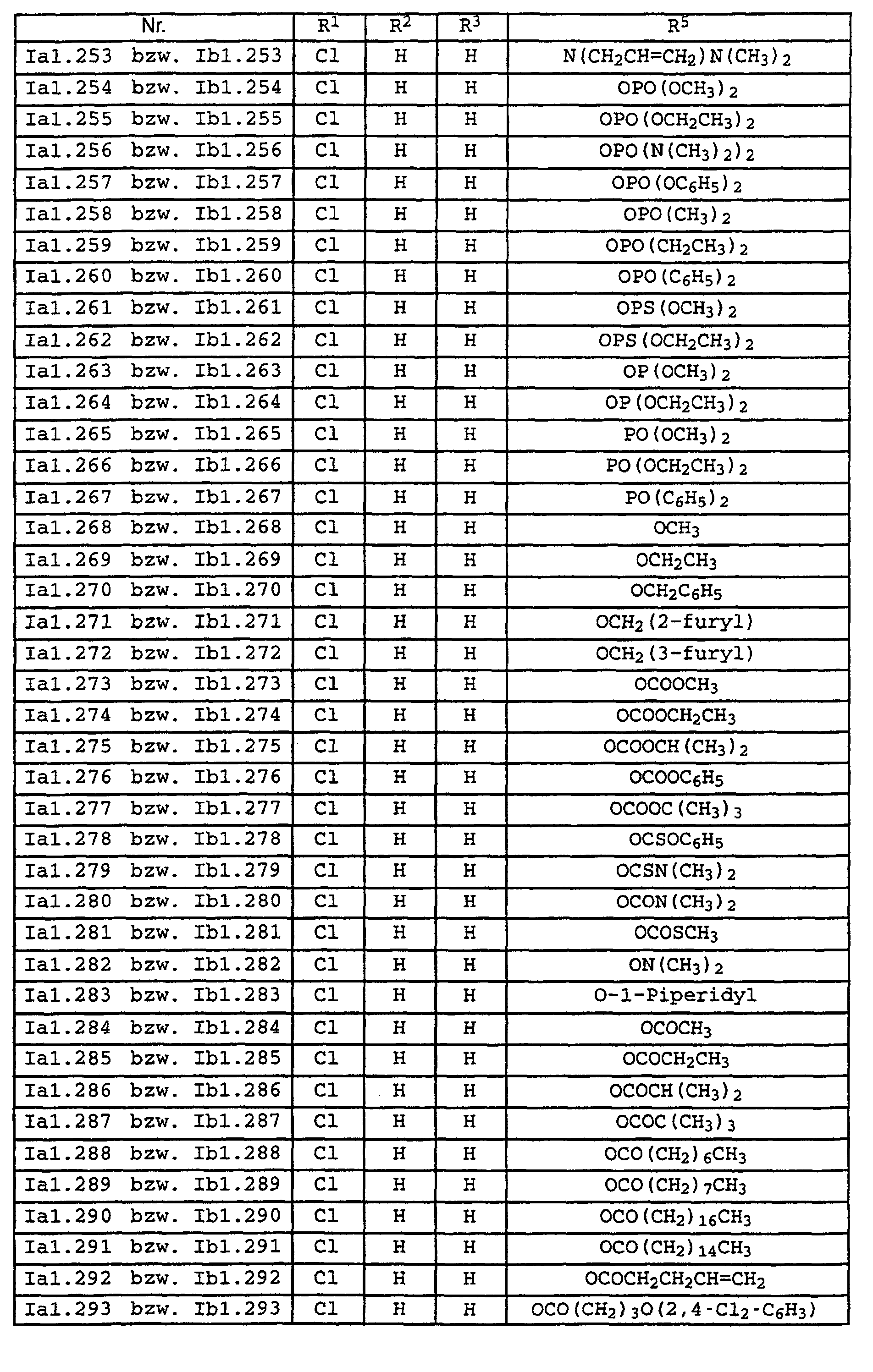

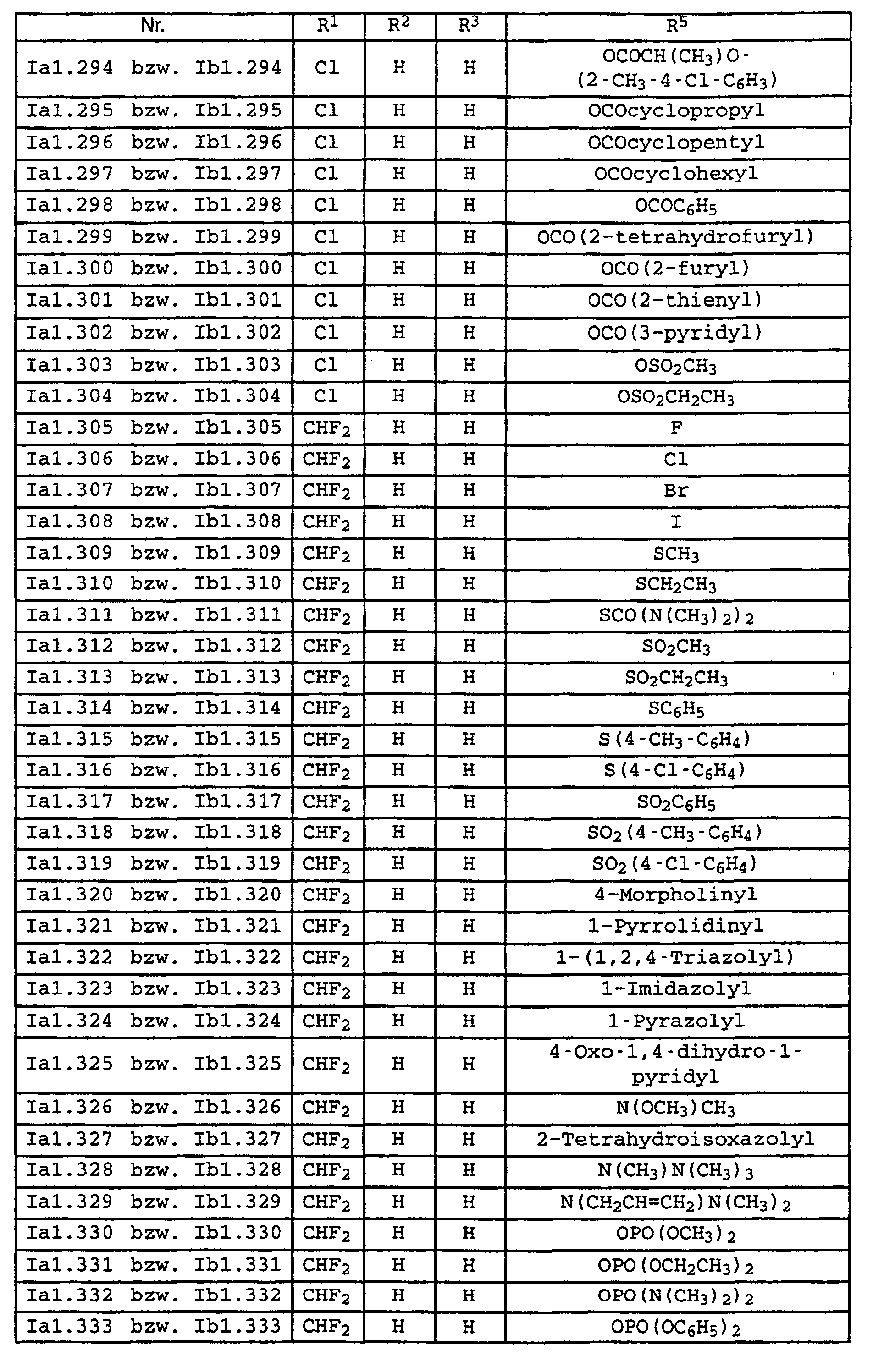

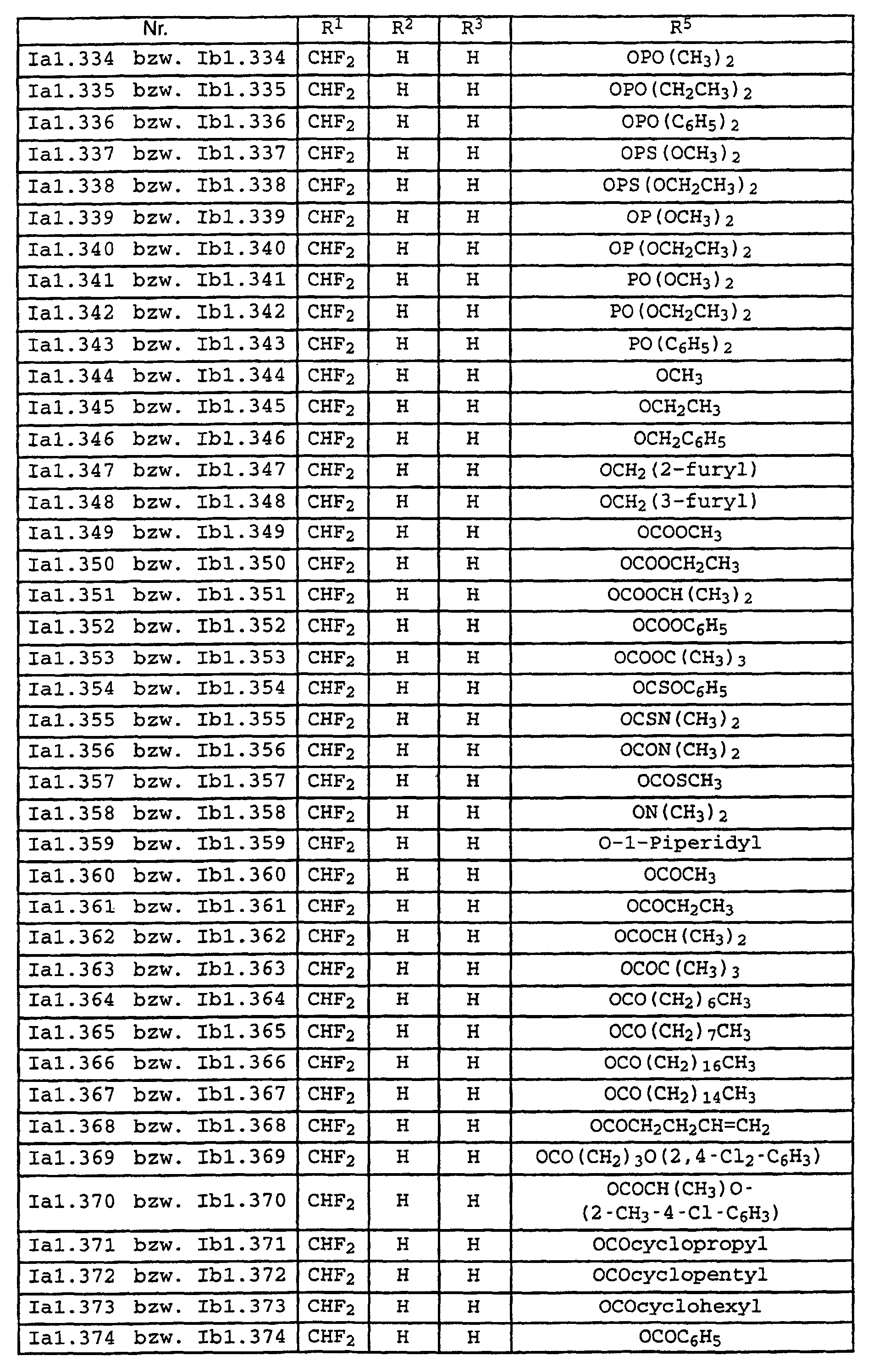

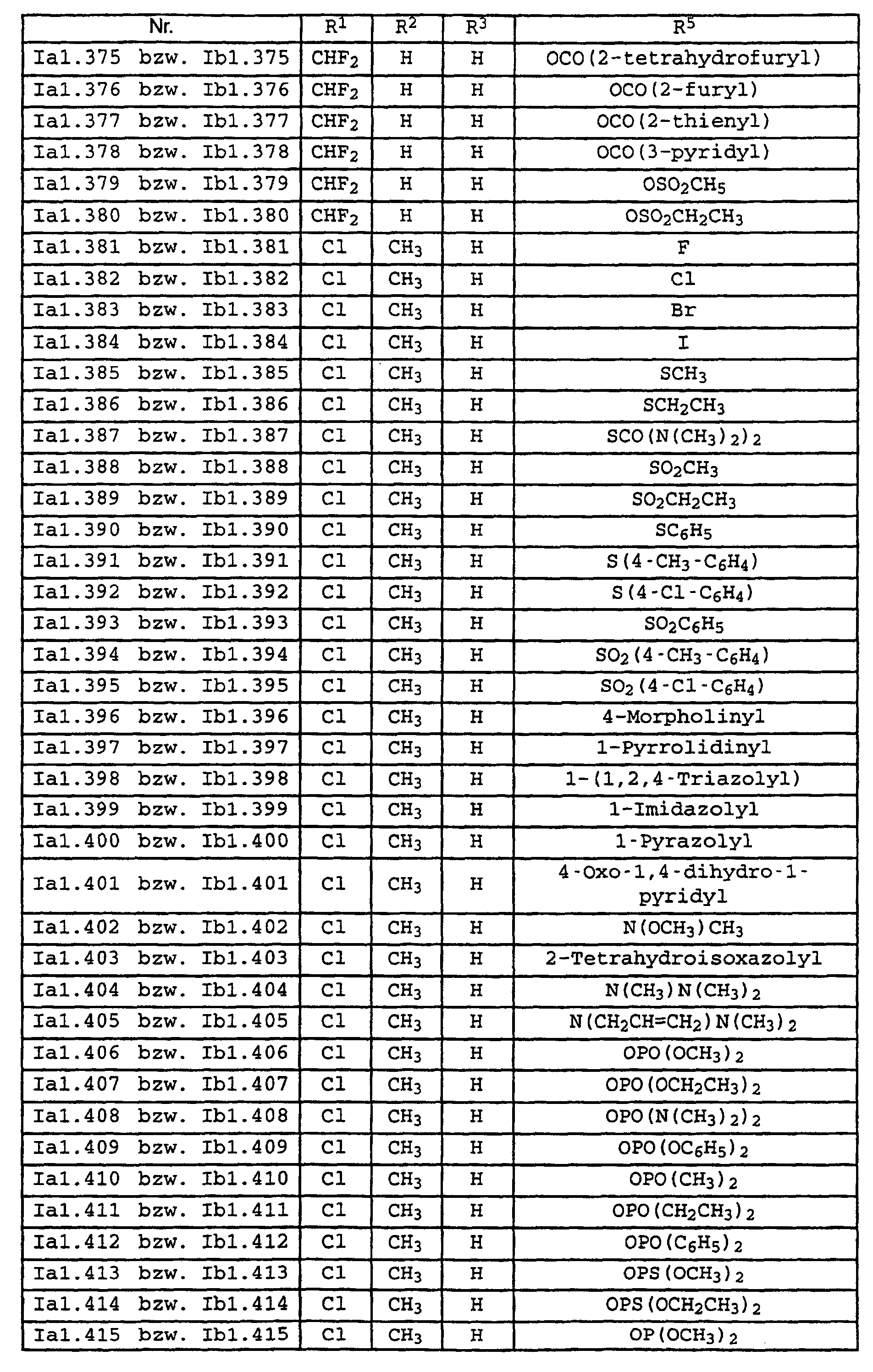

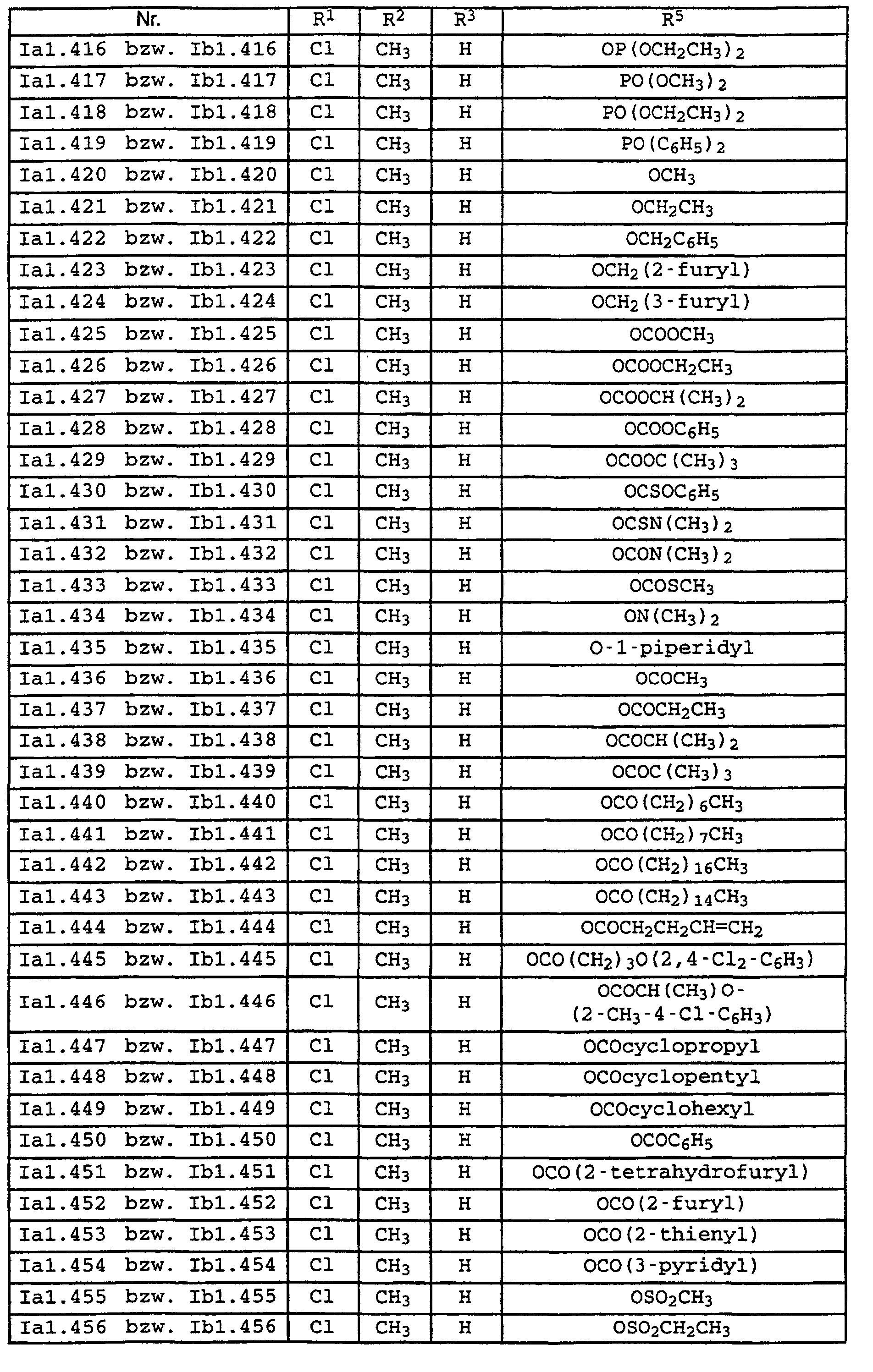

Desweiteren sind folgende Cyclohexenonchinolinoyl-Derivate der Formel I außerordentlich bevorzugt:
- Die Verbindungen der Formel Ia2 und Ib2, insbesondere die Verbindungen Ia2.1 bis Ia2.456 und die Verbindungen Ib2.1 bis Ib2.456, die sich von den Verbindungen Ia1.1 bis Ia1.456 bzw. Ib1.1 bis Ib1.456 dadurch unterscheiden, daß (R⁶)₁ "5,5-Dimethyl" bedeutet. 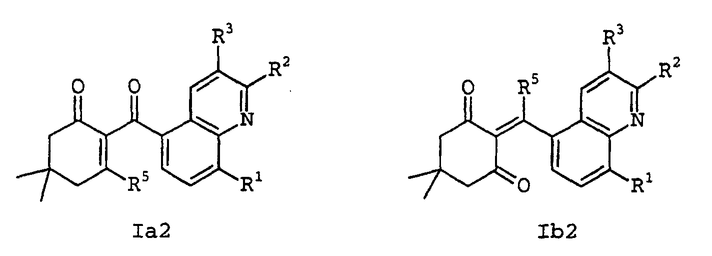
- Die Verbindungen der Formel Ia3 und Ib3, insbesondere die Verbindungen Ia3.1 bis Ia3.456 und die Verbindungen Ib3.1 bis Ib3.456, die sich von den Verbindungen Ia1.1 bis Ial.456 bzw. Ib1.1 bis Ib1.456 dadurch unterscheiden, daß (R⁶)₁ "5-Methyl" bedeutet. 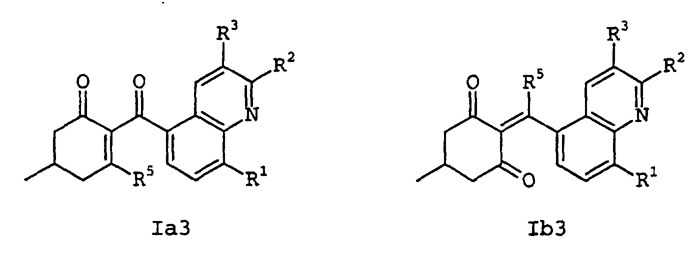
- Die Verbindungen der Formel Ia4 und Ib4, insbesondere die Verbindungen Ia4.1 bis Ia4.456 und die Verbindungen Ib4.1 bis Ib4.456, die sich von den Verbindungen Ia1.1 bis Ia1.456 bzw. Ib1.1 bis Ib1.456 dadurch unterscheiden, daß (R⁶)₁ "4,4-Dimethyl" bedeutet. 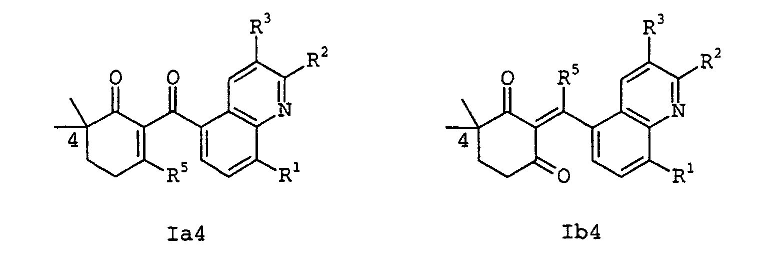
- Die Verbindungen der Formel Ia5 und Ib5, insbesondere die Verbindungen Ia5.1 bis Ia5.456 und die Verbindungen Ib5.1 bis Ib5.456, die sich von den Verbindungen Ia1.1 bis Ia1.456 bzw. Ib1.1 bis Ib1.456 dadurch unterscheiden, daß (R⁶)₁ "6,6-Dimethyl" bedeutet. 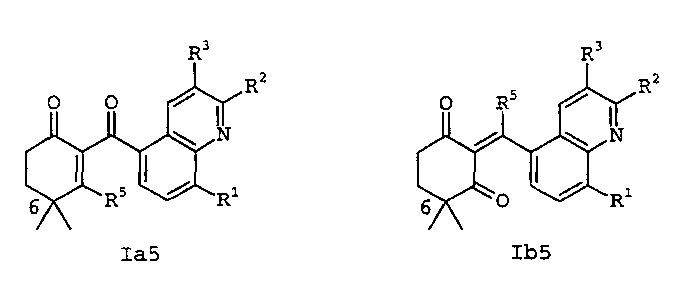
- Die Verbindungen der Formel Ia6 und Ib6, insbesondere die Verbindungen Ia6.1 bis Ia6.456 und die Verbindungen Ib6.1 bis Ib6.456, die sich von den Verbindungen Ia1.1 bis Ia1.456 bzw. Ib1.1 bis Ib1.456 dadurch unterscheiden, daß (R⁶)₁ "4,4,6,6-Tetramethyl-5-oxo" bedeutet. 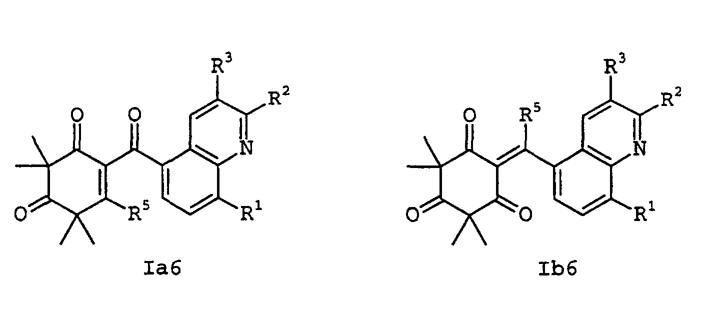
- Die Verbindungen der Formel Ia7 und Ib7, insbesondere die Verbindungen Ia7.1 bis Ia7.456 und die Verbindungen Ib7.1 bis Ib7.456, die sich von den Verbindungen Ia1.1 bis Ia1.456 bzw. Ib1.1 bis Ib1.456 dadurch unterscheiden, daß (R⁶)₁ "6-Methyl" bedeutet. 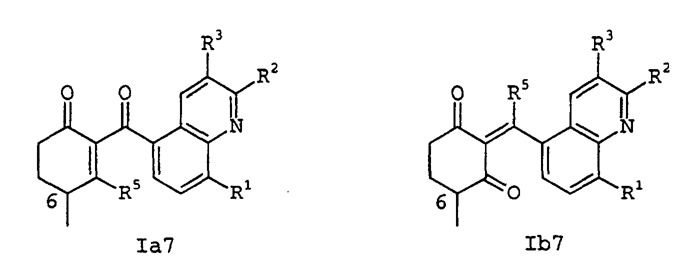
- Die Verbindungen der Formel Ia8 und Ib8, insbesondere die Verbindungen Ia8.1 bis Ia8.456 und die Verbindungen Ib8.1 bis Ib8.456, die sich von den Verbindungen Ia1.1 bis Ia1.456 bzw. Ib1.1 bis Ib1.456 dadurch unterscheiden, daß (R⁶)₁ "5-Hydroxy-4,4,6,6-tetramethyl" bedeutet.

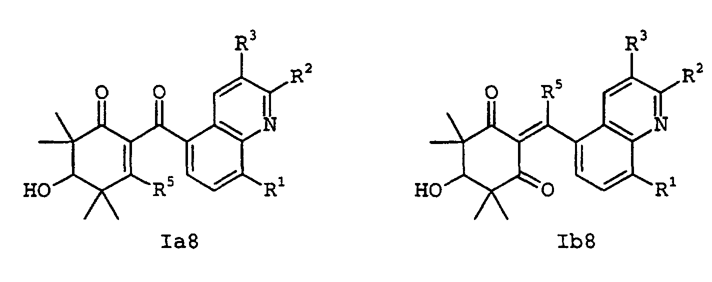

Die Cyclohexenonchinolinoyl-Derivate der Formel I sind auf verschiedene Art und Weise erhältlich, beispielsweise nach folgenden Verfahren:
A. Darstellung von Verbindungen der Formel I mit R⁵ = Halogen durch Umsetzung von Cyclohexandion-Derivaten der Formel III mit Halogenierungsmitteln: 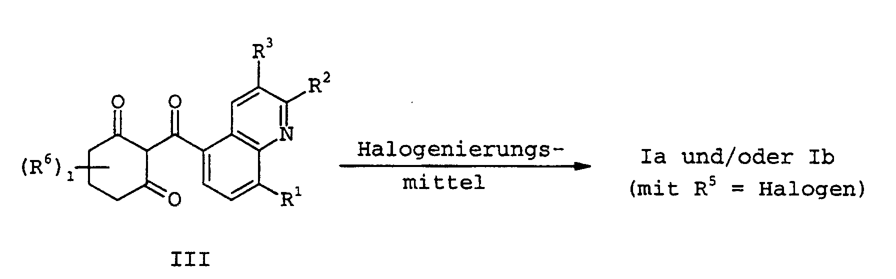
   Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxaylylbromid, Phosphoroxybromid etc.
B. Darstellung von Verbindungen der Formel I mit R⁵ = OR⁷, OSO₂R⁸, OPR⁸R⁹, OPOR⁸R⁹ oder OPSR⁸R⁹ durch Umsetzung von Cyclohexandion-Derivaten der Formel III mit Alkylierungs-, Sulfonylierungs- bzw. Phosphonylierungsmitteln IVα, IVβ, IVγ, IVδ bzw. IVε. 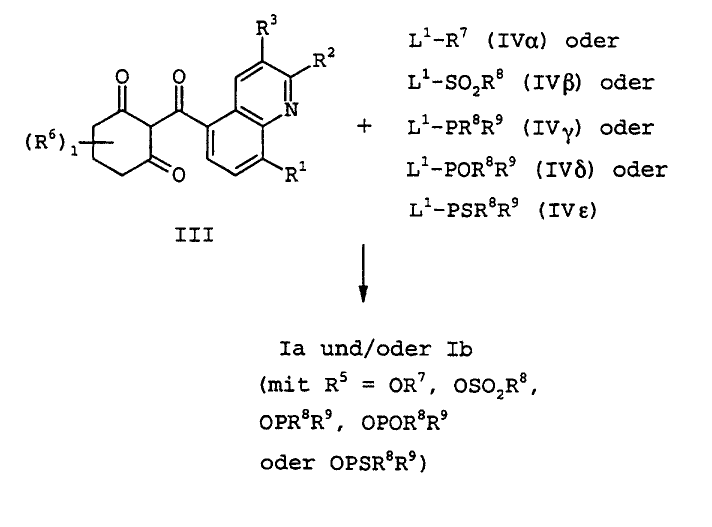
   L¹ steht für eine nucleophil verdrängbare Abgangsgruppe, wie Halogen, z. B. Chlor oder Brom, Hetaryl, z. B. Imidazolyl, Carboxylat, z. B. Acetat, oder Sulfonat, z. B. Mesylat oder Triflat etc.
   Die Verbindungen der Formel IVα, IVβ, IVγ, IVδ oder IVε können direkt eingesetzt werden wie z. B. im Fall der Carbonsäurehalogenide oder in situ erzeugt werden, z. B. aktivierte Carbonsäuren (mit Carbonsäure und Dicyclohexylcarbodiimid etc.).
C. Darstellung von Verbindungen der Formel I mit R⁵ = OR⁷, SR⁷, POR⁸R⁹, NR¹⁰R¹¹, ONR¹¹R¹², N-gebundenes Heterocyclyl oder O-(Ngebundenes Heterocyclyl) durch Umsetzung von Verbindungen der Formel I mit R⁵ = Halogen, OSO₂R⁸ (Iα) mit Verbindungen der Formel Vα, Vβ, Vγ, Vδ, Vε, Vη oder Vϑ, gegebenenfalls in Gegenwart einer Base oder unter vorangehender Salzbildung. 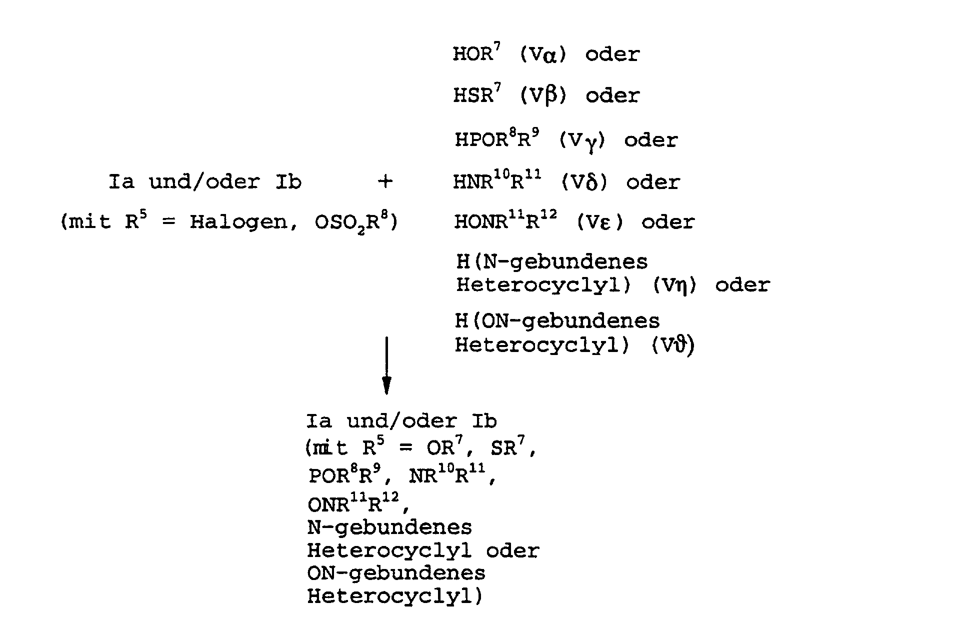
D. Darstellung von Verbindungen der Formel I mit R⁵ = SOR⁸, SO₂R⁸ durch Umsetzung von Verbindungen der Formel I mit R⁵ = SR⁸ (Iβ) mit einem Oxidationsmittel. 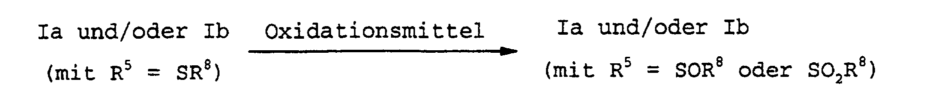
   Als Oxidationsmittel kommen beispielsweise m-Chlorperbenzoesäure, Peroxyessigsäure, Trifluorperoxyessigsäure, Wasserstoffperoxid, ggf. in Gegenwart eines Katalysators wie Wolframat, in Betracht.

Für die oben genannten Reaktionen gelten folgende Bedingungen: Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzungen in Gegenwart einer Base durchzuführen. Die Reaktanden und die Base werden dabei zweckmäßigerweise in äquimolaren Mengen eingesetzt. Ein Überschuß der Base z.B. 1,5 bis 3 Moläquivalente, bezogen auf Ia und/oder Ib (mit R⁵ = Halogen oder OSO₂R⁸) oder III, kann unter Umständen vorteilhaft sein.

Als Basen eignen sich tertiäre Alkylamine, wie Triethylamin, aromatische Amine, wie Pyridin, Alkalimetallcarbonate, z.B. Natriumcarbonat oder Kaliumcarbonat, Alkalimetallhydrogencarbonate, wie Natriumhydrogencarbonat und Kaliumhydrogencarbonat, Alkalimetallalkoholate wie Natriummethanolat, Natriumethanolat, Kalium-tert.butanolat oder Alkalimetallhydride, z.B. Natriumhydrid. Bevorzugt verwendet werden Triethylamin oder Pyridin.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

In Abhängigkeit von den Reaktionsbedinungen können die Verbindungen Ia, Ib oder Gemische hiervon gebildet werden. Letztere können durch klassische Trennmethoden, wie z.B. Kristallisation, Chromatographie etc., getrennt werden.

Die Cyclohexandion-Derivate der Formel III sind bekannt oder können nach an sich bekannten Verfahren hergestellt werden (z.B. DE-A 19 532 311). Beispielsweise durch Umsetzung von Cyclohexanonen der Formel VI mit einer aktivierten Benzoesäure VIIa oder einer Benzoesäure VIIb, die vorzugsweise in situ aktiviert wird, zu dem Acylierungsprodukt und anschließende Umlagerung.

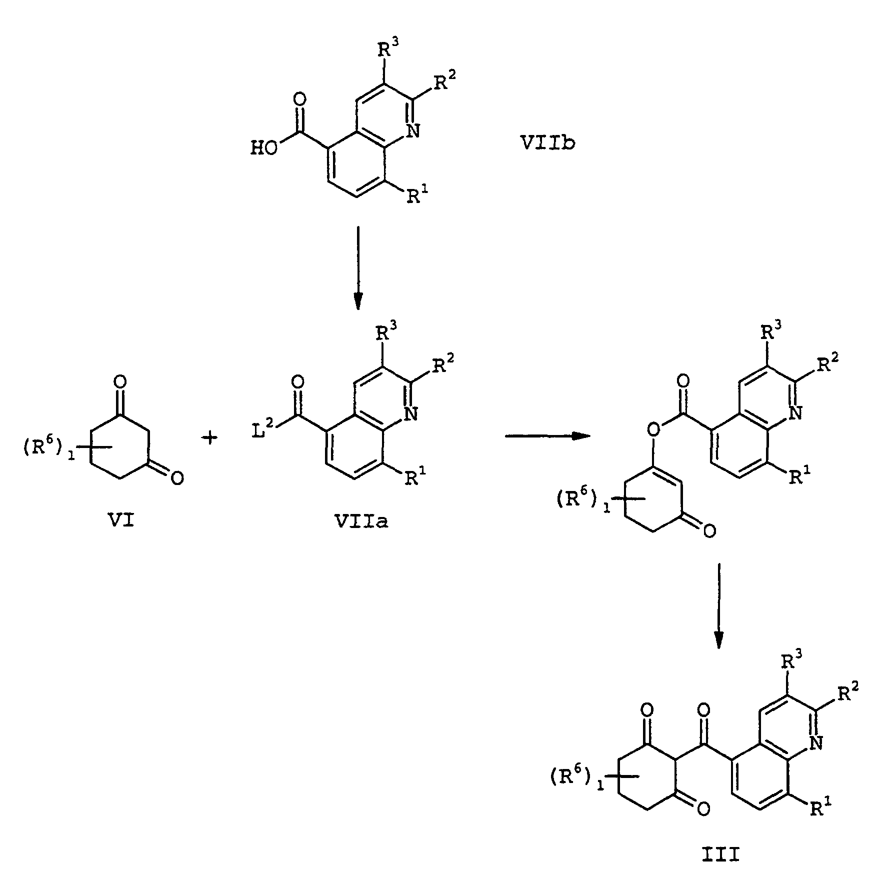

L² steht für eine nucleophil verdrängbare Abgangsgruppe, wie Halogen z.B. Brom oder Chlor, Hetaryl, z.B. Imidazolyl oder Pyridyl, Carboxylat, z.B. Acetat oder Trifluoracetat etc.

Die aktivierte Benzoesäure VIIa kann direkt eingesetzt werden, wie im Fall der Benzoylhalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf VII, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel III erfolgt zweckmäßigerweise bei Temperaturen von 20 bis 100°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonat verwendet, vorzugsweise in doppelt äquimolaren Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid oder Essigsäureethylester extrahiert. Der organische Extrakt kann mit 5-10%iger Alkalicarbonatlösung, z.B. Natriumcarbonat- oder Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

Die Benzoylhalogenide der Formel VIIa (mit L² = Cl, Br) können auf an sich bekannte Art und Weise durch Umsetzung der Benzoesäuren der Formel VIIb mit Halogenierungsreagentien wie Thionylchlorid, Thionylbromid, Phosgen, Diphosgen, Triphosgen, Oxalylchlorid, Oxalylbromid hergestellt werden.

Die Benzoesäuren der Formel VIIb können in bekannter Weise durch saure oder basische Hydrolyse aus den entsprechenden Estern hergestellt werden. Letztere sind literaturbekannt oder können auf an sich bekannte Art und Weise dargestellt werden.

8-Difluormethyl-5-alkoxycarbonyl-chinoline können durch Fluorierung aus den korrespondierenden 8-Aldehyd-Derivaten erhalten werden. Als Fluorierungsagens kommt unter anderem DAST in Betracht. Das Formylchinolin wird durch Oxidation des entsprechenden Brommethylchinolins erhalten.

Weiterhin ist es möglich 8-Difluormethoxy-5-alkoxycarbonyl-chinoline aus den entsprechenden 8-Hydroxy-Derivaten durch Umsetzung mit Chlordifluormethan zu gewinnen. Bevorzugt wird diese Reaktion in Gegenwart einer Base, wie Kaliumhydroxid oder Natriumhydroxid, in einen aprotischen Lösungsmittel durchgeführt. Die 8-Hydroxy-5-alkoxycarbonylchinoline werden durch an sich bekannte Veresterungsreaktionen aus 8-Hydroxy-5-hydroxycarbonyl-chinolin erhalten.

### Herstellungsbeispiele:

2-[(8-Chlorchinolin-5-yl)-carbonyl]-1-chlor-4,4,6,6-tetramethylcyclohex-1-en-3,5-dion (Verbindung 2.22) und
2-[(8-Chlorchinolin-5-yl)-chlormethyliden]-4,4,6,6-tetramethylcyclohexan-1,3,5-trion (Verbindung 3.1)

4,0 g (10,8 mmol) 2-(8-Chlorchinolin-5-yl)-carbonyl-4,4,6,6-tetramethyl-cyclohexan-1,3,5-trion wurden in 40 ml Dichlormethan gelöst, 4,1 g (32,4 mmol) Oxalylchlorid und 1,5 ml Dimethylformamid zugegeben. Nach 1,5 Stunden Rühren bei 25°C wurde das Lösungsmittel entfernt. Man erhielt 3,9 g farblose Kristalle. Nach Chromatographie an Kieselgel (Eluent: Toluol/Methyl-tert.butylether) erhielt man:
2-[(8-Chlorchinolin-5-yl)-carbonyl]-1-chlor-4,4,6,6-tetramethylcyclohex-1-en-3,5-dion: Ausbeute 0,65 g (farblose Kristalle); Fp.: 180°C;
2-[(8-Chlorchinolin-5-yl)-chlormethyliden-4,4,6,6-tetramethylcyclohexan-1,3,5-trion: Ausbeute 0,35 g (farblose Kristalle); Fp.: 156°C.

2-[(8-Chlorchinolin-5-yl)-1-(4'-oxo-1',4'-dihydropyrid-1'-yl)-4,4,6,6-tetramethyl-cyclohex-1-en-3,5-dion (Verbindung 2.46) und 2[(8-chlorchinolin-5-yl)-(4'-oxo-1',4'-dihydropyrid-1'-yl')-methyliden]-4,4,6,6-tetramethyl-cyclohexan-1,3,5-trion (Verbindung 3.5)

1,0 g (2,6 mmol) einer Mischung der Verbindungen 2.22 und 3.1 wurde in 25 ml Methylenchlorid gelöst, 0,82 g (8,7 mmol) 4-Hydroxy-pyridin zugegeben und 8 Stunden bei 40°C gerührt. Anschlieβend wurden unlösliche Bestandteile filtriert, das Lösungsmittel entfernt und der Rückstand an Kieselgel chromatograhiert (Eluent: Methylenchlorid/Methanol). Man erhielt: 2-[(8-Chlorchinolin-5-yl)-4'-oxo-1',4'-dihydropyridin-1'yl)methyliden-4,4,6,6-tetramethylcyclohexan-1,3,5-trion: Ausbeute 0,40 g (farbloses Öl);
2-[(8-Chlorchinolin-5-yl)-carbonyl]-1-(4'-oxo-1',4'-dihydropyrid-1'-yl)-4,4,6,6-tetramethylcyclohex-1-en-3,5-dion: Ausbeute 0,25 g (farblose Kristalle); Fp. > 210°C.

2-(8-Fluorchinolin-5-yl)-carbonyl-1,5-di(ethoxycarbonyloxy)-4,4,6,6-tetramethyl-cyclohex-1-en-3,5-dion (Verbindung 3.20) 0,12 g (4 mmol) Natriumhydrid wurden in 10 ml Tetrahydrofuran gelöst, bei Raumtemperatur 0,36 g (1 mmol) 2-[(8-Fluorchinolin-5-yl)-carbonyl]-4,4,6,6-tetramethyl-1-hydroxy-cyclohexan-3,5-dion in 5 ml Tetrahydroforan zugetropft und 1 Stunde bei 40°C gerührt. Anschließend wurde bei Raumtemperatur 0,43 g (4 mmol) Chlorameisensäureethylester zugetropft und 3 Stunden unter Rückfluß erhitzt. Nach Abkühlen wurden Wasser zugegeben, mit Essigsäureethylester extrahiert, die organische Phase mit 2-prozentiger Kaliumcarbonatlösung und Wasser gewaschen, getrocknet und das Lösungsmittel entfernt. Man erhielt 0,45 g eines farblosen Öles.

2-[(8-Chlorchinolin-5-yl)-carbonyl]-1-[dimethylamino)carbonylthio]-4,4,6,6-tetramethyl-cyclo-hex-1-en-3,5-dion (Verbindung 2.45) und
2-{(8-Chlorchinolin-5-yl)-[dimethylamino)carbonylthio]-methyliden}-4,4,6,6-tetramethylcyclohexan-1,3,5-trion (Verbindung 3.4)

0,50 g (1,3 mmol) 2-[(8-Chlorchinolin-5-yl)-carbonyl]-4,4,6,6-tetramethyl-cyclohexan-1,3,5-trion wurden in 15 ml Tetrahydrofuran gelöst, 0,52 g (5,2 mmol) Triethylamin zugegeben und 0,32 g (2,6 mmol) Dimethylaminothiocarbonylchlorid in 5 ml Tetrahydrofuran zugetropft. Nach 30 Stunden Rühren bei Raumtemperatur, wurde das Lösungsmittel entfernt, der Rückstand in Essigsäureethylester aufgenommen, mit 5-prozentiger Kaliumcarbonatlösung und Wasser gewaschen, getrocknet, eingeengt und mit Cyclohexan/Essigsäureethylester an Kieselgel chromatographiert. Man erhielt 2-[(8-Chlorchinolin-5-yl)-carbonyl]-1-[dimethylamino)carbonylthio]-4,4,6,6-tetramethyl-cyclohex-1-en-3,5-dion: Ausbeute 0,5 g (farblose Kristalle); Fp. 138°C;
2-{(8-Chlorchinolin-5-yl)-[[dimethylamino)carbonyl-thio]methyliden}-4,4,6,6-tetramethylcyclohexan-1,3,5-trion: Ausbeute: 0,2 g (farblose Kristalle) Fp. 75°C.

2-[(8-Difluormethylchinolin-5-yl)carbonyl]-1-chlor-4,4,6,6-tetramethyl-cyclohex-1-en-3,5-dion (Verbindung 2.31)

### Stufe a) 8-Formyl-5-chinolincarbonsäuremethylester

28,8 g (103 mmol) 8-(Brommethyl)-5-chinolincarbonsäuremethylester wurden in 200 ml Acetonitril gelöst, 36,1 g (309 mmol) N-Methylmorpholin-N-oxid zugegeben, 7 Stunden bei 25°C gerührt und anschließend das Lösungsmittel entfernt. Nach Chromatographie an Kieselgel (Eluent: Cyclohexan/Essigsäureethylester) erhielt man 12,0 g 8-Formyl-5-chinolincarbonsäure-methylester (farblose Kristalle), Fp.: 128°C.

### Stufe b) 8-Difluormethyl-5-chinolincarbonsäuremethylester

0,5 g (2,3 mmol) 8-Formyl-5-chinolincarbonsäuremethylester wurden in 50 ml Dichlorethan gelöst und bei -20°C 1,1 g (6,8 mmol) Diethylaminoschwefeltrifluorid (DAST) zugetropft. Nach 30 min. Rühren bei -20°C wurde auf 25°C erwärmt und 50 ml Wasser zugetropft. Die wäßrige Phase wurde mit Methylenchlord extrahiert, die vereinigten organischen Phasen mit NatriumhydrogencarbonatLösung gewaschen, getrocknet und das Lösungsmittel entfernt. Ausbeute: 0,7 g farblose Kristalle;
¹H-NMR (δ in ppm, d⁶-DMSO): 9.28 (d,1H); 9.04 (s, 1H); 8.36 (d, 1H); 8.11 (d, 1H); 7.90 (t, 1H); 7.80 (brs, 1H); 3.96 (s,3H).

### Stufe c) 8-Difluormethyl-5-chinolincarbonsäure

0,5 g (2,0 mmol) 8-Difluormethyl-5-chinolincarbonsäuremethylester wurden in 5 ml Ethanol gelöst, 0,43 g (10,5 mmol) Natriumhydroxyd und 1 ml Wasser zugegeben und 20 Stunden bie 25°C gerührt. Anschließend wurden die Lösungsmittel.entfernt, der Rückstand in Wasser aufgenommen, zweimal mit Methylenchlorid gewaschen, mit 10 N Salzsäure auf pH 1 gestellt und der Niederschlag abgesaugt.
Nach dem Trocknen erhielt man 0,5 g 8-Difluormethyl-5-chinolincarbonsäure (farblose Kristalle);
¹H-NMR (δ in ppm, d⁶-DMSO): 9.35 (d, 1H); 9.04 (s, 1H); 8.38 (d, 1H); 8.10 (d, 1H); 7.92 (t, 1H); 7.78 (brs, 1H).

### Stufe d) 2-[(8-Difluormethylchinolin-5-yl)-carbonyl]-4,4,6,6-tetramethylcyclohexan-1,3,5-trion

0,26 g (1,4 mmol) 2,2,4,4-Tetramethylcyclohexan-1,3,5-trion wurden in 10 ml Acetonitril gelöst, 0,34 g (1,4 mmol) 8-Difluormethyl-5-chinolincarbonsäure und 0,38 g (1,9 mmol) Dicyclohexylcarbodiimid zugegeben und 17 Stunden bei 25°C gerührt. Zu der Suspension wurden dann 0,57 g (5,6 mmol)Triethylamin und 5 Tropfen Trimethylsilylcyanid gegeben und weitere 25 Stunden bei 25°C gerührt. Anschließend wurde 50 ml 5-prozentige Kaliumcarbonatlösung zugegeben, filtriert, das Filtrat mit Methyl-tert.-Butylether gewaschen, die wäßrige Phase mit konzentrierter Salzsäure auf pH 2 gestellt und der Niederschlag abfiltriert, mit Wasser gewaschen und getrocknet. Ausbeute: 0,25 g (farblose Kristalle); ¹H-NMR (δ in ppm, CDCl₃): 17.5 (s,1H); 9.02 (q, 1H); 8.24 (d, 1H); 8.06 (d, 1H); 7.82 (t, 1H); 7.50 (m, 2H); 1.60 (s,6H); 1.36 (s, 6H).

### Stufe e) 2-[(8-Difluormethylchinolin-5-yl)-carbonyl]-1-chlor-4,4,6,6-tetramethyl-cyclohex-1-en-3,5-dion (Verbindung 2.31)

0,25 g (0,65 mmol) 2-(8-Difluormethylchinolin-5-yl)-carbonyl-4,4,6,6-tetramethyl-cyclohexan-1,3,5-trion wurden in 15 ml Dichlormethan gelöst, 0,25 g (1,95 mmol) Oxalylchlorid und 7 Tropfen Dimethylformamind zugegeben. Nach 17 Stunden Rühren bie 25°C wurde das Lösungsmittel entfernt. Man erhielt 0,2 g farblose Kristalle.

### Darstellung des Vorprodukts 2-[(8-Difluormethoxychinolin-5-yl)carbonyl]-4,4,6,6-tetramethylcyclohexan-1,3,5-trion

### Stufe a) 8-Hydroxy-5-chinolincarbonsäuremethylester

16,25 g (86 mmol) 8-Hydroxy-5-chinolincarbonsäure wurden in 70 ml Methanol gelöst, 3 ml konzentrierte Schwefelsäure zugegeben und 25 Stunden unter Rückfluß erhitzt. Das Lösungsmittel wurde dann entfernt, der Rückstand in Eiswasser aufgenommen, mit Natriumcarbonatlösung ein pH-Wert von 8 eingestellt und heiß filtriert. Der Rückstand wurde am Heiß-Extraktor 7 Stunden mit Methyl-tert.butylether extrahiert und anschließend das Lösungsmittel vom Extrakt entfernt. Man erhielt 6,8 g eines braunen Pulvers;
¹H-NMR (δ in ppm, d⁶-DMSO): 9.38 (d, 1H); 8.90 (d, 1H); 8.26 (d, 1H); 7.71 (dd, 1H); 7.15 (d, 1H); 3.93 (s, 3H).

### Stufe b) 8-Difluormethoxy-5-chinolincarbonsäuremethylester

1,0 g (5,0 mmol) 8-Hydroxy-5-chinolincarbonsäuremethylester wurden in 20 ml Dimethylformamid gelöst, 0,76 g (5,5 mmol) Kaliumcarbonat zugegeben und bei 40°C über 2 Stunden 14 g Chlordifluormethan eingesetzt. Feste Bestandteile wurden dann abfiltriert, das Lösungsmittel enfernt, der Rückstand mit Wasser gewaschen und getrocknet. Man erhielt 0,75 g eines braunen Pulvers;
¹H-NMR (δ in ppm, CDCl₃): 9.45 (d,1H); 9.00 (d, 1H); 8.30 (d, 1H); 7.61 (dd, 1H); 7.49 (d, 1H); 7.18 (t, 1H); 3.99 (s, 3H).

### Stufe c) 8-Difluormethoxy-5-chinolincarbonsäure

0,7 g (2,8 mmol) 8-Difluormethoxy-5-chinolincarbonsäuremethylester wurden in 15 ml Wasser suspendiert und 0,4 g (10 mmol) Natriumhydroxid zugegeben. Es wurde 20 Stunden bei 25°C gerüht, abfiltriert und das Filtrat mit Methyl-tert.-butylether gewaschen. Die wäßrige Phase wurde mit konzentrierter Salzsäure auf pH 3 gestellt, abfiltriert und der Rückstand getrocknet. Man erhält 0,45 g eines farblosen Pulvers;
¹H-NMR (δ in ppm, d⁶-DMSO): 13.5 (br, 1H); 9.39 (d, 1H); 9.03 (d, 1H); 8.32 (d, 1H); 7.78 (dd, 1H); 7.62 (d, 1H); 7.60 (t, 1H).

### Stufe d) 2-[(8-Difluormethoxychinolin-5-yl)carbonyl]-4,4,6,6-tetramethylcyclohexan-1,3,5-trion

0,4 g (1,7 mmol) 8-Difluormethoxy-5-chinolincarbonsäure wurden in 20 ml Acetonitril gelöst, 0,4 g (1,9 mmol) N,N-Dicyclohexylcarbodiimid und 0,3 g (1,7 mmol) 2,2,4,4-Tetramethylcyclohexan-1,3,5-trion zugegeben und 20 Stunden bei 25°C gerührt. Dann wurden 0,4 g (4,0 mmol) Triethylamin und 2 Tropfen Trimethylsilylcyanid zugegeben und weitere 3 Stunden bei 30 - 35°C gerührt. Der Niederschlag wurde abfiltriert, das Filtrat eingeengt, 20 ml5-prozentige Kaliumcarbonatlösung zugegeben und mit Methyl-tert.-butylether gewaschen. Die wäßrige Phase wurde anschließend mit konzentrierter Salzsäure auf pH 3 gestellt und mit Essigsäureethylester extrahiert. Nach Entfernen des Lösungsmittels wurde an Kieselgel chromatographiert (Eluent: Methylenchlord/Methanol). Man erhielt 0,2 g eines farblosen Pulvers;
¹H-NMR (δ in ppm, CDCl₃): 16.5 (br, 1H); 9.02 (d, 1H); 8.30 (d, 1H); 7.51 (m, 2H); 7.21 (d, 1H); 7.17 (t, 1H); 1.60 (s, 6H); 1.35 (s, 6H).

In den Tabellen 2 und 3 sind neben den veranstehend beschriebenen Cyclohexanonchinolinoyl-Derivaten der Formel I weitere aufgeführt, die in analoger Weise oder auf an sich bekannte Art und Weise hergestellt wurden oder herstellbar sind:

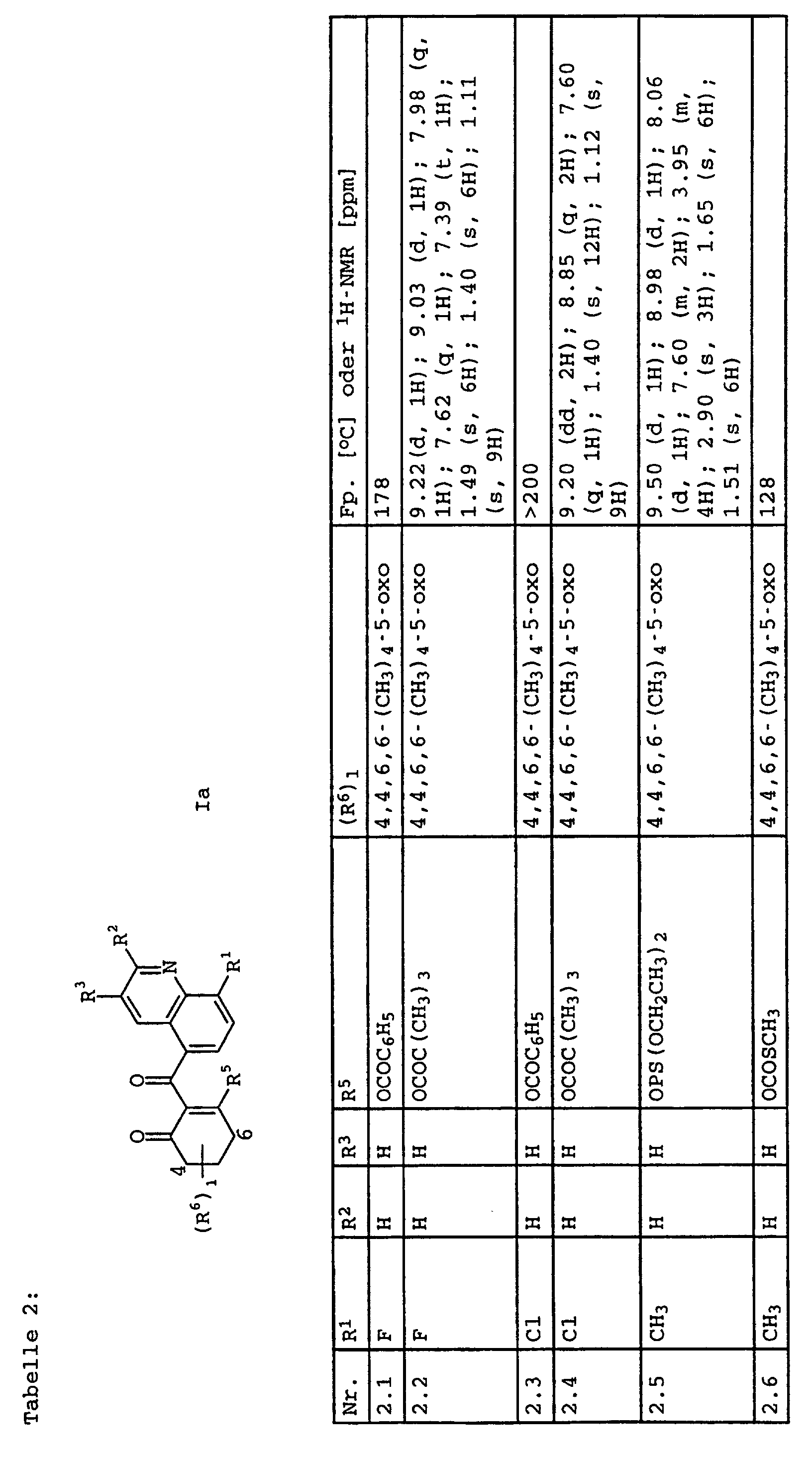

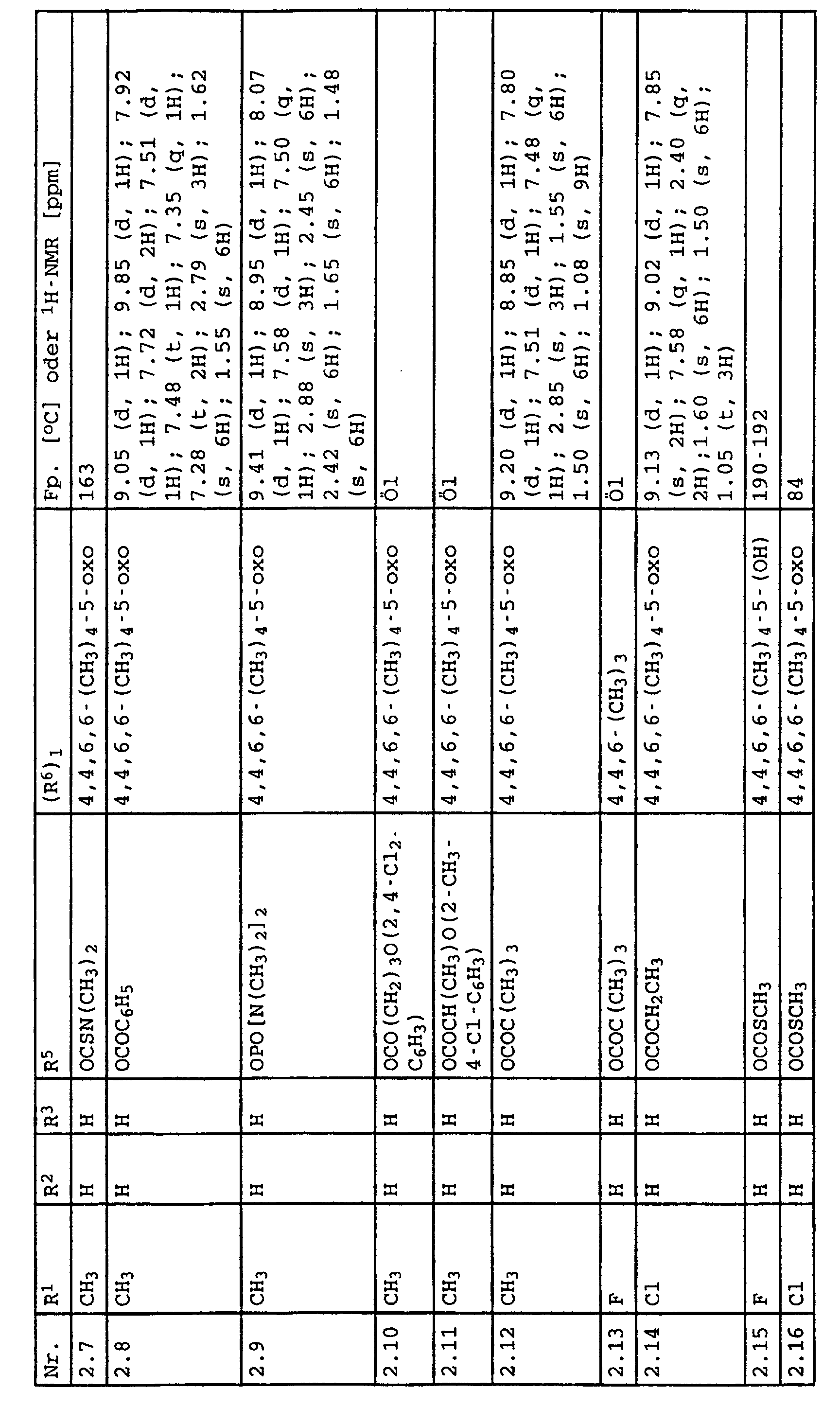

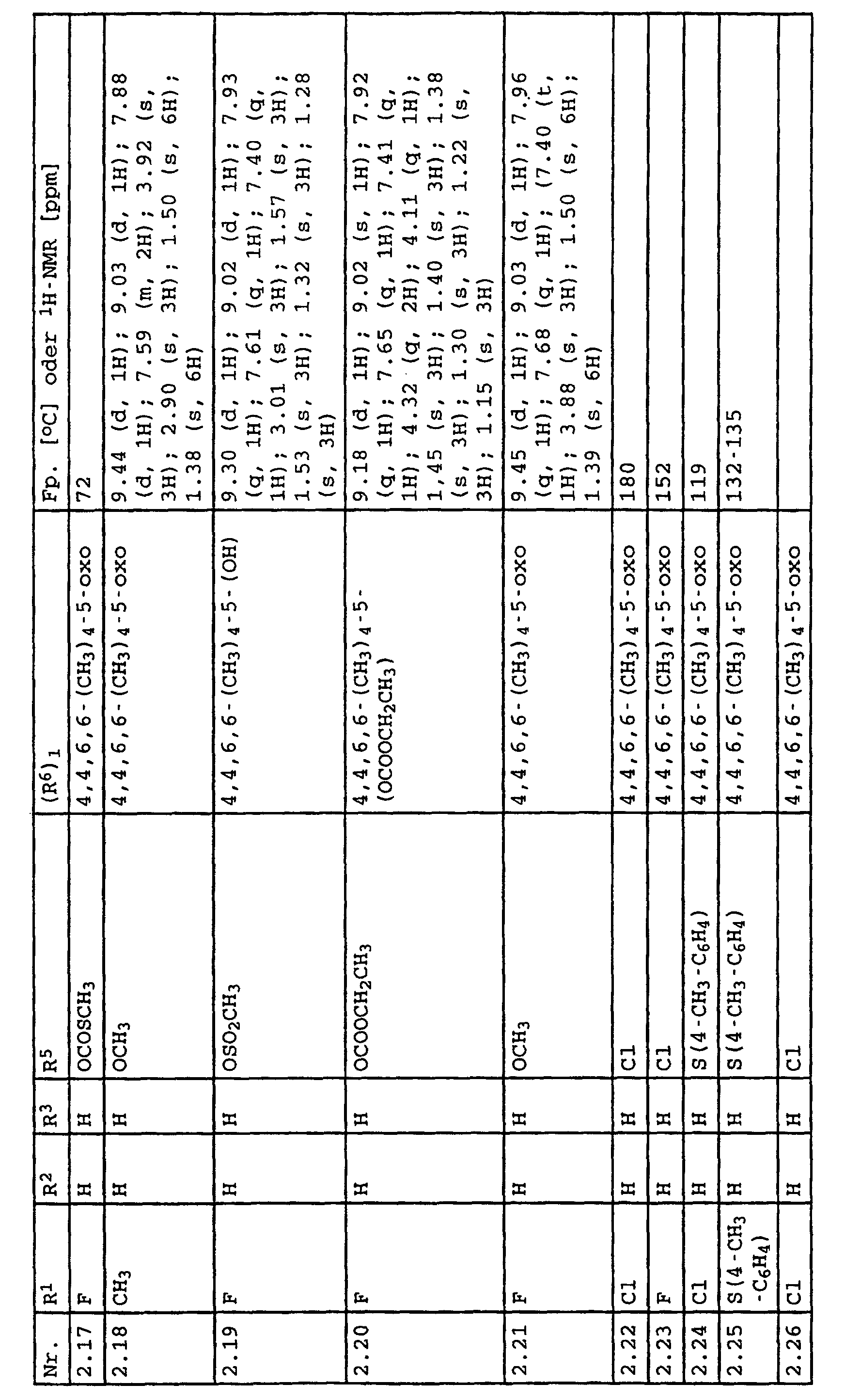

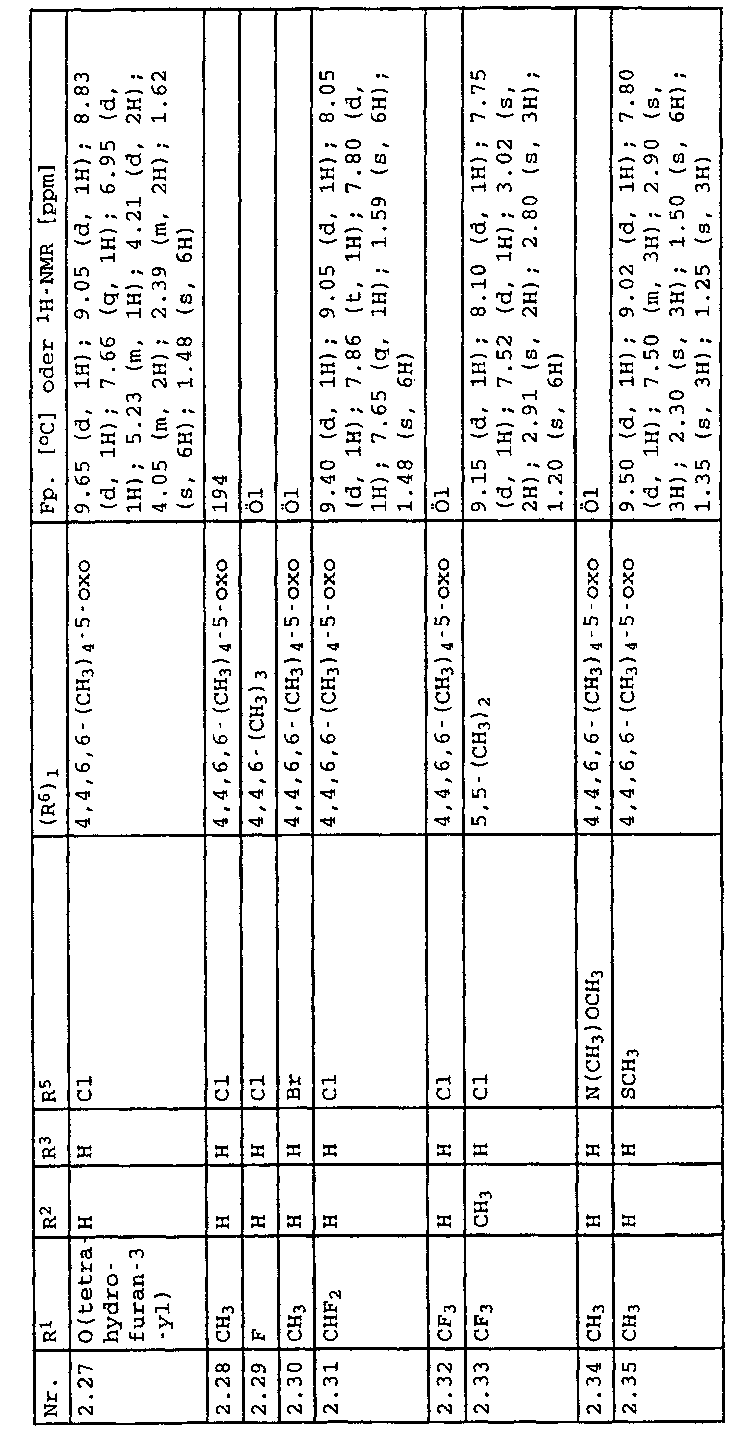

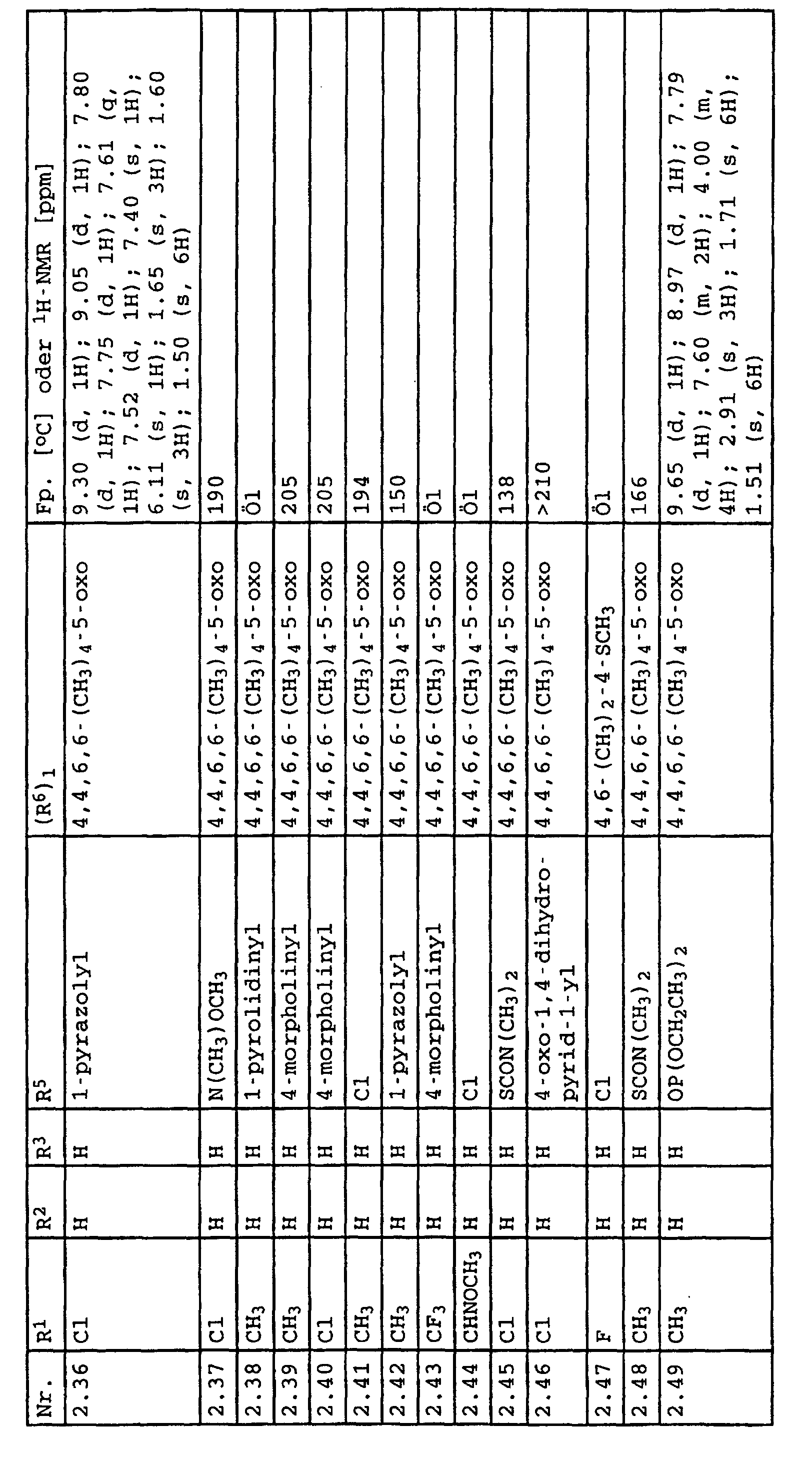

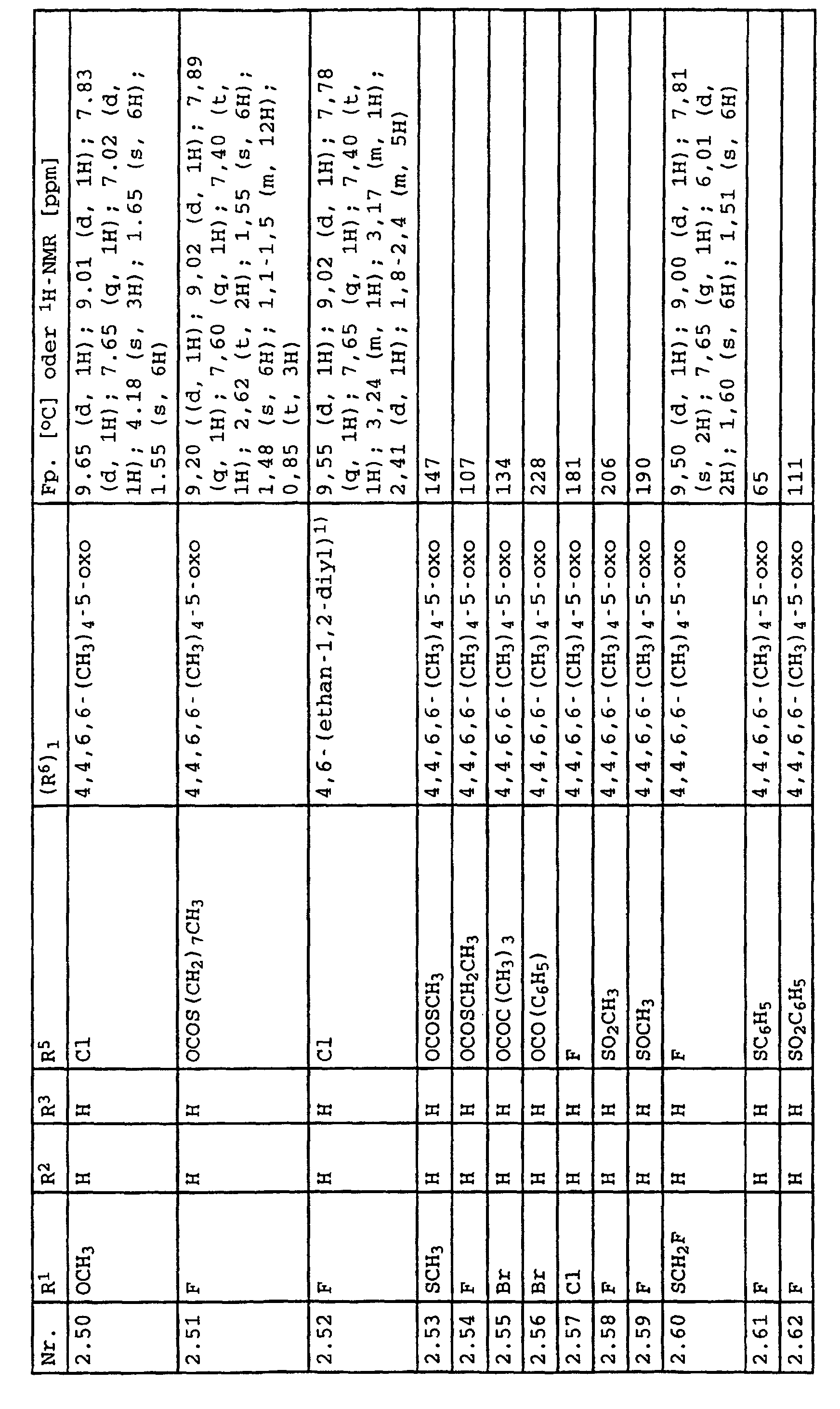

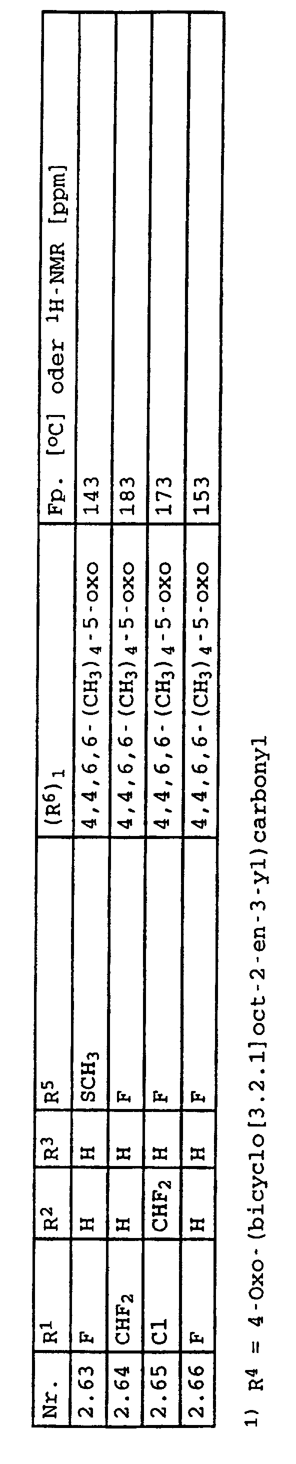

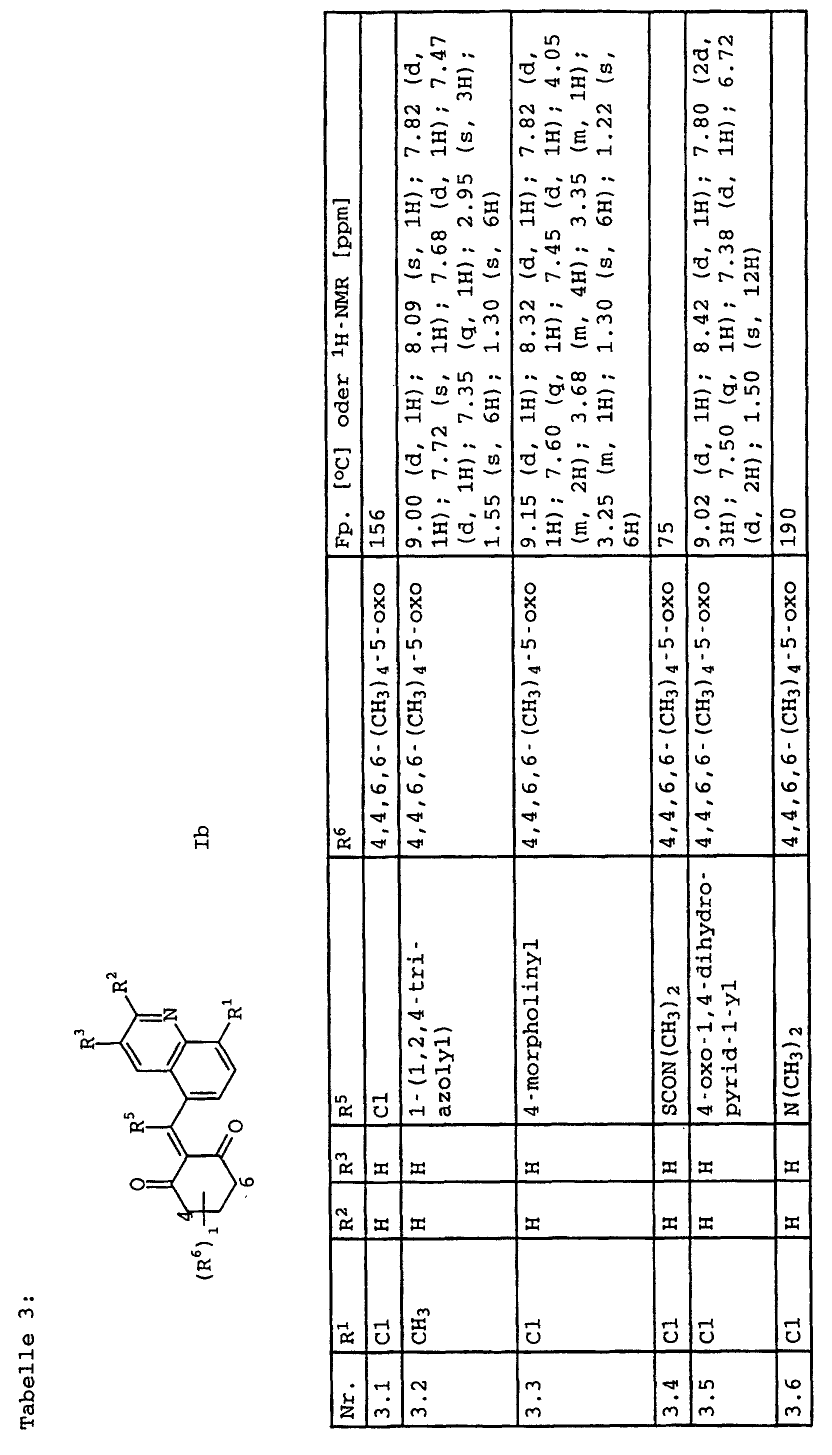

Die Verbindungen der Formel I und deren landwirtschaftlich brauchbaren Salze eignen sich sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline und deren Derivate, alkylierte Benzole oder deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Cyclohexenonchinolinoyl-Derivate der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 2.4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile der Verbindung Nr. 2.16 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile der Verbindung Nr. 2.18 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile der Verbindung Nr. 2.22 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile der Verbindung Nr. 2.46 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 3.1 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. 3.4 wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3.0 vorzugsweise 0.01 bis 1.0 kg/ha aktive Substanz (a.s.).

Zur Verbreiterung des Wirkungsspektrums und zur Erzielung synergistischer Effekte können die Cyclohexenonchinolinoyl-Derivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-Aroyl-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der Cyclohexenonchinolinoyl-Derivate der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.25 bzw. 0.125 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Chenopodium album | Weißer Gänsefuß | lambsquarters |
| Galium aparine | Klettenlabkraut | catchweed bedstraw |
| Ipomoea spp. | Prunkwindearten | morningglory |
| Setaria faberi | Borstenhirse | giant foxtail |
| Setaria viridis | Grüne Borstenhirse | green foxtail |
| Solanum nigrum | Schwarzer Nachtschatten | black nightshade |

Bei Aufwandmengen von 0.25 bzw. 0.125 kg/ha a.S. zeigten die Verbindungen 2.2, 2.4 und 2.16 im Nachauflauf eine sehr gute Wirkung gegen Schadpflanzen wie Borstenhirse, grüne Borstenhirse und schwarzen Nachtschatten. Weiterhin bekämpfen die Verbindungen 2.2 und 2.4 chinesischen Hanf und Prunkwinden sehr gut. Verbindung 2.16 zeigt zudem hervorragende Wirkung gegenüber den Unkräutern weißer Gänsefuß und Klettenlabkraut.

## Patentansprüche

1. Cyclohexenonchinolinoyl-Derivate der Formel I 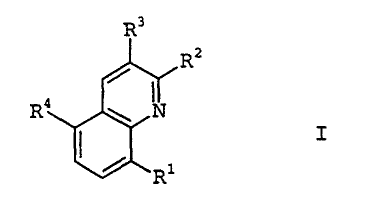 in der die Variablen folgende Bedeutungen haben:
R¹ Wasserstoff, Nitro, Halogen, Cyano, C₁-C₆-Alkyl, C₁-C₆-Halogenalkyl, C₁-C₆-Alkoxyiminomethyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)amino, N-(C₁-C₆-Alkyl)-N-(C₁-C₆-alkylsulfonyl)amino, N-(C₁-C₆-Alky)-N-(C₁-C₆-Halogenalkylsulfonyl)-amino, Phenoxy, Heterocycloyloxy, Phenylthio oder Heterocyclylthio, wobei die vier letztgenannten Reste partiell oder vollständig halogeniert sein können und/oder einen bis drei der nachfolgend genannten Substituenten tragen können: Nitro, Cyano, C₁-C₄-Alkyl, C₁-C₄-Halogenalkyl, C₁-C₄-Alkoxy oder C₁-C₄-Halogenalkoxy;
R², R³ Wasserstoff, C₁-C₆-Alkyl, C₁-C₆-Halogenalkyl oder Halogen;
R⁴ eine Verbindung IIa oder IIb 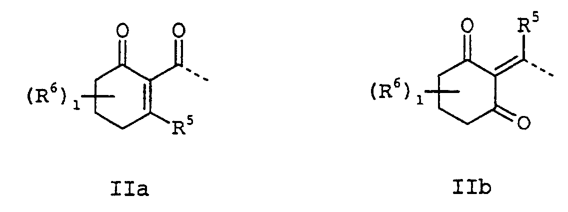
wobei
R⁵ Halogen, OR⁷, SR⁷, SOR⁸, SO₂R⁸, OSO₂R⁸, POR⁸R⁹, OPR⁸R⁹, OPOR⁸R⁹, OPSR⁸R⁹, NR¹⁰R¹¹, ONR¹¹R¹², N-gebundenes Heterocyclyl oder O-(N-gebundenes Heterocyclyl), wobei der Heterocyclyl-Rest der beiden letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R⁶ Nitro, Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylcarbonyl, C₁-C₆-Halogenalkylcarbonyl, C₁-C₆-Alkoxycarbonyl oder C₁-C₆-Halogenalkoxycarbonyl;
oder
zwei Reste R⁶, die am gleichen Kohlenstoff gebunden sind, bilden gemeinsam eine -O-(CH₂)ₘ-O-, -O-(CH₂)ₘ-S-, -S-(CH₂)ₘ-S-, -O-(CH₂)ₙ- oder -S-(CH₂)ₙ-Kette, die durch einen bis drei Reste aus folgender Gruppe substituiert sein kann:
Halogen, Cyano, C₁-C₄-Alkyl, C₁-C₄-Halogenalkyl oder C₁-C₄-Alkoxycarbonyl;
oder
zwei Reste R⁶, die am gleichen Kohlenstoff gebunden sind, bilden gemeinsam eine -(CH₂)ₚ-Kette, die durch Sauerstoff oder Schwefel unterbrochen sein kann und/ oder durch einen bis vier Reste aus folgender Gruppe substituiert sein kann:
Halogen, Cyano, C₁-C₄-Alkyl, C₁-C₄-Halogenalkyl oder C₁-C₄-Alkoxycarbonyl;
oder
zwei Reste R⁶, die am gleichen Kohlenstoff gebunden sind, bilden gemeinsam eine Methylidengruppe, die durch einen bis zwei Reste aus folgender Gruppe substituiert sein kann:
Halogen, Hydroxy, Formyl, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
oder
zwei Reste R⁶, die am gleichen Kohlenstoff gebunden sind, bilden gemeinsam mit diesem Kohlenstoff eine Carbonylgruppe aus;
oder
zwei Reste R⁶, die an verschiedenen Kohlenstoffen gebunden sind, bilden gemeinsam eine -(CH₂)ₙ-Kette, die durch einen bis drei Reste aus folgender Gruppe substituiert sein kann:
Halogen, C₁-C₆-Alkyl, C₁-C₆-Alkoxy, Hydroxy oder C₁-C₆-Alkoxycarbonyl;
R⁷ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₂₀-Alkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, (C₁-C₂₀-Alkylthio)carbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)- N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Heterocyclyloxycarbonyl, Phenoxythiocarbonyl, Heterocyclyloxythiocarbonyl, Phenoxy-C₁-C₆-alkylcarbonyl, Heterocyclyoxy-C₁-C₆-alkylcarbonyl, Phenylaminocarbonyl, N-(C₁-C₆-Alkyl)-N-(phenyl)-aminocarbonyl, Heterocyclylaminocarbonyl, N-(C₁-C₆-Alkyl)-N-(heterocyclyl)-aminocarbonyl, Phenyl-C₂-C₆-alkenylcarbonyl oder Heterocyclyl-C₂-C₆-alkenylcarbonyl, wobei der Phenyl- und der Heterocyclyl-Rest der 20 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R⁸, R⁹ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, Amino, C₁-C₆-Alkylamino, C₁-C₆-Halogenalkylamino, Di-(C₁-C₆alkyl)amino oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R¹⁰ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-Alkyl)-amino oder C₁-C₆-Alkylcarbonylamino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R¹¹, R¹² C₁-C₆-Alkyl, C₃-C₆-Alkenyl, C₃-C₆-Alkinyl oder C₁-C₆-Alkylcarbonyl;
l 0 bis 6;
m 2 bis 4;
n 1 bis 5;
p 2 bis 5;
sowie deren landwirtschaftlich brauchbaren Salze.

2. Cyclohexenonchinolinoyl-Derivate der Formel I, gemäß Anspruch 1, wobei
R¹ Halogen, C₁-C₆-Alkyl, C₁-C₆-Halogenalkyl, C₁-C₆-Alkoxy, C₁-C₆-Alkylthio, Heterocyclyloxy oder Phenylthio, wobei die zwei letztgenannten Reste partiell oder vollständig halogeniert sein können und/oder einen bis drei der nachfolgend genannten Substituenten tragen können:
Nitro, Cyano, C₁-C₄-Alkyl, C₁-C₄-Halogenalkyl, C₁-C₄-Alkoxy oder C₁-C₄-Halogenalkoxy;
R⁵ Halogen, OR⁷, SR⁷, SOR⁸, SO₂R⁸, OSO₂R⁸, OPR⁸R⁹, OPOR⁸R⁹, OPSR⁸R⁹, NR¹⁰R¹¹ oder N-gebundenes Heterocyclyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
bedeuten.

3. Cyclohexenonchinolinoyl-Derivate der Formel I, gemäß den Ansprüchen 1 oder 2, wobei
R⁵ Halogen, OR⁷, NR¹⁰R¹¹ oder N-gebundenes Heterocyclyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
bedeutet.

4. Cyclohexenonchinolinoyl-Derivate der Formel I, gemäß den Ansprüchen 1 bis 3, wobei
R⁷ C₁-C₆-Alkyl, C₁-C₂₀-Alkylcarbonyl, C₁-C₆-Alkoxycarbonyl, (C₁-C₂₀-Alkylthio)carbonyl, N,N-Di(C₁-C₆-alkyl)aminocarbonyl, Phenyl, Phenylcarbonyl oder Phenoxy-C₁-C₆-alkylcarbonyl, wobei der Phenylrest der drei letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R¹⁰ C₁-C₆-Alkyl oder C₁-C₆-Alkoxy;
R¹¹ C₁-C₆-Alkyl;
bedeuten.

5. Cyclohexenonchinolinoyl-Derivate der Formel I, gemäß den Ansprüchen 1 bis 4, wobei
R⁶ Nitro, Halogen, Cyano, C₁-C₆-Alkyl, C₁-C₆-Halogenalkyl, Di-(C₁-C₆-alkoxy)-methyl, Di-(C₁-C₆-alkylthio)-methyl, (C₁-C₆-Alkoxy)(C₁-C₆-alkylthio)-methyl, Hydroxy, C₁-C₆-Alkoxy, C₁-C₆-Halogenalkoxy, C₁-C₆-Alkoxycarbonyloxy, C₁-C₆-Alkylthio, C₁-C₆-Halogenalkylthio, C₁-C₆-Alkylsulfinyl, C₁-C₆-Halogenalkylsufinyl, C₁-C₆-Alkylsulfonyl, C₁-C₆-Halogenalkylsulfonyl, C₁-C₆-Alkylcarbonyl, C₁-C₆-Halogenalkylcarbonyl, C₁-C₆-Alkoxycarbonyl oder C₁-C₆-Halogenalkoxycarbonyl;
bedeutet
oder
zwei Reste R⁶, die am gleichen Kohlenstoff gebunden sind, bilden gemeinsam eine -O-(CH₂)ₘ-O-, -O-(CH₂)ₘ-S-, -S-(CH₂)ₘ-S-, -O-(CH₂)ₙ- oder -S-(CH₂)ₙ-Kette, die durch einen bis drei Reste aus folgender Gruppe substituiert sein kann:
Halogen, Cyano, C₁-C₄-Alkyl, C₁-C₄-Halogenalkyl oder C₁-C₄-Alkoxycarbonyl;
oder
zwei Reste R⁶, die am gleichen Kohlenstoff gebunden sind, bilden eine -(CH₂)ₚ-Kette, die durch Sauerstoff oder Schwefel unterbrochen sein kann und/oder durch einen bis vier Reste aus folgender Gruppe substituiert sein kann:
Halogen, Cyano, C₁-C₄-Alkyl, C₁-C₄-Halogenalkyl oder C₁-C₄-Alkoxycarbonyl;
oder
zwei Reste R⁶, die am gleichen Kohlenstoff gebunden sind, bilden gemeinsam mit diesem Kohlenstoff eine Carbonylgruppe aus.

6. Verfahren zur Herstellung von Verbindungen der Formel I gemäß den Ansprüchen 1 bis 5 mit R⁵ = Halogen, **dadurch gekennzeichnet, daß** man ein Cyclohexandion-Derivat der Formel III, 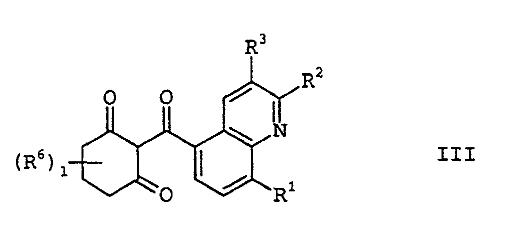 wobei die Variablen R¹ bis R³, R⁶ und 1 die in den Ansprüchen 1 bis 5 genannte Bedeutung haben, mit einem Halogenierungsmittel umsetzt.

7. Verfahren zur Herstellung von Verbindungen der Formel I gemäß den Ansprüchen 1 bis 5 mit R⁵ = OR⁷, OSO₂R⁸, OPR⁸R⁹, OPOR⁸R⁹ oder OPSR⁸R⁹ **dadurch gekennzeichnet, daß** man ein Cyclohexandion-Derivat der Formel III, 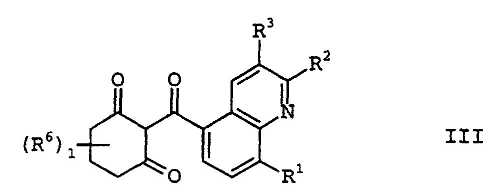 wobei die Variablen R¹ bis R³, R⁶ und 1 die in den Ansprüchen 1 bis 5 genannte Bedeutung haben, mit einer Verbindung der Formel IVα, IVβ, IVγ, IVδ oder IVε, 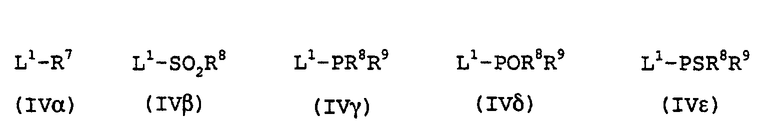 wobei die Variablen R⁷ bis R⁹ die in den Ansprüchen 1 bis 5 genannte Bedeutung haben und L¹ für eine nucleophil verdrängbare Abgangsgruppe steht, umsetzt.

8. Verfahren zur Herstellung von Verbindungen der Formel I gemäß den Ansprüchen 1 bis 5 mit R⁵ = OR⁷, SR⁷, POR⁸R⁹, NR¹⁰R¹¹, ONR¹¹R¹², N-gebundenes Heterocyclyl oder O(N-gebundenes Heterocyclyl), **dadurch gekennzeichnet, daß** man eine Verbindung der Formel Iα (≡ I mit R⁵ = Halogen, OSO₂R⁸), 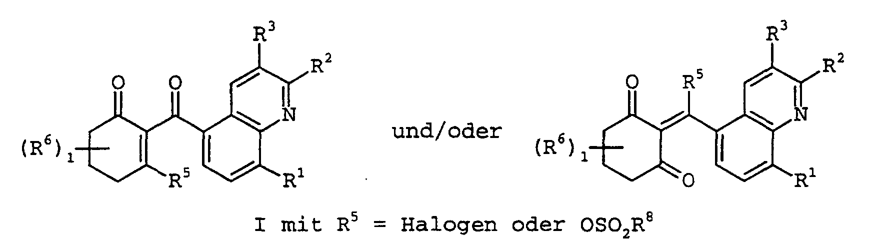 wobei die Variablen R¹ bis R³, R⁶ und die in den Ansprüchen 1 bis 5 genannte Bedeutung haben, mit einer Verbindung der Formel Vα, Vβ, Vγ, Vδ, Vε, Vη oder Vϑ, 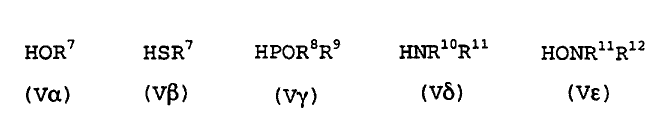 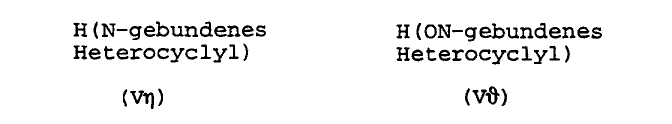 wobei die Variablen R⁷ bis R¹² die in den Ansprüchen 1 bis 5 genannte Bedeutung haben, gegebenenfalls in Gegenwart einer Base, umsetzt.

9. Verfahren zur Herstellung von Verbindungen der Formel I gemäß den Ansprüchen 1, 2 oder 5 mit R⁵ = SOR⁸, SO₂R⁸, **dadurch gekennzeichnet, daß** man eine Verbindung der Formel Iβ (≡ I mit R⁵ = SR⁸), 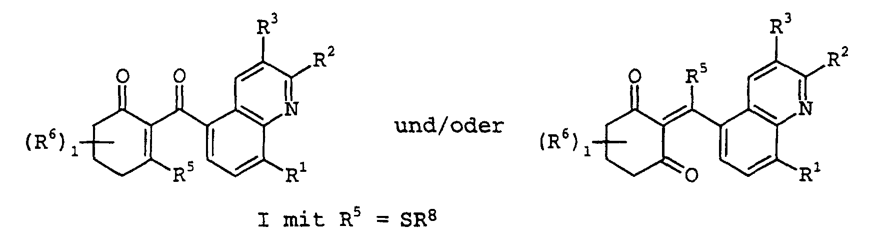 wobei die Variablen R¹ bis R⁸ und 1 die in den Ansprüchen 1, 2 oder 5 genannte Bedeutung haben, mit einem Oxidationsmittel umsetzt.

10. Mittel, enthaltend eine herbizid wirksame Menge mindestens eines Cyclohexenonchinolinoyl-Derivates der Formel I oder eines landwirtschaftlich brauchbaren Salzes von I gemäß den Ansprüchen 1 bis 5, und für die Formulierung von Pflanzenschutzmitteln übliche Hilfsmittel.

11. Verfahren zur Herstellung von Mitteln gemäß Anspruch 10, **dadurch gekennzeichnet, daß** man eine herbizid wirksame Menge mindestens eines Cyclohexenonchinolinoyl-Derivates der Formel I oder eines landwirtschaftlich brauchbaren Salzes von I gemäß den Ansprüchen 1 bis 5 und für die Formulierung von Pflanzenschutzmitteln übliche Hilfsmittel mischt.

12. Verfahren zur Bekämpfung von unerwünschtem Pflanzenwuchs, **dadurch gekennzeichnet, daß** man eine herbizid wirksame Menge mindestens eines Cyclohexenonchinolinoyl-Derivates der Formel I oder eines landwirtschaftlich brauchbaren Salzes von I gemäß den Ansprüchen 1 bis 5, auf Pflanzen, deren Lebensraum und/oder auf Samen einwirken läßt.

13. Verwendung von Cyclohexenonchinolinoyl-Derivaten der Formel I oder deren landwirtschaftlich brauchbaren Salze gemäß den Ansprüchen 1 bis 5 als Herbizide.

## Claims

1. A cyclohexenonequinolinoyl derivative of the formula I 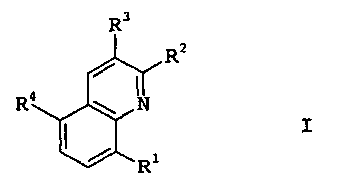 where:
R¹ is hydrogen, nitro, halogen, cyano, C₁-C₆-alkyl, C₁-C₆-haloalkyl, C₁-C₆-alkoxyiminomethyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, aminosulfonyl, N-(C₁-C₆-alkyl)aminosulfonyl, N,N-di-(C₁-C₆-alkyl)aminosulfonyl, N-(C₁-C₆-alkylsulfonyl)amino, N-(C₁-C₆-haloalkylsulfonyl)amino, N-(C₁-C₆-alkyl)-N-(C₁-C₆-alkylsulfonyl)amino, N-(C₁-C₆-alkyl)-N-(C₁-C₆-haloalkylsulfonyl)amino, phenoxy, heterocyclyloxy, phenylthio or heterocyclylthio, where the four last-mentioned radicals may be partially or fully halogenated and/or may carry one to three of the following substituents:
nitro, cyano, C₁-C₄-alkyl, C₁-C₄-haloalkyl, C₁-C₄-alkoxy or C₁-C₄-haloalkoxy;
R², R³ are hydrogen, C₁-C₆-alkyl, C₁-C₆-haloalkyl or halogen;
R⁴ is a compound IIa or IIb 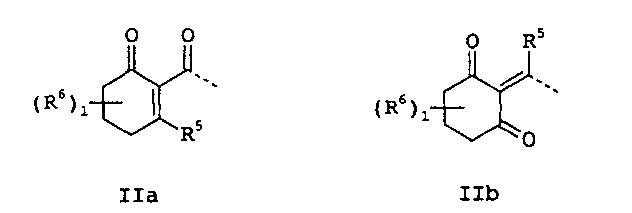
where
R⁵ is halogen, OR⁷, SR⁷, SOR⁸, SO₂R⁸, OSO₂R⁸, POR⁸R⁹, OPR⁸R⁹, OPOR⁸R⁹, OPSR⁸R⁹, NR¹⁰R¹¹, ONR¹¹R¹², N-linked heterocyclyl or O-(N-linked heterocyclyl), where the heterocyclyl radical of the two last-mentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R⁶ is nitro, halogen, cyano, C₁-C₆-alkyl, C₁-C₆-haloalkyl, di-(C₁-C₆-alkoxy)methyl, di-(C₁-C₆-alkylthio)methyl, (C₁-C₆-alkoxy)(C₁-C₆-alkylthio)methyl, hydroxyl, C₁-C₆-alkoxy, C₁-C₆-haloalkoxy, C₁-C₆-alkoxycarbonyloxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, C₁-C₆-alkylcarbonyl, C₁-C₆-haloalkylcarbonyl, C₁-C₆-alkoxycarbonyl or C₁-C₆-haloalkoxycarbonyl;
or
two radicals R⁶, which are linked to the same carbon, together form an -O-(CH₂)ₘ-O-, -O-(CH₂)ₘ-S-, -S-(CH₂)ₘ-S-, -O-(CH₂)ₙ- or -S-(CH₂)ₙ chain which may be substituted by one to three radicals from the following group:
halogen, cyano, C₁-C₄-alkyl, C₁-C₄-haloalkyl or C₁-C₄-alkoxycarbonyl;
or
two radicals R⁶, which are linked to the same carbon, together form a -(CH₂)ₚ chain which may be interrupted by oxygen or sulfur and/or may be substituted by one to four radicals from the following group:
halogen, cyano, C₁-C₄-alkyl, C₁-C₄-haloalkyl or C₁-C₄-alkoxycarbonyl;
or
two radicals R⁶, which are linked to the same carbon, together form a methylidene group which may be substituted by one or two radicals from the following group:
halogen, hydroxyl, formyl, cyano, C₁-C₆-alkyl, C₁-C₆-haloalkyl, C₁-C₆-alkoxy, C₁-₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl;
or
two radicals R⁶, which are linked to the same carbon, together with this carbon form a carbonyl group;
or
two radicals R⁶, which are linked to different carbons, together form a -(CH₂)ₙ chain which may be substituted by one to three radicals from the following group:
halogen, C₁-C₆-alkyl, C₁-C₆-alkoxy, hydroxyl or C₁-C₆-alkoxycarbonyl;
R⁷ is C₁-C₆-alkyl, C₃-C₆-alkenyl, C₃-C₆-haloalkenyl, C₃-C₆-alkynyl, C₃-C₆-haloalkynyl, C₃-C₆-cycloalkyl, C₁-C₂₀-alkylcarbonyl, C₂-C₆-alkenylcarbonyl, C₂-C₆-alkynylcarbonyl, C₃-C₆-cycloalkylcarbonyl, C₁-C₆-alkoxycarbonyl, C₃-C₆-alkenyloxycarbonyl, C₃-C₆-alkynyloxycarbonyl, (C₁-C₂₀-alkylthio)carbonyl, C₁-C₆-alkylaminocarbonyl, C₃-C₆-alkenylaminocarbonyl, C₃-C₆-alkynylaminocarbonyl, N,N-di-(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)aminocarbonyl, di-(C₁-C₆-alkyl)-aminothiocarbonyl, C₁-C₆-alkylcarbonyl-C₁-C₆-alkyl, C₁-C₆-alkoxyimino-C₁-C₆-alkyl, N-(C₁-C₆-alkylamino)imino-C₁-C₆-alkyl or N,N-di-(C₁-C₆-alkylamino)imino-C₁-C₆-alkyl, where the abovementioned alkyl, cycloalkyl and alkoxy radicals may be partially or fully halogenated and/or may carry one to three of the following groups:
cyano, C₁-C₄-alkoxy, C₁-C₄-alkylthio, di-(C₁-C₄-alkyl)amino, C₁-C₄-alkylcarbonyl, C₁-C₄-alkoxycarbonyl, C₁-C₄-alkoxy-C₁-C₄-alkoxycarbonyl, di-(C₁-C₄-alkyl)amino-C₁-C₄-alkoxycarbonyl, hydroxycarbonyl, C₁-C₄-alkylaminocarbonyl, di-(C₁-C₄-alkyl)aminocarbonyl, aminocarbonyl, C₁-C₄-alkylcarbonyloxy or C₃-C₆-cycloalkyl;
phenyl, heterocyclyl, phenyl-C₁-C₆-alkyl, heterocyclyl-C₁-C₆-alkyl, phenylcarbonyl-C₁-C₆-alkyl, heterocyclylcarbonyl-C₁-C₆-alkyl, phenylcarbonyl, heterocyclylcarbonyl, phenoxycarbonyl, heterocyclyloxycarbonyl, phenoxythiocarbonyl, heterocyclyloxythiocarbonyl, phenoxy-C₁-C₆-alkylcarbonyl, heterocyclyloxy-C₁-C₆-alkylcarbonyl, phenylaminocarbonyl, N-(C₁-C₆-alkyl)-N-(phenyl)aminocarbonyl, heterocyclylaminocarbonyl, N-(C₁-C₆-alkyl)-N-(heterocyclyl)aminocarbonyl, phenyl-C₂-C₆-alkenylcarbonyl or heterocyclyl-C₂-C₆-alkenylcarbonyl, where the phenyl and the heterocyclyl radical of the 20 last-mentioned substituents may be partially or fully halogenated and/or may carry one to three of the following radicals:
nitro, cyano, C₁-C₄-alkyl, C₁-C₄-halogenalkyl, C₁-C₄-alkoxy or C₁-C₄-haloalkoxy;
R⁸, R⁹ are C₁-C₆-alkyl, C₃-C₆-alkenyl, C₃-C₆-haloalkenyl, C₃-C₆-alkynyl, C₃-C₆-haloalkynyl, C₃-C₆-cycloalkyl, hydroxyl, C₁-C₆-alkoxy, amino, C₁-C₆-alkylamino, C₁-C₆-haloalkylamino, di-(C₁-C₆-alkyl)amino or di-(C₁-C₆-haloalkyl)amino, where the abovementioned alkyl, cycloalkyl and alkoxy radicals may be partially or fully halogenated and/or may carry one to three of the following groups:
cyano, C₁-C₄-alkoxy, C₁-C₄-alkylthio, di-(C₁-C₄-alkyl)amino, C₁-C₄-alkylcarbonyl, C₁-C₄-alkoxycarbonyl, C₁-C₄-alkoxy-C₁-C₄-alkoxycarbonyl, di-(C₁-C₄-alkyl)amino-C₁-C₄-alkoxycarbonyl, hydroxycarbonyl, C₁-C₄-alkylaminocarbonyl, di-(C₁-C₄-alkyl)aminocarbonyl, aminocarbonyl, C₁-C₄-alkylcarbonyloxy or C₃-C₆-cycloalkyl;
phenyl, heterocyclyl, phenyl-C₁-C₆-alkyl, heterocyclyl-C₁-C₆-alkyl, phenoxy, heterocyclyloxy, where the phenyl and the heterocyclyl radical of the last-mentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R¹⁰ is C₁-C₆-alkyl, C₃-C₆-alkenyl, C₃-C₆-haloalkenyl, C₃-C₆-alkynyl, C₃-C₆-haloalkynyl, C₃-C₆-cycloalkyl, hydroxyl, C₁-C₆-alkoxy, C₃-C₆-alkenyloxy, C₃-C₆-alkynyloxy, amino, C₁-C₆-alkylamino, di-(C₁-C₆-alkyl)amino or C₁-C₆-alkylcarbonylamino, where the abovementioned alkyl, cycloalkyl and alkoxy radicals may be partially or fully halogenated and/or may carry one to three radicals from the following group:
cyano, C₁-C₄-alkoxy, C₁-C₄-alkylthio, di-(C₁-C₄-alkyl)amino, C₁-C₄-alkylcarbonyl, C₁-C₄-alkoxycarbonyl, C₁-C₄-alkoxy-C₁-C₄-alkoxycarbonyl, di-(C₁-C₄-alkyl)amino-C₁-C₄-alkoxycarbonyl, hydroxycarbonyl, C₁-C₄-alkylaminocarbonyl, di-(C₁-C₄-alkyl)aminocarbonyl, aminocarbonyl, C₁-C₄-alkylcarbonyloxy or C₃-C₆-cycloalkyl;
phenyl, heterocyclyl, phenyl-C₁-C₆-alkyl or heterocyclyl-C₁-C₆-alkyl, where the phenyl or heterocyclyl radical of the four last-mentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R¹¹, R¹² are C₁-C₆-alkyl, C₃-C₆-alkenyl, C₃-C₆-alkynyl or C₁-C₆-alkylcarbonyl;
l is 0 to 6;
m is 2 to 4;
n is 1 to 5;
p is 2 to 5;
and their agriculturally useful salts.

2. A cyclohexenonequinolinoyl derivative of the formula I as claimed in claim 1, where
R¹ is halogen, C₁-C₆-alkyl, C₁-C₆-haloalkyl, C₁-C₆-alkoxy, C₁-C₆-alkylthio, heterocyclyloxy or phenylthio, where the two last-mentioned radicals may be partially or fully halogenated and/or may carry one to three of the substituents mentioned below:
nitro, cyano, C₁-C₄-alkyl, C₁-C₄-haloalkyl, C₁-C₄-alkoxy or C₁-C₄-haloalkoxy;
R₅ is halogen, OR₇, SR⁷, SOR⁸, SO₂R⁸, OSO₂R⁸, OPR⁸R⁹, OPOR⁸R⁹, OPSR⁸R⁹, NR¹⁰R¹¹ or N-bonded heterocyclyl which may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy.

3. A cyclohexenonequinolinoyl derivative of the formula I as claimed in claim 1 or 2, where
R⁵ is halogen, OR⁷, NR¹⁰R¹¹ or N-bonded heterocyclyl which may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy.

4. A cyclohexenonequinolinoyl derivative of the formula I as claimed in claims 1 to 3, where
R⁷ is C₁-C₆-alkyl, C₁-C₂₀-alkylcarbonyl, C₁-C₆-alkoxycarbonyl, (C₁-C₂₀-alkylthio)carbonyl, N,N-di-(C₁-C₆-alkyl)aminocarbonyl, phenyl, phenylcarbonyl or phenoxy-C₁-C₆-alkylcarbonyl, where the phenyl radical of the three last-mentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R¹⁰ is C₁-C₆-alkyl or C₁-C₆-alkoxy;
R¹¹ is C₁-C₆-alkyl.

5. A cyclohexenonequinolinoyl derivative of the formula I as claimed in claims 1 to 4, where
R⁶ is nitro, halogen, cyano, C₁-C₆-alkyl, C₁-C₆-haloalkyl, di-(C₁-C₆-alkoxy)methyl, di-(C₁-C₆-alkylthio)methyl, (C₁-C₆-alkoxy)(C₁-C₆-alkylthio)-methyl, hydroxyl, C₁-C₆-alkoxy, C₁-C₆-haloalkoxy, C₁-C₆-alkoxycarbonyloxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, C₁-C₆-alkylcarbonyl, C₁-C₆-haloalkylcarbonyl, C₁-C₆-alkoxycarbonyl or C₁-C₆-haloalkoxycarbonyl;
or
two radicals R⁶, which are linked to the same carbon, together form an -O-(CH₂)ₘ-O-, -O-(CH₂)ₘ-S-, -S-(CH₂)ₘ-S-, -O-(CH₂)ₙ- or -S-(CH₂)ₙ chain which may be substituted by one to three radicals from the following group:
halogen, cyano, C₁-C₄-alkyl, C₁-C₄-haloalkyl or C₁-C₄-alkoxycarbonyl;
or
two radicals R⁶, which are linked to the same carbon, together form a -(CH₂)ₚ chain which may be interrupted by oxygen or sulfur and/or may be substituted by one to four radicals from the following group:
halogen, cyano, C₁-C₄-alkyl, C₁-C₄-haloalkyl or C₁-C₄-alkoxycarbonyl;
or
two radicals R⁶, which are linked to the same carbon, together with this carbon form a carbonyl group.

6. A process for preparing compounds of the formula I as claimed in claims 1 to 5 where R⁵ = halogen, which comprises reacting a cyclohexanedione derivative of the formula III, 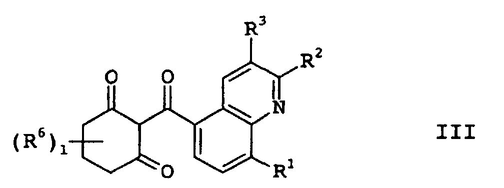 where the variables R¹ to R³, R⁶ and 1 are each as defined in claims 1 to 5, with a halogenating agent.

7. A process for preparing compounds of the formula I as claimed in claims 1 to 5 where R⁵ = OR⁷, OSO₂R⁸, OPR⁸R⁹, OPOR⁸R⁹ or OPSR⁸R⁹, which comprises reacting a cyclohexanedione derivative of the formula III, 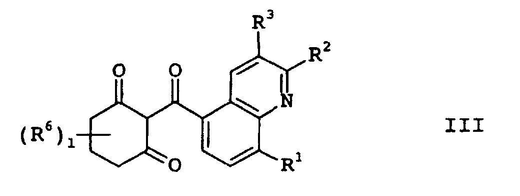 where the variables R¹ to R³, R⁶ and 1 are each as defined in claims 1 to 5, with a compound of the formula IVα, IVβ, IVγ, IVδ or IVε, 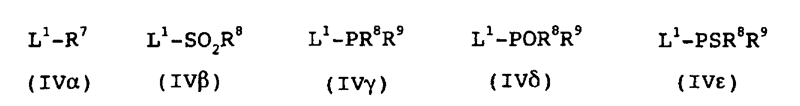 where the variables R⁷ to R⁹ are each as defined in claims 1 to 5 and L¹ is a nucleophilically replaceable leaving group.

8. A process for preparing compounds of the formula I as claimed in claims 1 to 5 where R⁵ = OR⁷, SR⁷, POR⁸R⁹, NR¹⁰R¹¹, ONR¹¹R¹², N-linked heterocyclyl or O-(N-linked heterocyclyl), which comprises reacting a compound of the formula Iα (≡ I where R⁵ = halogen, OSO₂R⁸), 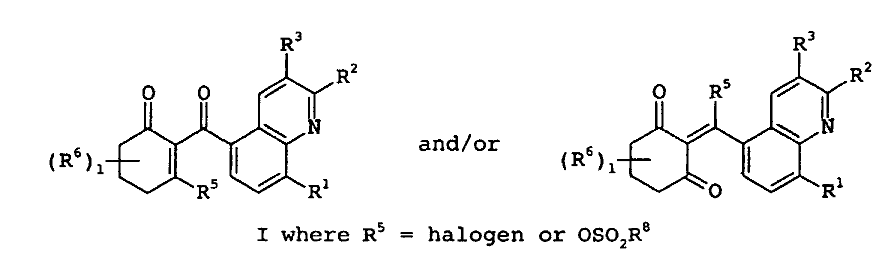 where the variables R¹ to R³, R⁶ and 1 are each as defined in claims 1 to 5, with a compound of the formula Vα, Vβ, Vγ, Vδ, Vε, Vη or Vϑ, 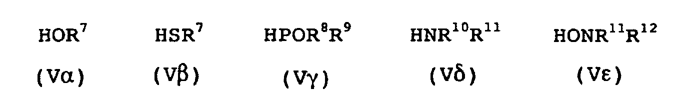 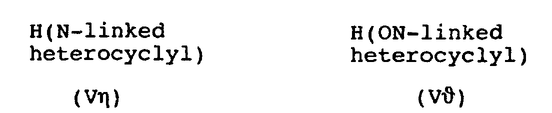 where the variables R⁷ to R¹² are each as defined in claims 1 to 5, if appropriate in the presence of a base.

9. A process for preparing compounds of the formula I as claimed in claims 1, 2 or 5, where R⁵ = SOR⁸, SO₂R⁸, which comprises reacting a compound of the formula Iβ (≡ I where R⁵ = SR⁸), 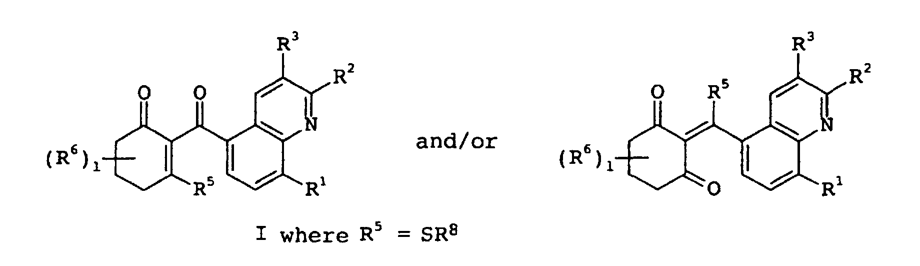 where the variables R¹ to R⁸ and 1 are each as defined in claims 1, 2 or 5, with an oxidizing agent.

10. A composition, comprising a herbicidally effective amount of at least one cyclohexenonequinolinoyl derivative of the formula I or an agriculturally useful salt of I as claimed in claims 1 to 5 and auxiliaries which are customarily used for formulating crop protection agents.

11. A process for preparing compositions as claimed in claim 10, which comprises mixing a herbicidally effective amount of at least one cyclohexenonequinolinoyl derivative of the formula I or an agriculturally useful salt of I as claimed in claims 1 to 5 and auxiliaries which are customarily used for formulating crop protection agents.

12. A method for controlling undesirable vegetation, which comprises allowing a herbicidally effective amount of at least one cyclohexenonequinolinoyl derivative of the formula I or an agriculturally useful salt of I as claimed in claims 1 to 5 to act on plants, their habitat and/or on seeds.

13. The use of cyclohexenonequinolinoyl derivatives of the formula I or their agriculturally useful salts as claimed in claims 1 to 5 as herbicides.

## Revendications

1. Dérivés de cyclohexénonequinolinoyle répondant à la formule I 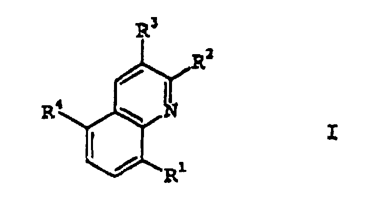 dans laquelle les variables possèdent les significations ci-après :
R¹ représente un atome d'hydrogène, un groupe nitro, un atome d'halogène, un groupe cyano, un groupe alkyle en C₁-C₆, un groupe halogénalkyle en C₁-C₆, un groupe alcoxy(en C₁-C₆)-iminométhyle, un groupe alcoxy en C₁-C₆, un groupe halogénalcoxy en C₁-C₆, un groupe alkyl(en C₁-C₆)thio, un groupe halogénalkyl(en C₁-C₆)thio, un groupe alkyl(en C₁-C₆)-sulfinyle, un groupe halogénalkyl(en C₁-C₆)sulfinyle, un groupe alkyl(en C₁-C₆)sulfonyle, un groupe halogénalkyl(en C₁-C₆)-sulfonyle, un groupe aminosulfonyle, un groupe N-(alkyl en C₁- C₆)-aminosulfonyle, un groupe N,N-(dialkyl en C₁-C₆)-aminosulfonyle, un groupe N-(alkyl(en C₁-C₆)sulfonyl)-amino, un groupe N-(halogénalkyl(en C₁-C₆)sulfonyl)-amino, un groupe N-(alkyl en C₁-C₆)-N-(alkyl(en C₁-C₆)sulfonyl)-amino, un groupe N-(alkyl en C₁-C₆)-N-(halogénalkyl(en C₁-C₆)sulfonyl)-amino, un groupe phénoxy, un groupe hétérocycloyl-oxy, un groupe phénylthio ou un groupe hétérocyclylthio, les quatre radicaux mentionnés en dernier lieu pouvant être soumis à une halogénation partielle ou complète et/ou pouvant porter de 1 à 3 substituants choisis parmi ceux repris ci-après :
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R², R³ représentent un atome d'hydrogène, un groupe alkyle en C₁- C₆, un groupe halogénalkyle en C₁-C₆ ou un atome d'halogène ;
R⁴ représente un composé IIa ou IIb 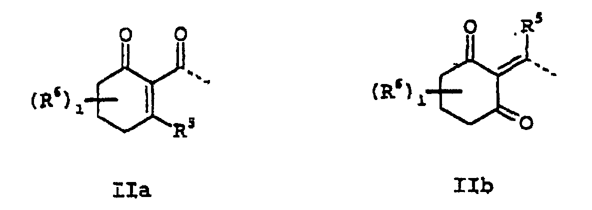
dans lesquels
R⁵ représente un atome d'halogène, un groupe OR⁷, un groupe SR⁷, un groupe SOR⁸, un groupe SO₂R⁸, un groupe OSO₂R⁸, un groupe POR⁸R⁹, un groupe OPR⁸R⁹, un groupe OPOR⁸R⁹, un groupe OPSR⁸R⁹, un groupe NR¹⁰R¹¹, un groupe ONR¹¹R¹², un groupe hétérocyclyle lié à un atome d'azote ou un groupe O-(hétérocyclyle lié à un atome d'azote), le radical hétérocyclyle des deux substituants mentionnés en dernier lieu pouvant être soumis à une halogénation partielle ou complète et/ou pouvant porter de 1 à 3 radicaux choisis parmi ceux repris ci-après :
un groupe nitro, un groupe cyano, un groupe alkyle en C₁-C₄, un groupe halogénalkyle en C₁-C4, un groupe alcoxy en C₁-C₄ ou un groupe halogénalcoxy en C1-C₄ ;
R⁶ représente un groupe nitro, un atome d'halogène, un groupe cyano, un groupe alkyle en C₁-C₆, un groupe halogénalkyle en C₁-C₆, un groupe di(alcoxy en C₁-C₆)méthyle, un groupe di(alkyl(en C₁-C6)thio)méthyle, un groupe (alcoxy en C₁-C₆)-(alkyl(en C₁-C₆)thio)méthyle, un groupe hydroxyle, un groupe alcoxy en C₁-C6, un groupe halogénalcoxy en C₁-C₆, un groupe alcoxy(en C₁-C₆)carbonyloxy, un groupe alkyl(en C₁- C₆)thio, un groupe halogénalkyl(en C₁-C₆)thio, un groupe alkyl(en C₁-C₆)sulfinyle, un groupe halogénalkyl(en C₁- C₆)sulfinyle, un groupe alkyl(en C₁-C₆)sulfonyle, un groupe halogénalkyl(en C₁-C₆)sulfonyle, un groupe alkyl(en C₁-C₆)-carbonyle, un groupe halogénalkyl(en C₁-C₆)-carbonyle, un groupe alcoxy(en C₁-C₆)carbonyle ou un groupe halogénalcoxy(en C₁-C₆)carbonyle ;
ou
deux radicaux R⁶, qui sont liés au même atome de carbone, forment ensemble une chaîne -O-(CH₂)ₘ-O-, une chaîne -O-(CH₂)ₘ-S-, une chaîne -S-(CH₂)ₘ-S-, une chaîne -O-(CH₂)ₙ- ou une chaîne -S-(CH₂)ₙ-, qui peut porter, à titre de substituants, de 1 à 3 radicaux choisis parmi le groupe ci-après:
un atome d'halogène, un groupe cyano, un groupe alkyle en C₁- C₄, un groupe halogénalkyle en C₁-C4, un groupe alcoxy(en C₁ - C₄)-carbonyle ;
ou
deux radicaux R⁶, qui sont liés au même atome de carbone, forment ensemble une chaîne -(CH₂)ₚ- qui peut être interrompue par un ou plusieurs atomes d'oxygène ou de soufre et/ou qui peut porter, à titre de substituants, de 1 à 4 radicaux choisis parmi le groupe ci-après :
un atome d'halogène, un groupe cyano, un groupe alkyle en C₁- C₄, un groupe halogénalkyle en C₁-C₄, un groupe alcoxy(en C₁ - C₄) carbonyle ;
ou
deux radicaux R⁶, qui sont liés au même atome de carbone, forment ensemble un groupe méthylidène qui peut porter, à titre de substituants de 1 à 2 radicaux choisis parmi le groupe ci-après : un atome d'halogène, un groupe hydroxyle, un groupe formyle, un groupe cyano, un groupe alkyle en C₁-C₆, un groupe halogénalkyle en C₁-C₆, un groupe alcoxy en C₁-C₆, un groupe halogénalcoxy en C₁-C₆, un groupe alkyl(en C₁-C₆)-thio, un groupe halogénalkyl(en C₁-C₆)thio, un groupe alkyl(en C₁-C₆)sulfinyle, un groupe halogénalkyl(en C₁-C₆)sulfinyle, un groupe alkyl(en C₁-C₆)sulfonyle ou un groupe halogénalkyl(en C₁-C₆)sulfonyle ;
ou
deux radicaux R⁶, qui sont liés au même atome de carbone, forment ensemble, avec cet atome de carbone, un groupe carbonyle ;
ou
deux radicaux R⁶, qui sont liés à des atomes de carbone différents, forment ensemble une chaîne -(CH₂)ₙ-, qui peut porter, à titre de substituants de 1 à 3 radicaux choisis parmi le groupe ci-après :
un atome d'halogène, un groupe alkyle en C₁-C₆, un groupe alcoxy en C₁-C₆, un groupe hydroxyle ou un groupe alcoxy(en C₁-C₆)carbonyle ;
R⁷ représente un groupe alkyle en C₁-C₆, un groupe alcényle en C₃ - C₆, un groupe halogénalcényle en C₃- C₆, un groupe alcynyle en C₃-C₆, un groupe halogénalcynyle en C₃-C₆, un groupe cycloalkyle en C₃-C₆, un groupe alkyl(en C₁-C₂₀)carbonyle, un groupe alcényl(en C₂-C₆)carbonyle, un groupe alcynyl(en C₂- C₆)carbonyle, un groupe cycloalkyl(en C₃-C₆)carbonyle, un groupe alcoxy(en C₁-C₆)carbonyle, un groupe alcényl(en C₃- C₆)oxycarbonyle, un groupe alcynyl(en C₃-C₆)oxycarbonyle, un groupe (alkyl(en C₁-C₂₀)thio)carbonyle, un groupe alkyl(en C₁- C₆)aminocarbonyle, un groupe alcényl(en C₃-C₆)aminocarbonyle, un groupe alcynyl(en C₃-C₆)aminocarbonyle, un groupe N,N-dialkyl(en C₁-C₆)aminocarbonyle, un groupe N-(alcényl en C₃-C₆)-N-(alkyl en C₁-C₆)aminocarbonyle, un groupe N-(alcynyl en C₃-C₆)-N-(alkyl en C₁-C₆)aminocarbonyle, un groupe N-(alcoxy en C₁-C₆)-N-(alkyl en C₁- C₆)aminocarbonyle, un groupe N-(alcényl en C₃-C₆)-N-(alcoxy en C₁-C₆)aminocarbonyle, un groupe N-(alcynyl en C₃-C₆)-N-(alcoxy en C₁-C₆)aminocarbonyle, un groupe di(alkyl en C₁- C₆)aminothiocarbonyle, un groupe alkyl(en C₁-C₆)carbonylalkyle en C₁-C₆, un groupe alcoxy(en C₁-C₆)iminoalkyle en C₁-C₆, un groupe N-(alkyl(en C₁-C₆)amino)iminoalkyle en C₁-C₆ ou un groupe N,N-di(alkyl(en C₁-C₆)amino)iminoalkyle en C₁-C₆, les radicaux alkyle, cycloalkyle et alcoxy mentionnés pouvant être soumis à une halogénation partielle ou complète et/ou pouvant porter de 1 à 3 groupes choisis parmi ceux repris ci-après :
un groupe cyano, un groupe alcoxy en C₁-C₄, un groupe alkyl(en C₁-C₄)thio, un groupe di(alkyl en C₁-C₄)amino, un groupe alkyl(en C₁-C₄)carbonyle, un groupe alcoxy(en C₁- C₄)carbonyle, un groupe alcoxy(en C₁-C₄)alcoxy(en C₁- C₄)carbonyle, un groupe di(alkyl en C₁-C₄)aminoalcoxy(en C₁- C₄)carbonyle, un groupe hydroxycarbonyle, un groupe alkyl(en C₁ - C₄)aminocarbonyle, un groupe di(alkyl en C₁- C₄)aminocarbonyle, un groupe amino-carbonyle, un groupe alkyl(en C₁-C₄)carbonyloxy ou un groupe cycloalkyle en C₃- C₆;
un groupe phényle, un groupe hétérocyclyle, un groupe phénylalkyle en C₁-C₆, un groupe hétérocyclylalkyle en C₁-C₆, un groupe phénylcarbonylalkyle en C₁-C₆, un groupe hétérocyclylcarbonylalkyle en C₁-C₆, un groupe phénylcarbonyle, un groupe hétérocyclylcarbonyle, un groupe phénoxycarbonyle, un groupe hétérocyclyloxycarbonyle, un groupe phénoxythiocarbonyle, un groupe hétérocyclyloxythiocarbonyle, un groupe phénoxyalkyl(en C₁-C₆)carbonyle, un groupe hétérocyclyloxyalkyl(en C₁-C₆)carbonyle, un groupe phénylaminocarbonyle, un groupe N-(alkyl en C₁-C₆)-N-(phényl)-aminocarbonyle, un groupe hétérocyclylaminocarbonyle, un groupe N-(alkyl en C₁-C₆)-N-(hétérocyclyl)aminocarbonyle, un groupe phénylalcényl(en C₂-C₆)carbonyle ou un groupe hétérocyclylalcényl(en C₂-C₆)carbonyle, le radical phényle et le radical hétérocyclyle des 20 derniers substituants pouvant être soumis à une halogénation partielle ou complète et/ou pouvant porter de 1 à 3 radicaux choisis ceux repris ci-après :
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R⁸, R⁹ représentent un groupe alkyle en C₁-C₆, un groupe alcényle en C₃- C₆, un groupe halogénalcényle en C₃-C₆, un groupe alcynyle en C₃-C₆, un groupe halogénalcynyle en C₃- C₆, un groupe cycloalkyle en C₃- C₆, un groupe hydroxyle, un groupe alcoxy en C₁-C₆, un groupe amino, un groupe alkyl(en C₁- C₆)amino, un groupe halogénalkyl(en C₁-C₆)amino, un groupe di(alkyl en C₁-C₆)amino ou un groupe di(halogénalkyl en C₁- C₆)amino, les radicaux alkyle, cycloalkyle et alcoxy mentionnés pouvant être soumis à une halogénation partielle ou complète et/ou pouvant porter de 1 à 3 groupes choisis parmi ceux repris ci-après :
un groupe cyano, un groupe alcoxy en C₁-C₄, un groupe alkyl(en C₁-C₄)thio, un groupe di(alkyl en C₁-C₄)amino, un groupe alkyl(en C₁-C₄)carbonyle, un groupe alcoxy(en C₁- C₄)carbonyle, un groupe alcoxy(en C₁-C₄)alcoxy(en C₁- C₄)carbonyle, un groupe di(alkyl en C₁-C₄)aminoalcoxy(en C₁- C₄)carbonyle, un groupe hydroxycarbonyle, un groupe alkyl(en C₁ - C₄)aminocarbonyle, un groupe di(alkyl en C₁-C₄)aminocarbonyle, un groupe aminocarbonyle, un groupe alkyl(en C₁- C₄)carbonyloxy ou un groupe cycloalkyle en C₃- C₆ ;
un groupe phényle, un groupe hétérocyclyle, un groupe phénylalkyle en C₁-C₆, un groupe hétérocyclylalkyle en C₁-C₆, un groupe phénoxy, un groupe hétérocyclyloxy, le radical phényle et le radical hétérocyclyle des substituants mentionnés en dernier lieu pouvant être soumis à une halogénation partielle ou complète et/ou pouvant porter de 1 à 3 radicaux choisis parmi ceux repris ci-après:
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R¹⁰ représente un groupe alkyle en C₁-C₆, un groupe alcényle en C₃ - C₆, un groupe halogénalcényle en C₃- C₆, un groupe alcynyle en C₃- C₆, un groupe halogénalcynyle en C₃- C₆, un groupe cycloalkyle en C₃- C₆, un groupe hydroxyle, un groupe alcoxy en C₁-C₆, un groupe alcényl(en C₃- C₅)oxy, un groupe alcynyl(en C₃- C₆)oxy, un groupe amino, un groupe alkyl(en C₁-C₆)amino, un groupe di(alkyl en C₁-C₆)amino ou un groupe alkyl(en C₁- C₆)carbonylamino, les radicaux alkyle, cycloalkyle et alcoxy mentionnés pouvant être soumis à une halogénation partielle ou complète et/ou pouvant porter de 1 à 3 groupes choisis parmi ceux repris ci-après :
un groupe cyano, un groupe alcoxy en C₁-C₄, un groupe alkyl(en C₁-C₄)thio, un groupe di(alkyl en C₁-C₄)amino, un groupe alkyl(en C₁-C₄)carbonyle, un groupe alcoxy(en C₁-C₄)-carbonyle, un groupe alcoxy(en C₁-C₄)alcoxy(en C₁-C₄)-carbonyle, un groupe di(alkyl en C₁-C₄)aminoalcoxy(en C₁- C₄)carbonyle, un groupe hydroxycarbonyle, un groupe alkyl(en C₁ - C₄)aminocarbonyle, un groupe di(alkyl en C₁-C₄)aminocarbonyle, un groupe aminocarbonyle, un groupe alkyl(en C₁- C₄)carbonyloxy ou un groupe cycloalkyle en C₃- C₆;
un groupe phényle, un groupe hétérocyclyle, un groupe phénylalkyle en C₁-C₆ ou un groupe hétérocyclylalkyle en C₁- C₆, le radical phényle ou le radical hétérocyclyle des quatre substituants mentionnés en dernier lieu pouvant être soumis à une halogénation partielle ou complète et/ou pouvant porter de 1 à 3 radicaux choisis parmi ceux repris ci-après :
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R¹¹, R¹² représentent un groupe alkyle en C₁-C₆, un groupe alcényle en C₃-C₆, un groupe alcynyle en C₃-C₆ ou un groupe alkyl(en C₁-C₆)carbonyle ;
l représente de 0 à 6 ;
m représente de 2 à 4 ;
n représente de 1 à 5 ;
p représente de 2 à 5 ;
ainsi que leurs sels utiles en agriculture.

2. Dérivés de cyclohexénonequinolinoyle répondant à la formule I selon la revendication 1, dans lesquels
R¹ représente un atome d'halogène, un groupe alkyle en C₁-C₆, un groupe halogénalkyle en C₁-C₆, un groupe alcoxy en C₁-C₆, un groupe alkyl(en C₁-C₆)thio, un groupe hétérocyclyloxy ou un groupe phénylthio, les deux radicaux mentionnés en dernier lieu pouvant être soumis à une halogénation partielle ou complète et/ou pouvant porter de 1 à 3 substituants choisis parmi ceux repris ci-après :
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄ ;
R⁵ représente un atome d'halogène, un groupe OR⁷, un groupe SR⁷, un groupe SOR⁸, un groupe SO₂R⁸, un groupe OSO₂R⁸, un groupe OPR⁸R⁹, un groupe OPOR⁸R⁹, un groupe OPSR⁸R⁹, un groupe NR¹⁰R¹¹ ou un groupe hétérocyclyle lié à un atome d'azote qui peut être soumis à une halogénation partielle ou complète et/ou qui peut porter de 1 à 3 radicaux choisis parmi ceux repris ci-après :
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄.

3. Dérivés de cyclohexénonequinolinoyle répondant à la formule I selon les revendications 1 ou 2, dans lesquels
R⁵ représente un atome d'halogène, un groupe OR⁷, un groupe NR¹⁰R¹¹ ou un groupe hétérocyclyle lié à un atome d'azote qui peut être soumis à une halogénation partielle ou complète et/ou qui peut porter de 1 à 3 radicaux choisis parmi ceux repris ci-après un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄.

4. Dérivés de cyclohexénonequinolinoyle répondant à la formule I selon les revendications 1 à 3, dans lesquels
R⁷ représente un groupe alkyle en C₁-C₆, un groupe alkyl(en C₁- C₂₀)carbonyle, un groupe alcoxy(en C₁-C₆)carbonyle, un groupe alkyl(en C₁-C₂₀)thiocarbonyle, un groupe N,N-di(alkyl en C₁-C₆)aminocarbonyle, un groupe phényle, un groupe phénylcarbonyle ou un groupe phénoxyalkyl(en C₁-C₆)carbonyle, le radical phényle des trois substituants mentionnés en dernier lieu pouvant être soumis à une halogénation partielle ou complète et/ou pouvant porter de 1 à 3 radicaux choisis parmi ceux repris ci-après :
un groupe nitro, un groupe cyano, un groupe alkyle en C₁-C₄, un groupe halogénalkyle en C₁-C₄, un groupe alcoxy en C₁-C₄ ou un groupe halogénalcoxy en C₁-C₄.
R¹⁰ représente un groupe alkyle en C₁-C₆, un groupe alcoxy en C₁- C₆,
R¹¹ représente un groupe alkyle en C₁-C₆,

5. Dérivés de cyclohexénonequinolinoyle répondant à la formule I selon les revendications 1 à 4, dans lesquels
R⁶ représente un groupe nitro, un atome d'halogène, un groupe cyano, un groupe alkyle en C₁-C₆, un groupe halogénalkyle en C₁-C₆, un groupe di(alcoxy en C₁-C₆)méthyle, un groupe di(alkyl(en C₁-C₆)thio)méthyle, un groupe (alcoxy en C₁-C₆)-(alkyl(en C₁-C₆)thio)méthyle, un groupe hydroxyle, un groupe alcoxy en C₁-C₆, un groupe halogénalcoxy en C₁-C₆, un groupe alcoxycarbonyl(en C₁-C₆)oxy, un groupe alkyl(en C₁- C₆)thio, un groupe halogénalkyl(en C₁-C₆)thio, un groupe alkyl(en C₁-C₆)sulfinyle, un groupe halogénalkyl(en C₁- C₆)sulfinyle, un groupe alkyl(en C₁-C₆)sulfonyle, un groupe halogénalkyl(en C₁-C₆)sulfonyle, un groupe alkyl(en C₁-C₆)-carbonyle, un groupe halogénalkyl(en C₁-C₆)-carbonyle, un groupe alcoxy(en C₁-C₆)carbonyle ou un groupe halogénalcoxy(en C₁-C₅)carbonyle ;
ou
deux radicaux R⁶, qui sont liés au même atome de carbone, forment ensemble une chaîne -O-(CH₂)ₘ-O-, une chaîne -O-(CH₂)ₘ-S-, une chaîne -S-(CH₂)ₘ-S-, une chaîne -O-(CH₂)ₙ- ou une chaîne -S-(CH₂)ₙ-, qui peut porter, à titre de substituants, de 1 à 3 radicaux choisis parmi le groupe ci-après:
un atome d'halogène, un groupe cyano, un groupe alkyle en C₁- C₄, un groupe halogénalkyle en C₁-C₄ ou un groupe alcoxy(en C₁-C₄)carbonyle ;
ou
deux radicaux R⁶, qui sont liés au même atome de carbone, forment une chaîne -(CH₂)ₚ- qui peut être interrompue par un ou plusieurs atomes d'oxygène ou de soufre et/ou qui peut porter, à titre de substituants, de 1 à 4 radicaux choisis parmi le groupe ci-après: un atome d'halogène, un groupe cyano, un groupe alkyle en C₁- C₄, un groupe halogénalkyle en C₁-C4 ou un groupe alcoxy(en C₁-C₄)carbonyle ;
ou
deux radicaux R⁶, qui sont liés au même atome de carbone, forment ensemble, avec cet atome de carbone, un groupe carbonyle.

6. Procédé pour la préparation de composés répondant à la formule I selon les revendications 1 à 5, dans lesquels R⁵ représente un atome d'halogène **caractérisé en ce qu'**on fait réagir un dérivé de cyclohexanedione répondant à la formule III 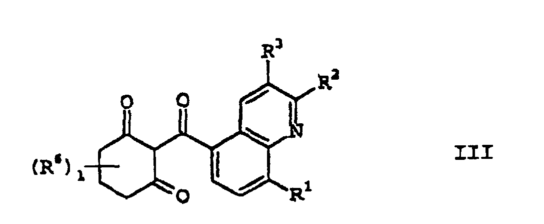 dans laquelle les variables R¹ à R³, R⁶ et I ont la signification mentionnée dans les revendications 1 à 5, avec un agent d'halogénation.

7. Procédé pour la préparation de composés répondant à la formule I selon les revendications 1 à 5, dans lesquels R⁵ représente un groupe OR⁷, un groupe OSO₂R⁸, un groupe OPR⁸R⁹, un groupe OPOR⁸R⁹ ou un groupe OPSR⁸R⁹, **caractérisé en ce qu'**on fait réagir un dérivé de cyclohexanedione répondant à la formule III 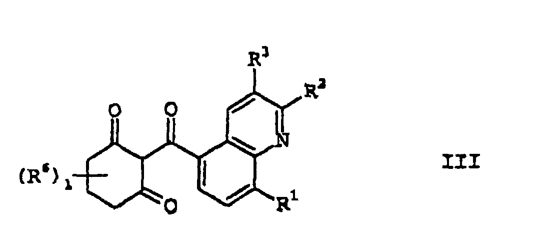 dans laquelle les variables R¹ à R³, R⁶ et I ont la signification mentionnée dans les revendications 1 à 5, avec un composé répondant aux formules IVα, IVβ, IVγ, IVδ ou IVε, 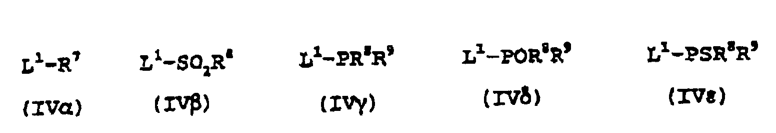 dans lesquelles les variables R⁷ à R⁹ ont la signification mentionnée dans les revendications 1 à 5, et L¹ représente un groupe séparable apte à un déplacement nucléophile.

8. Procédé pour la préparation de composés répondant à la formule 1 selon les revendications 1 à 5, dans lesquels R⁵ représente un groupe OR⁷, un groupe SR⁷, un groupe POR⁸R⁹, un groupe NR¹⁰R¹¹, un groupe ONR¹¹R¹², un groupe hétérocyclyle lié à un atome d'azote ou un groupe O-(hétérocyclyle lié à un atome d'azote), **caractérisé en ce qu'**on fait réagir un composé répondant à la formule Iα (≡ I dans laquelle R⁵ représente un atome d'halogène, un groupe OSO₂R⁸), 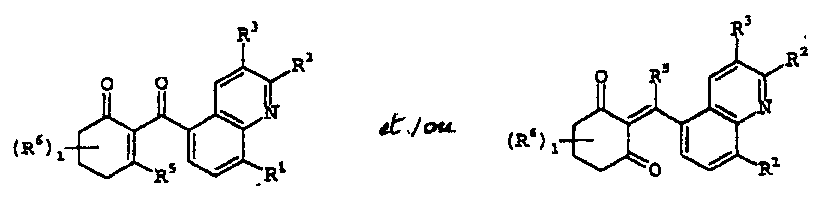 I avec R⁵ = un atome d'halogène ou un groupe OSO₂R⁸
dans lesquelles les variables R¹ à R³, R⁶ et I ont la signification mentionnée dans les revendications 1 à 5, avec un composé répondant aux formules Vα, Vβ, Vγ, Vδ, Vε, Vη ou Vϑ, 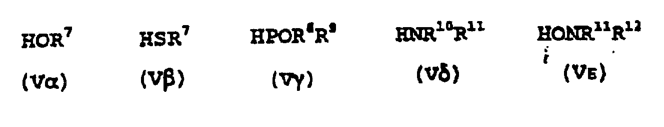 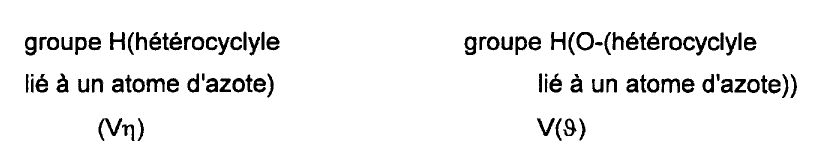 dans lesquelles les variables R⁷ à R¹² ont la signification mentionnée dans les revendications 1 à 5, le cas échéant en présence d'une base.

9. Procédé pour la préparation de composés répondant à la formule I selon les revendications 1, 2 ou 5, dans lesquels R⁵ représente un groupe SOR⁸, un groupe SO₂R⁸, **caractérisé en ce qu'**on fait réagir un composé répondant à la formule Iβ (≡ I dans laquelle R⁵ représente un groupe SR⁸), 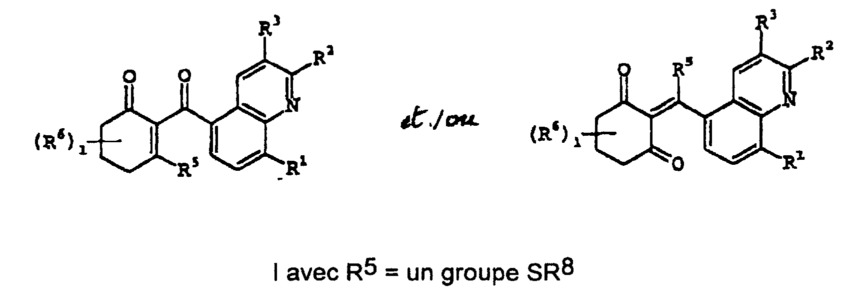 dans lesquelles les variables R¹ à R⁸ et I ont la signification mentionnée dans les revendications 1, 2 ou 5, avec un agent d'oxydation.

10. Agent contenant une quantité efficace du point de vue herbicide d'au moins un dérivé de cyclohexénonequinolinoyle répondant à la formule I ou d'un sel utile en agriculture du dérivé répondant à la formule I selon les revendications 1 à 5 et des adjuvants habituels pour la formulation d'agents de protection des plantes.

11. Procédé pour la préparation d'agents selon la revendication 10, **caractérisé en ce qu'**on mélange une quantité efficace du point de vue herbicide d'au moins un dérivé de cyclohexénonequinolinoyle répondant à la formule I ou d'un sel utile en agriculture du dérivé répondant à la formule I selon les revendications 1 à 5 et des adjuvants habituels pour la formulation d'agents de protection des plantes.

12. Procédé pour lutter pour la croissance non désirée de plantes, **caractérisé en ce qu'**on laisse agir une quantité efficace du point de vue herbicide d'au moins un dérivé de cyclohexénonequinolinoyle répondant à la formule I ou d'un sel utile en agriculture du dérivé répondant à la formule I selon les revendications 1 à 5, sur des plantes, sur leur biotope et/ou sur des semences.

13. Utilisation de dérivés de cyclohexénonequinolinoyle répondant à la formule I ou de leurs sels utiles en agriculture selon les revendications 1 à 5, à titre d'herbicides.
